# EUROPEAN PATENT SPECIFICATION

(11) **EP 3 809 871 B1**
(45) Date of publication and mention of the grant of the patent: **14.02.2024**
(21) Application number: 19820577.5
(22) Date of filing: 13.06.2019
(51) Int. Cl.: A61K 31/519, A61P 9/00, A61P 19/06, A23L 2/56, A23L 2/60, A23L 2/52, A61K 36/899, A61K 36/82, A61K 36/78, A61K 36/752, A61K 36/73, A61K 36/62, A61K 36/48, A61K 36/28, A61K 36/235, A61K 36/185, A61K 36/16, A23L 33/105

(54) **DEVELOPMENT OF HEALTH FOOD SUPPLEMENTS AND ANTIOXIDANTS FOR CONTROLLING HYPERURICEMIA AND OXIDATIVE STRESS**
ENTWICKLUNG VON GESUNDHEITSFÖRDERNDEN NAHRUNGSERGÄNZUNGEN UND ANTIOXIDANTIEN ZUR KONTROLLE VON HYPERURIKÄMIE UND OXIDATIVEM STRESS
ÉLABORATION DE COMPLÉMENTS ALIMENTAIRES NATURELS ET D'ANTIOXYDANTS POUR LUTTER CONTRE L'HYPERURICÉMIE ET LE STRESS OXYDATIF

(30) Priority: 13.06.2018 US 201862684406 P
(43) Date of publication of application: 28.04.2021
(73) Proprietor: Baylor College of Medicine, Houston, TX 77030-3498 (US)
(72) Inventor: CHEN, Changyi, Houston, TX 77056 (US); YAO, Qizhi, Houston, TX 77056 (US); LU, Jian-Ming, Pearland, TX 77584 (US)
(74) Representative: J A Kemp LLP
(86) International application number: PCT/IB2019/000801
(87) International publication number: WO 2019/239215

(56) References cited:
- CN-A- 104 888 994
- CN-A- 104 922 292
- CN-B- 103 330 782
- US-A1- 2005 163 870
- US-A1- 2009 252 796
- US-A1- 2010 202 980
- US-A1- 2011 038 959
- US-A1- 2014 255 371
- YANG XIAOLING ET AL: "In renal hypertension, Cirsium japonicum strengthens cardiac function via the intermedin/nitric oxide pathway", BIOMEDICINE & PHARMACOTHERAPY, vol. 101, 1 May 2018 (2018-05-01), pages 787-791, XP055884648, FR ISSN: 0753-3322, DOI: 10.1016/j.biopha.2018.02.126
- JANG MIRAN ET AL: "Cirsium japonicum Extracts Show Antioxidant Activity and PC12 Cell Protection against Oxidative Stress", KOREAN JOURNAL OF FOOD SCIENCE AND TECHNOLOGY, SEOUL, KR, vol. 48, no. 2, 30 April 2016 (2016-04-30) , pages 172-177, XP053035556, ISSN: 0367-6293, DOI: 10.9721/KJFST.2016.48.2.172
- LAI WAN-CHUN ET AL: "Bioactive Constituents of Cirsium japonicum var. australe", JOURNAL OF NATURAL PRODUCTS, vol. 77, no. 7, 25 July 2014 (2014-07-25), pages 1624-1631, XP055884475, US ISSN: 0163-3864, DOI: 10.1021/np500233t Retrieved from the Internet: URL:https://pubs.acs.org/doi/pdf/10.1021/n p500233t>
- LIU S ET AL: "Tumor inhibition and improved immunity in mice treated with flavone from Cirsium japonicum DC", INTERNATIONAL IMMUNOPHARMACOLOGY, ELSEVIER, AMSTERDAM, NL, vol. 6, no. 9, 1 September 2006 (2006-09-01), pages 1387-1393, XP024976903, ISSN: 1567-5769, DOI: 10.1016/J.INTIMP.2006.02.002 [retrieved on 2006-09-01]
- HYUN AH JUNG ET AL: "Anti-inflammatory activity of Korean thistleand its major flavonoid, luteolin 5--glucoside", FOOD AND CHEMICAL TOXICOLOGY, PERGAMON, GB, vol. 50, no. 6, 6 April 2012 (2012-04-06), pages 2171-2179, XP028508522, ISSN: 0278-6915, DOI: 10.1016/J.FCT.2012.04.011 [retrieved on 2012-04-16]
- JESSE DAWSON ET AL: "Uric acid and xanthine oxidase: future therapeutic targets in the prevention of cardiovascular disease?", BRITISH JOURNAL OF CLINICAL PHARMACOLOGY, vol. 62, no. 6, 1 December 2006 (2006-12-01), pages 633-644, XP055071543, ISSN: 0306-5251, DOI: 10.1111/j.1365-2125.2006.02785.x
- PACHER P ET AL: "Therapeutic effects of xanthine oxidase inhibitors: Renaissance half a century after the discovery of allopurinol", PHARMACOLOGICAL REVIEWS, AMERICAN SOCIETY FOR PHARMACOLOGY AND EXPERIMENTAL THERAPEUTICS, US, vol. 58, no. 1, 1 March 2006 (2006-03-01), pages 87-114, XP002602841, ISSN: 0031-6997, DOI: 10.1124/PR.58.1.6

## Description

### TECHNICAL FIELD

Embodiments of the disclosure encompass at least the fields of cell biology, molecular biology, and medicine.

### BACKGROUND

Based on the 2018 report from the American Heart Association, there are 836,546 deaths caused by cardiovascular disease (CVD) in the United States in 2015; it accounts about 2,300 Americans who died from CVD each day, an average of 1 death every 38 seconds. CVD still remains the number one cause of death in the United States. About 92.1 million American adults are living with some form of CVD. In addition to several traditional CVD risk factors, there are many new risk factors, which have recently been recognized and further investigated. For example, hyperuricemia has long been established as the major etiologic factor in gout. Besides gout, the association of hyperuricemia with CVD was described 2 centuries ago. Only in the last few years, several large clinical studies have confirmed that hyperuricemia is a significant and independent risk factor for CVD including ischemic heart disease, heart failure and hypertension after an extensive adjustment for almost all of the possible confounding conditions. Hyperuricemia is an independent risk factor of atrial fibrillation. High levels of serum uric acid (SUA) directly impair the cardiovascular system in a concentration-dependent matter. For each increase of 1 mg/dL of SUA, the overall risks of coronary heart disease and all-cause mortality increased by 20 and 9 %, respectively. Increase in SUA level by 1 mg/dl resulted in an equivalent risk of 20 mg/dl increase in serum cholesterol as well as 10 mm Hg elevation of systolic blood pressure. SUA levels increased by 1 mg/dL, the risk of hypertension increased by 15% to 23%. The risk of type 2 diabetes mellitus was also increased by 6% for every 1 mg/dL increase.

The prevalence of gout and hyperuricemia in the USA is about 4% and 21%, respectively. The pooled prevalence of gout and hyperuricemia in mainland China from 2000 to 2014 was 1.1% and 13-25%, respectively. In the some areas of the world, the prevalence of gout and hyperuricemia is high, up to 11.7% and 41.1, respectively. Xanthine oxidase (XO) is a ratelimiting enzyme in purine catabolism, generating final product, uric acid; during the chemical reaction, XO also produces reactive oxygen species (superoxide anion and H₂O₂). Thus, both hyperuricemia and oxidative stress generated from XO significantly contributes to the development of CVD. Accordingly, urate-lowering drugs such as XO-inhibitors could play an important role in the prevention and treatment of CVD. Thus, SUA is a central player, not an innocent bystander, in CVD and several other diseases. Mechanistically, hyperuricemia contributes to the progression of CVD through oxidative stress, systemic inflammation, and endothelial dysfunction. Clinically, treatment with Allopurinol, a XO-inhibitor drug, significantly reduced the risk of myocardial infarction, reduced all-cause and cardiovascular mortality in high-risk patients, and improved endothelial functions in several clinical trials. However, because of its potential adverse effects, clinically available XO-inhibitor drug, Allopurinol, is not indicated for clinical management of chronic hyperuricemia-related CVD and other diseases including diabetes, insulin resistance, metabolic syndrome, chronic kidney disease, psoriatic arthritis, micro-albuminuria, erectile dysfunction, preeclampsia, cancers, immune disorders, ischemia reperfusion injury, inflammatory diseases, or tumor lysis syndrome.

Febuxostat is another FDA-approved XO-inhibitor drug; however, it also has potential side effects, which prevent it from long-term administration for asymptomatic hyperuricemia.

In the clinical trials, the 10 most common treatment-related adverse events in LTE studies by preferred term during febuxostat treatment (regardless of dose) were: hepatic enzyme increased, ALT increased, liver function test abnormal, AST increased, hyperlipidaemia, blood creatinine increased, nephrolithiasis, arthritis, blood urea increased, and gammaglutamyltransferase (GGT) increased. A recent report showed a clinical case of febuxostat-induced liver injury. Febuxostat cannot be recommended for patients with moderate or severe hepatic impairment. The most common adverse effect leading to discontinuation of febuxostat was elevated liver function tests: in some studies up to 2-3% of patients developed transaminase elevations greater than three times the upper limit of normal. However, these studies did not establish a dose-effect relationship between febuxostat and elevated liver function tests.

In the first trials, cardiovascular events were a concern related to febuxostat treatment, especially concerning thromboembolic events, myocardial infarcts, and strokes. This is initial evidence of nonfatal cardiovascular events as febuxostat side effects, leading the FDA to require a long-term cardiovascular study as a condition for approving the drug.

In addition, initial clinical studies showed that febuxostat can also lead to cutaneous adverse effects in about 2% of patients. Cases of severe febuxostat hypersensitivity reactions such as SJS and anaphylactic shock are reported. These serious adverse effects with febuxostat are potentially associated with a history of skin reaction to allopurinol, particularly in patients with renal failure. A case report also showed that febuxostat induced rhabdomyolysis. Thus, febuxostat is currently not recommended for the treatment of asymptomatic hyperuricemia.

Currently, the development of newer agents with differing pharmacological mechanisms and less toxicity is an active field of research.

Additionally, there is an active research on the development of alternative medicines such as functional food/dietary health supplements and herb medicines for the long-term control of hyperuricemia with no or much less adverse effects compared to current XO-inhibitor drugs. Certain commercially-available dietary supplements might be beneficial in lowering uric acid levels in the body, but not all supplements used for this purpose are backed by extensive scientific research studies.

### BRIEF SUMMARY

Embodiments of the disclosure include methods and compositions for the treatment of one or more medical conditions. In specific embodiments, the medical condition is one in which inhibition of Xanthine oxidase (XO) activity would be therapeutically effective, such as to reduce the presence, severity, or onset of at least one symptom of the medical condition.

The present disclosure concerns the development of alternative medicines , and in an initial study there was testing of XO inhibitory effects of 72 traditional Chinese herb medicines. The dynamic effects of folic acid on XO inhibition was examined, because folic acid is one of the most commonly used dietary supplements. Folic acid and its derivatives have been demonstrated to have XO inhibitory effects.

In particular embodiments, some traditional Chinese herb medicines have potent XO-inhibitory activities; individual herb activities may have additive or synergistic effects with other herb medicines and/or with folic acid and/or one or more of its derivatives. Folic acid and its derivative 5-methyl tetrahydrofolate (5-MTHF) are XO inhibitors. Recent clinical trials have shown that folic acid supplement reduces SUA levels in hypertensive patients and reduced the risk for CVD. The functional food/dietary supplement formulation of the present disclosure that includes the use of traditional Chinese herb extracts and/or folic acid and/or one or more of its derivatives provides a new opportunity to prevent and/or treat a variety of medical conditions associated with XO, such as asymptomatic hyperuricemia, for example. Given the high prevalence of asymptomatic hyperuricemia (about 20% in general population in China and US) and lack of safe XO-inhibitor drugs, this novel approach is useful for its long-term management of asymptomatic hyperuricemia. Given that asymptomatic hyperuricemia is an independent risk factor for CVD and many other diseases, the disclosed methods have enormous impact on disease prevention.

In particular embodiments, the compositions comprising one or more herb extracts are considered non-natural, because extracts are not found in nature.

The foregoing has outlined rather broadly the features and technical advantages of the present disclosure in order that the detailed description that follows may be better understood.

Specifically, the present invention is defined in the appended set of claims. Hence, the composition of the present invention comprises Japanese Thistle extract and one or more of: Grape Seed Extract; Amla Fruit Extract; Pine Bark Extract; and Chinese Mint Extract. The composition of the present invention optionally further comprises one or more of folic acid and a functionally active derivative thereof, wherein the functionally active derivative is dihydrofolic acid, tetrahydrofolic acid, 5-methyl tetrahydrofolate (5-MTHF), or a combination thereof. It is emphasized that any disclosure herein relating to compositions other than the composition of the present invention are included purely for context and for better understanding of the present invention.

Furthermore, the present invention also provides the composition of the present invention for use in a method of treating or preventing a hyperuricemic condition in an individual, the method comprising the step of providing to the individual an effective amount of the composition. Optionally in the composition for use of the present invention, the method comprises providing the composition to an individual who has received, is receiving, and/or will receive another therapy for a hyperuricemic condition, wherein the another therapy is Febuxostat, Allopurinol, or both. It is emphasized that any disclosure relating to a method for treatment of the human or animal body by therapy comprising administration of a composition is to be interpreted as a reference to the composition for use in such a method. Furthermore, to the extent that such a composition for use relates to a method other than a method of treating or preventing a hyperuricemic condition in an individual, then it is disclosed herein purely for context and for better understanding of the composition for use of the present invention.

### BRIEF DESCRIPTION OF THE DRAWINGS

For a more complete understanding of the present disclosure, reference is now made to the following descriptions taken in conjunction with the accompanying drawings.

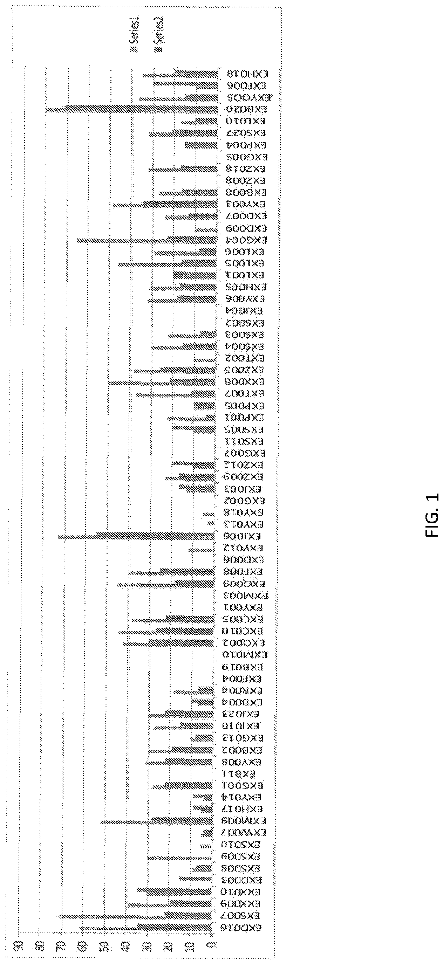

FIG. 1 shows XO inhibitory effects of 72 traditional Chinese herb medicines (standard extracts) in a cell-free system. Each extract was studied in both water-soluble (blue (left of a pair)) and dimethyl sulfoxide (DMSO)-soluble (red (right of a pair)) fractions. Final concentration of each extract is 163 ug/ml). Blue bar: DMSO-soluble fraction; Red bar: water-soluble fraction of each extract.

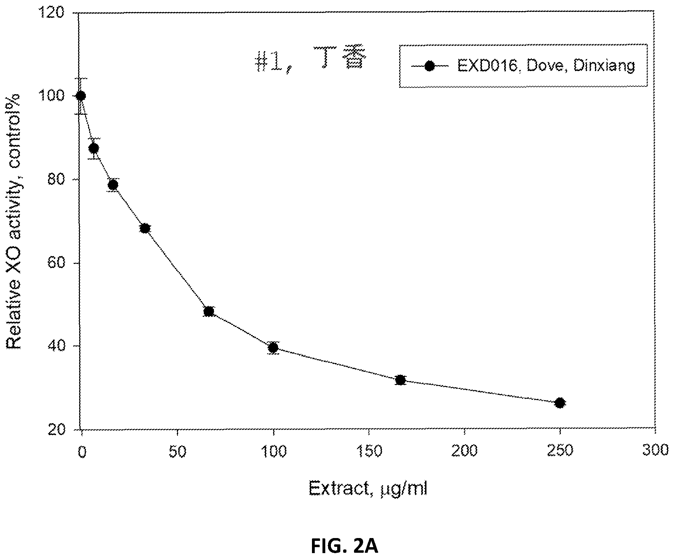

FIGS. 2A-2N demonstrate dose-dependent effects of 14 standard herb extracts on XO inhibition in the cell free system. DMSO-soluble fractions of these extracts were used in this assay.

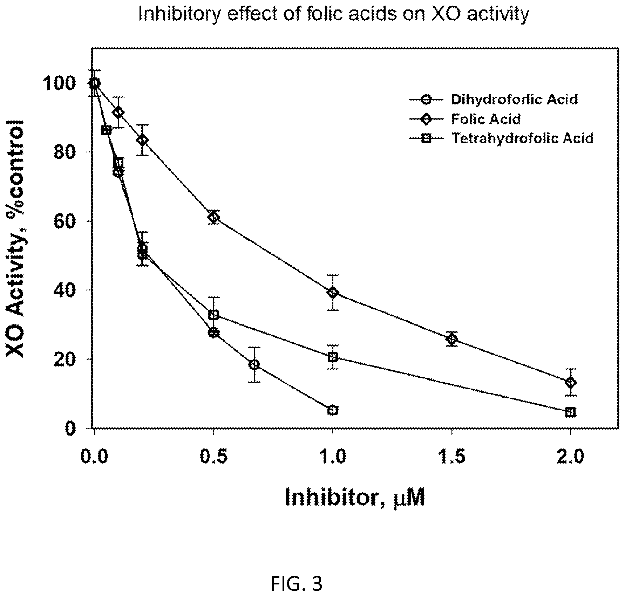

FIG. 3 shows XO inhibitory effects of folic acid and its two derivatives, dihydrofolic acid and tetrahydrofolic acid, in a cell-free system.

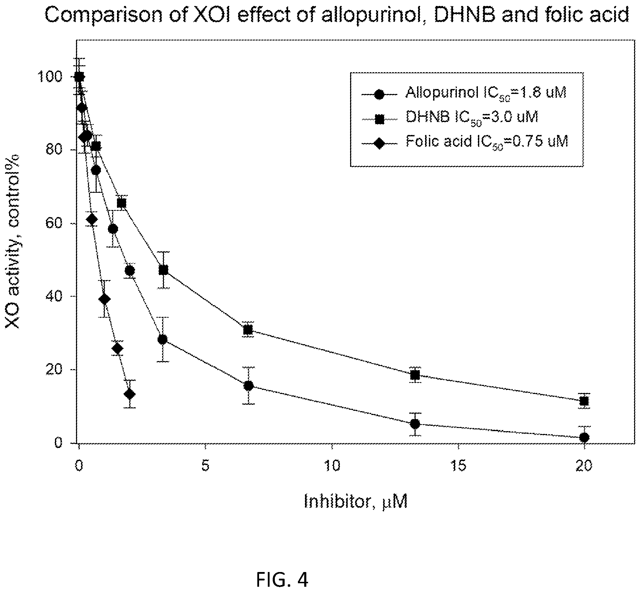

FIG. 4 provides comparison of folic acid, allopurinol and 3,4-Dihydroxy-5-nitrobenzaldehyde (DHNB) on XO inhibition in a cell-free system.

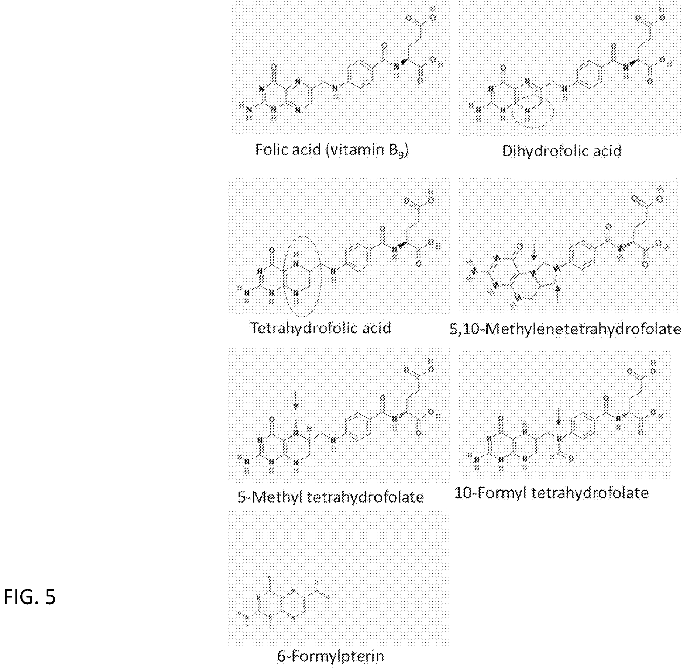

FIG. 5 illustrates the chemical structure of folic acid, dihydrofolic acid (DHF), tetrahydrofolic acid (THF), 5,10-methylenetetrahydrofolate (5,10-MTHFR), 5-methyl tetrahydrofolate (5-MTHF), 10-Formyl tetrahydrofolate (10-FTHF), and 6 -formylpterin.

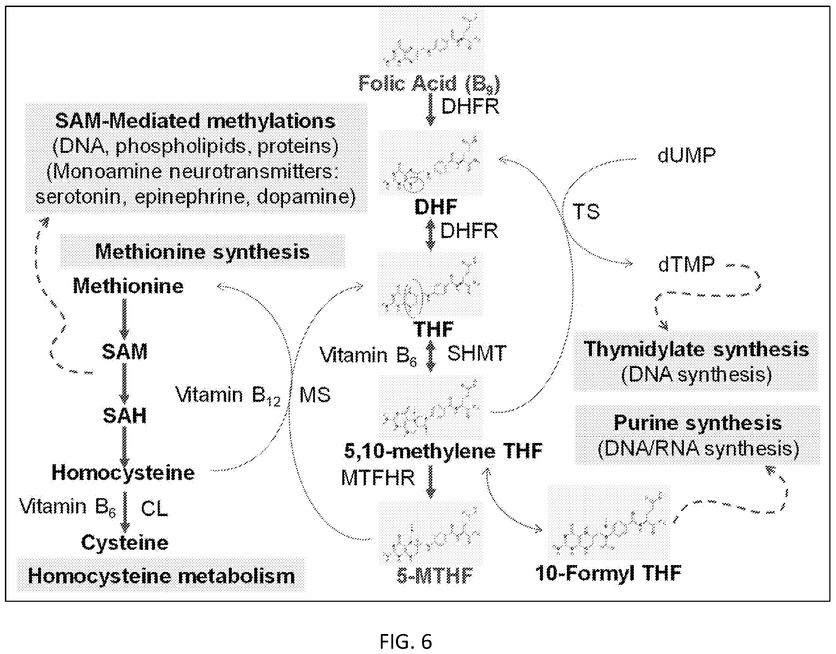

FIG. 6. illustrates that folic acid is a critical vitamin, which have many important biological functions in the body, including DNA and RNA synthesis, methylation of DNA phospholipid, and proteins for functional regulation, homocysteine metabolism for reducing its toxicity and for methionine synthesis for multiple functions. Serine hydroxymethyltransferase (SHMT); Dihydrofolate Reductase (DHFR); Thymidylate synthase (TS); Methylenetetrahydrofolate reductase (MTHFR); Methionine Synthase (MS); S-adenosyl-methionine (SAM).

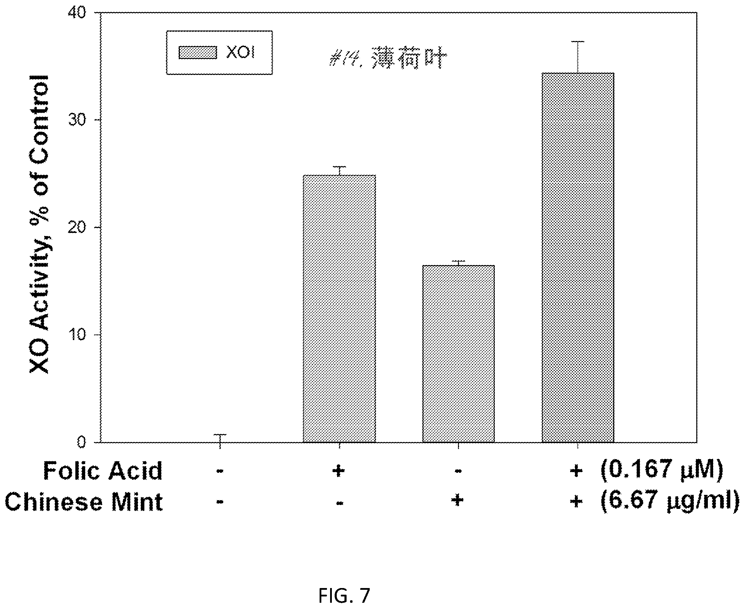

FIG. 7 shows a combination of Chinese mint leave extract with folic acid at relatively small concentrations for XO inhibitory assay. The result shows an additive effect of Chinese mint leave extract with folic acid for XO inhibition. Chinese mint leave extract and folic acid are separately added into XO reaction system, without pre-mixing.

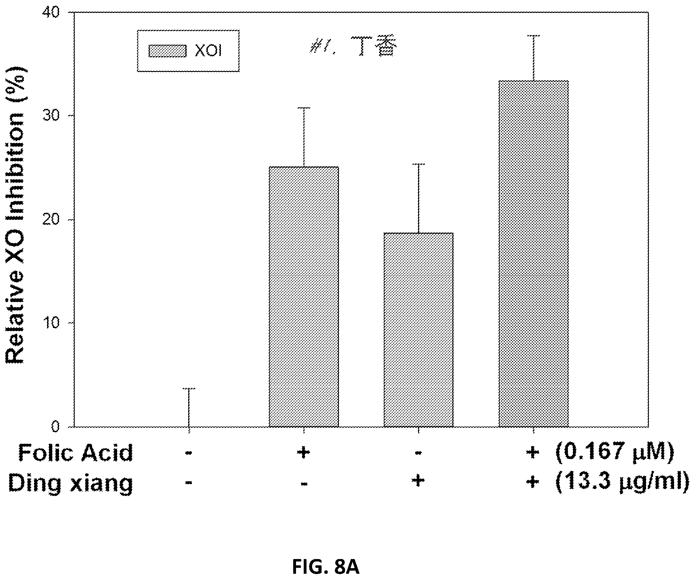

FIGS. 8A-8N show the effect of combination of traditional Chinese medicine herb extracts and folic acid on XO inhibition. All 14 traditional Chinese medicine herb extracts were tested. Folic acid (0.167 uM) and extract (13.3 ug/ml) were separately added into the XO reaction system without pre-mixing. XO activity was measured.

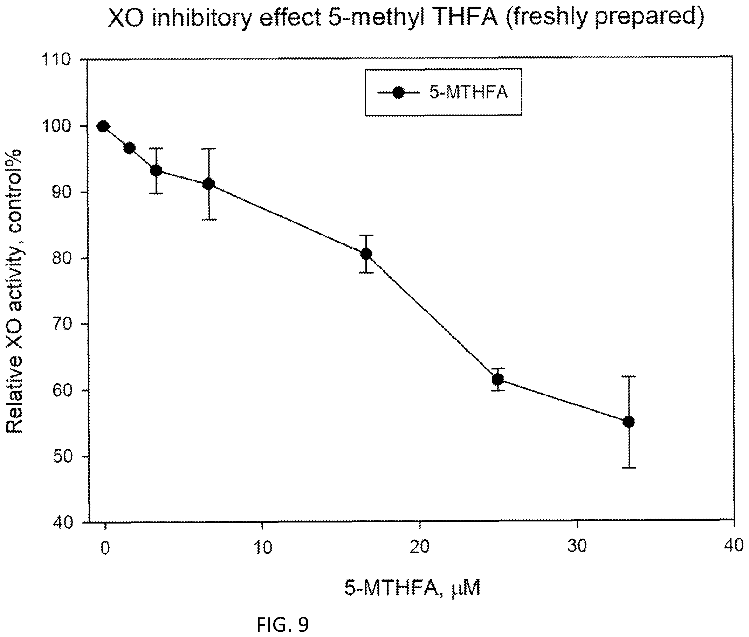

FIG. 9 provides the effect of 5-methyl tetrahydrofolate (5-MTHF) on XO inhibition. Fresh prepared 5-MTHF inhibited XO activities in a concentration-dependent manner. IC₅₀ for 5-MTHF is about 35.6 uM. However, its effect is weaker than folic acid.

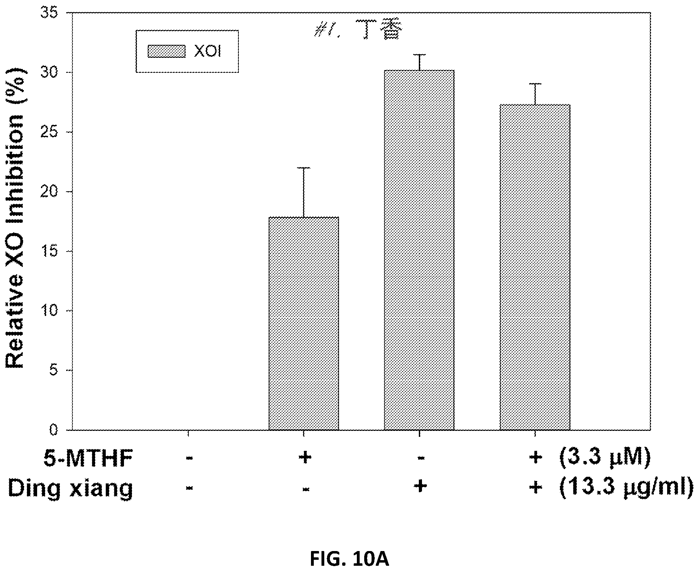

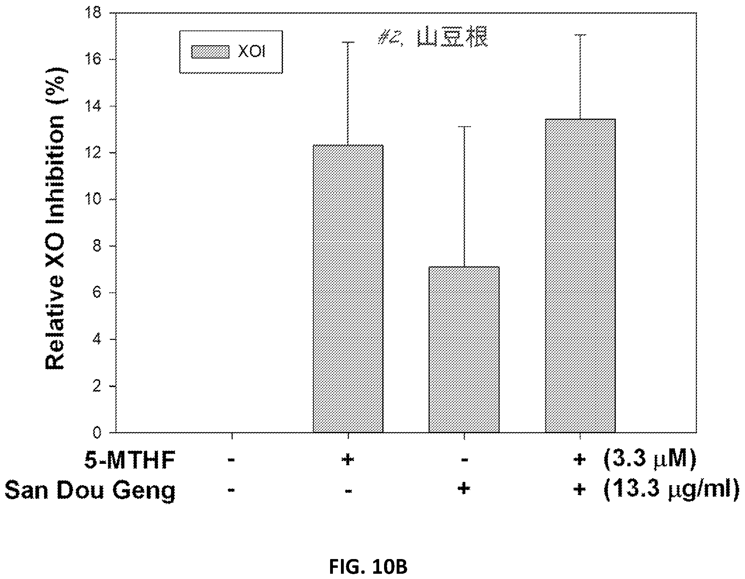

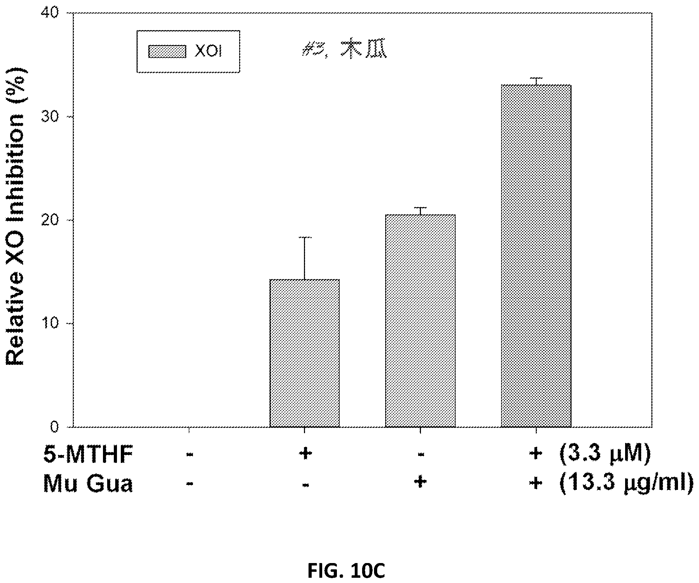

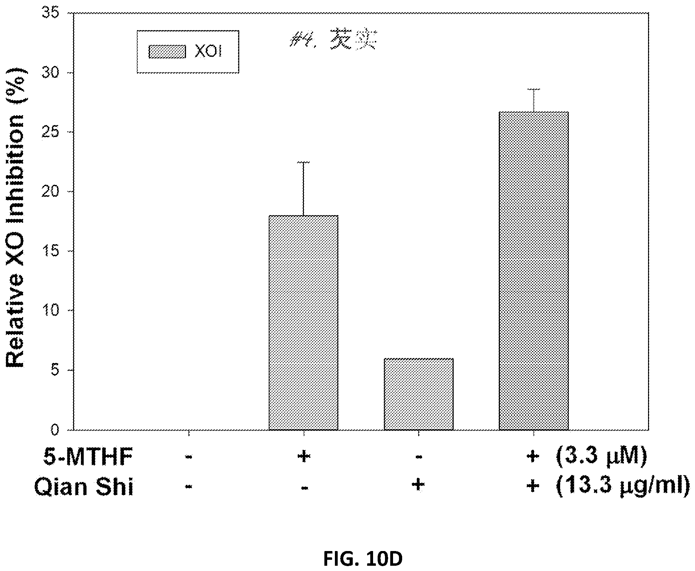

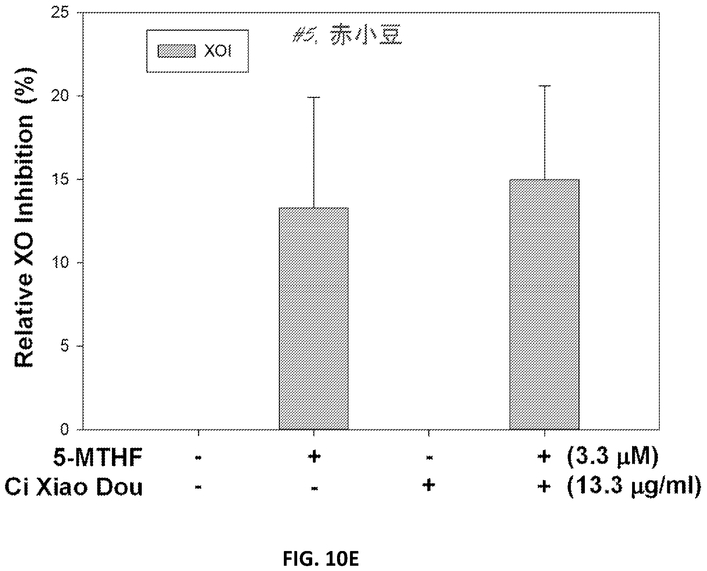

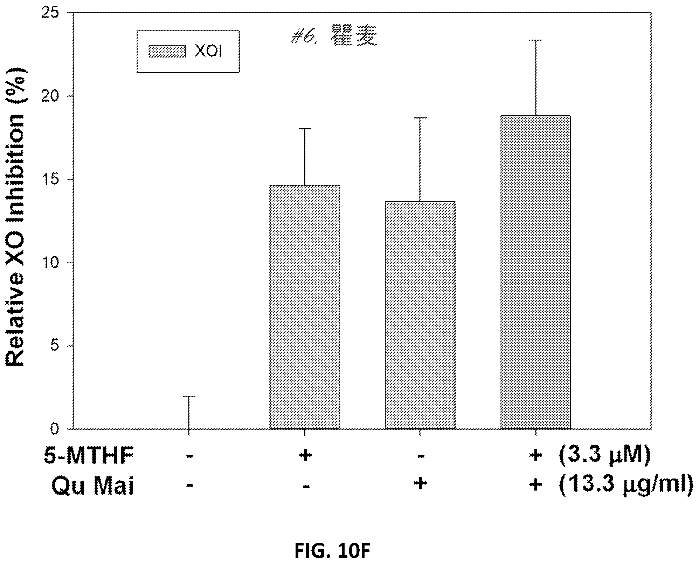

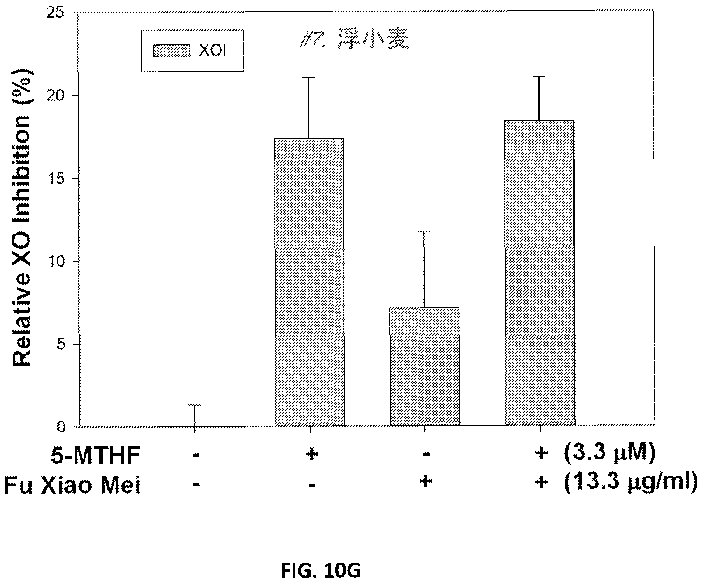

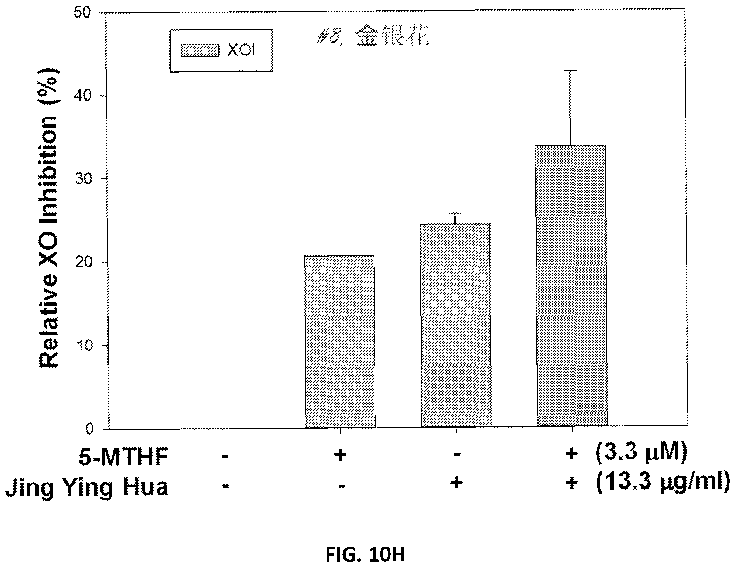

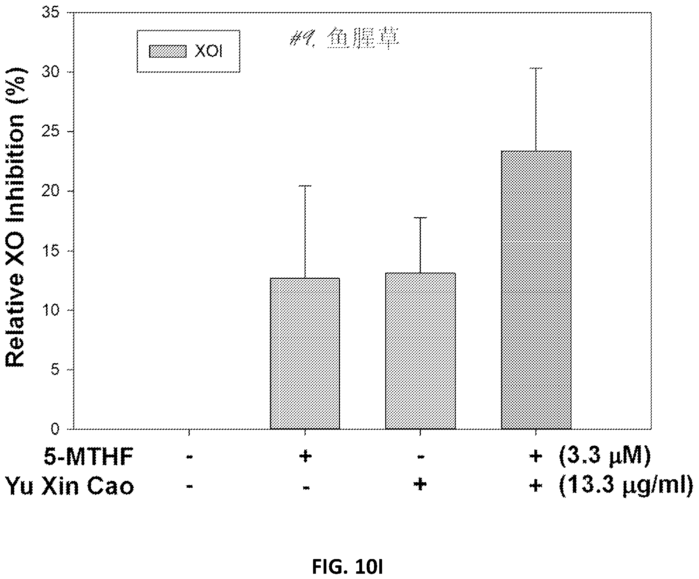

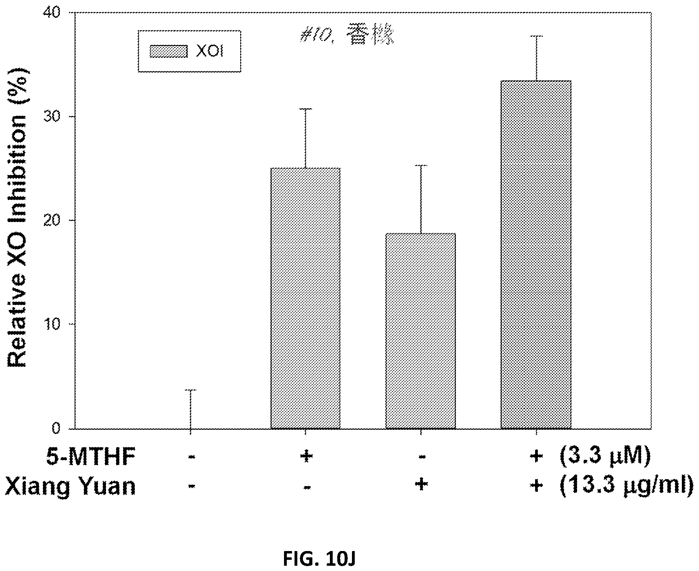

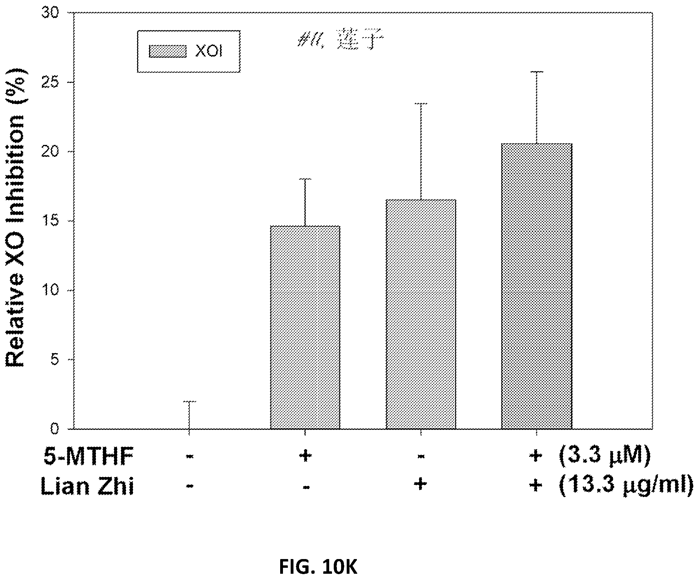

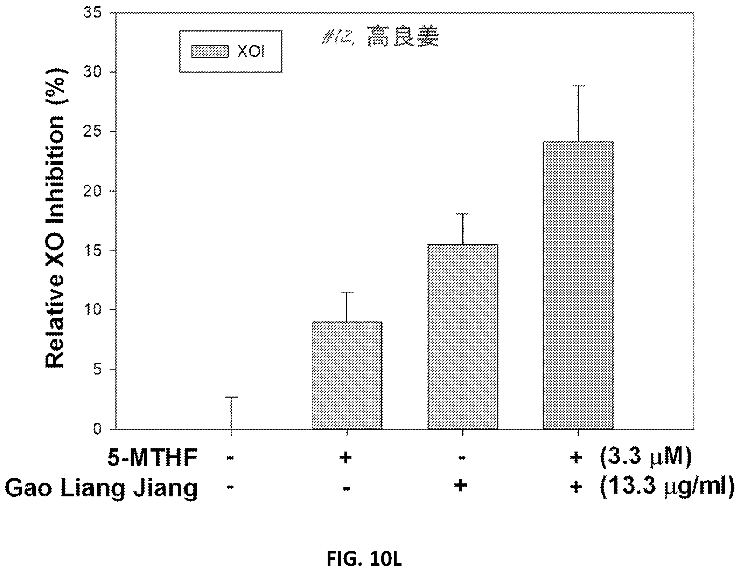

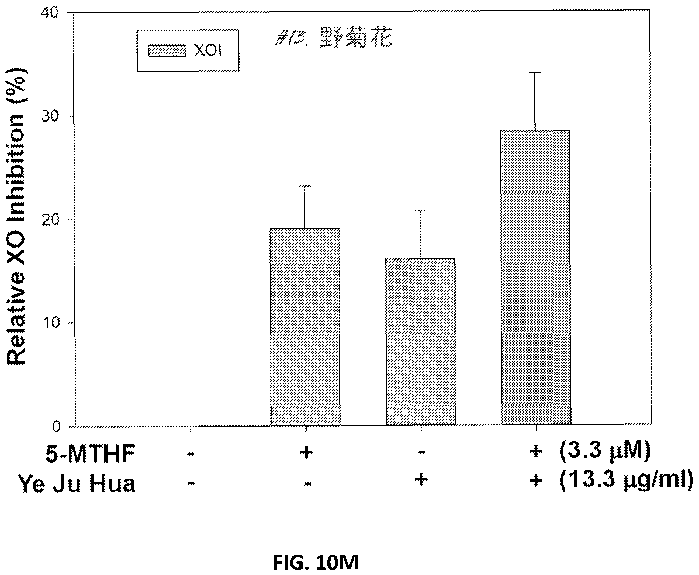

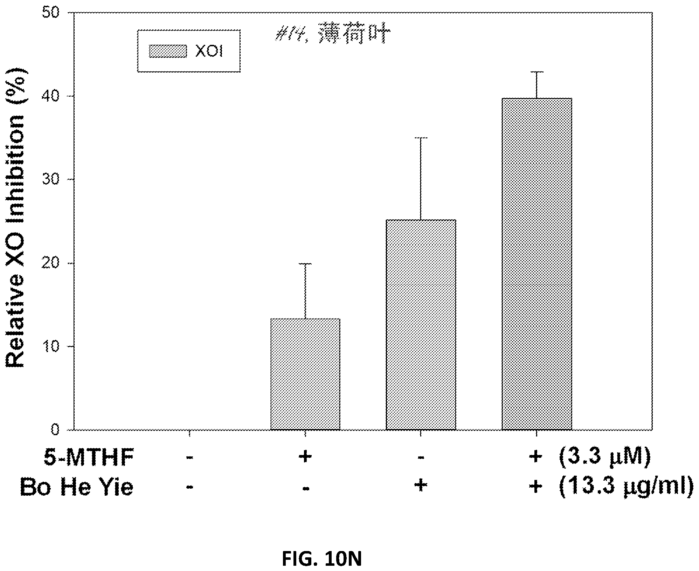

FIGS. 10A-10N demonstrate the effect of combination of 5-MTHF and herb extracts on XO inhibition *in vitro.* Fresh prepared 5-MTHF (3.3 uM) and herb extract (13.3 ug/ml) was added to XO reaction system separately, XO activity was recorded. 5-MTHF had an additive effect on XO inhibition with 10 out of 14 herb extracts: Flowering Quince (Fruit) Extract (Mu Gua 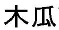 ); Euryale (Seed) Extract (Gian Shi 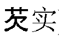 ); Fringed Pink (Aboveground Parts) Extract (Qu Mai, 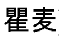 ), Honeysuckle (Flower Bud) Extract (Jing Ying Hua, 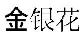 ), Houttuynia (Aboveground Parts) (Yu Xin Cao 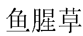 ); Citron (Fruit) Extract (Xiang Yuan 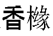 ); Sacred Lotus (Seed) Extract (Lian Zhi, 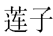 ); Lesser Galangal Extract (Gao Liang Jiang, 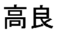 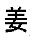 ); Wild Chrysanthemum (Flower) Extract (Ye Ju Hua ( 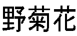 ); and Chinese mint (Bo He Yie, 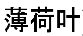 ) (Figure 10), but not with Clove (Flower Bud) Extract (Ding xiang 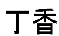 ); Vietnamese Sophora (Root) Extract (San Dou Geng 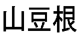 ); Rice Bean Extract (Ci Xiao Dou 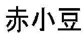 ); and Wheat (Immature Fruit) Extract (Fu Xiao Mei 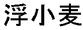 ).

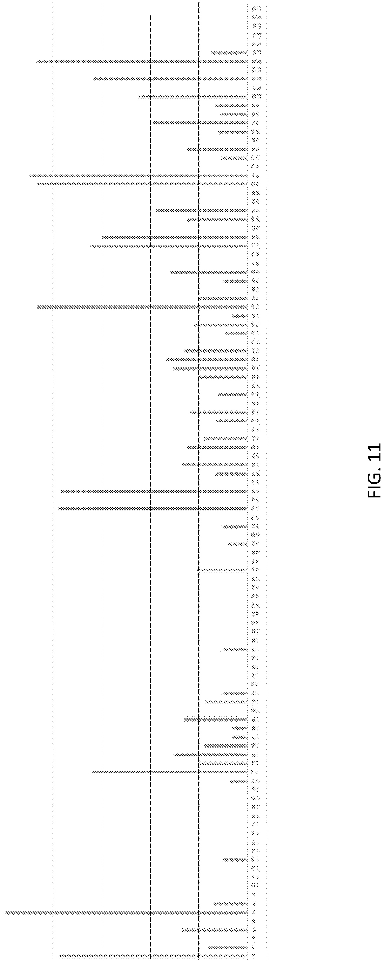

FIG. 11 shows XO inhibitory effects of 112 herbal extracts in a cell-free system. Each extract was studied in a DMSO-soluble fraction. Final concentration of each extract is 166.7 ug/ml) for the majority of extracts; a few extracts at lower concentrations were used due to the solubility issue.

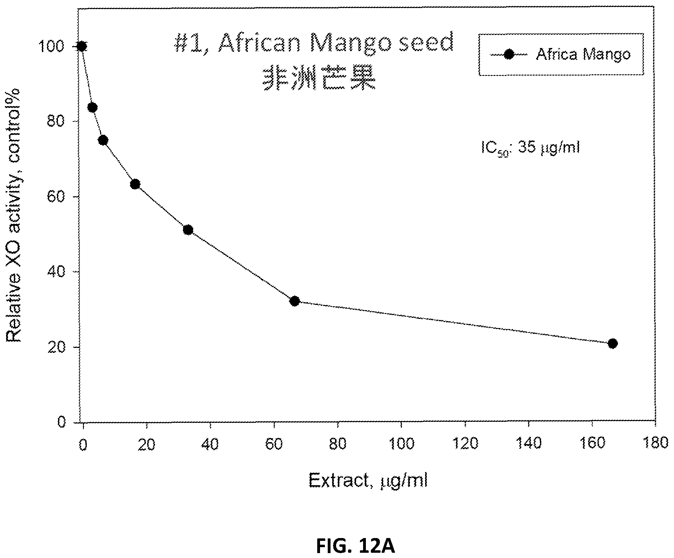

FIGS. 12A-12O demonstrate dose-dependent effects of 15 standard herb extracts on XO inhibition in the cell free system. DMSO-soluble fractions of these extracts were used in this assay.

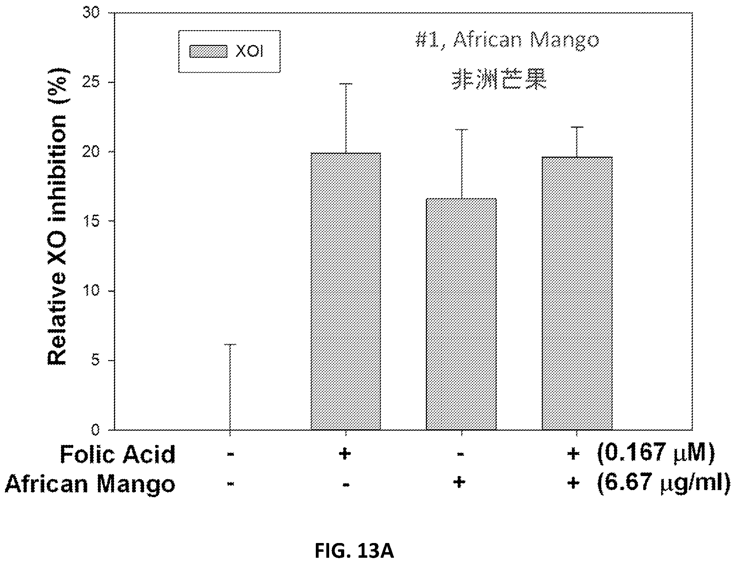

FIGS. 13A-13H provide an effect of combination of selected herbal extracts and folic acid on XO inhibition. Eight herbal extracts were selected and tested. Folic acid (0.167 uM) and each extract at a defined concentration (ug/ml) were separately added into the XO reaction system without pre-mixing. XO activity was measured.

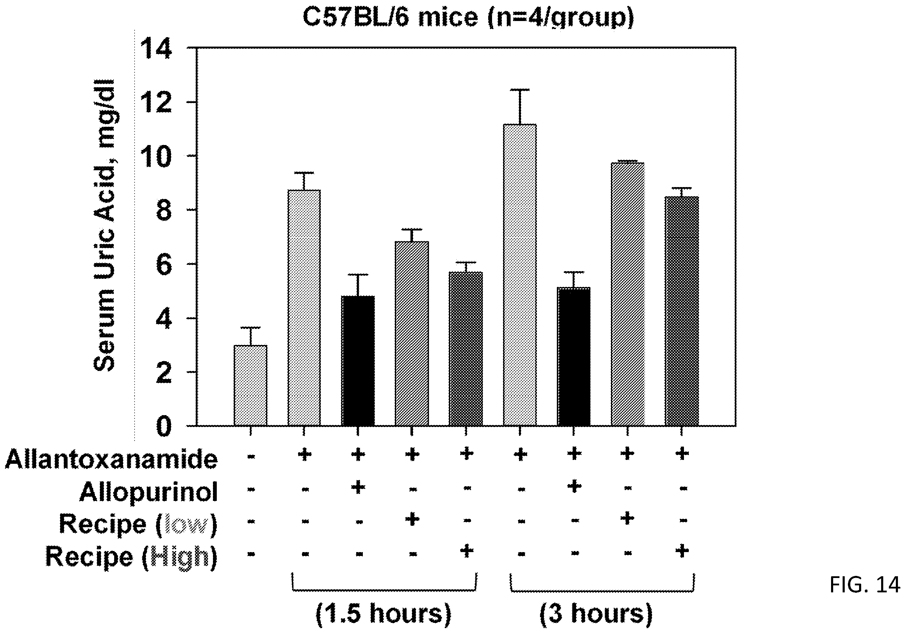

FIG. 14 shows an effect of rationally-designed dietary supplement recipe on reduction of serum uric acid levels in a mouse model. Recipe includes Japanese Thistle Extract ( 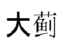 ), Grape Seed Extract ( 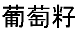 ), Amla Fruit Extract ( 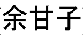 ), Pine Bark Extract ( 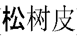 ), Chinese Mint Extract ( 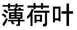 ) and Folic acid. Mice were Intraperitoneally (i.p.)-injected with uricase inhibitor allantoxanamide to induce hyperuricemia in mice. The mice were then oralgavaged with 200 ul to 250 ul (for 20 to 25 g mice) allopurinol (positive control) or dietary health supplement recipe (low dose and high dose). Blood was taken from facial vein at 1.5 hour and 3 hour after the treatment. Serum uric acid level was measured the next day with a phosphotunstate method. N=4 per group.

### DETAILED DESCRIPTION

As used herein the specification, "a" or "an" may mean one or more. As used herein in the claim(s), when used in conjunction with the word "comprising", the words "a" or "an" may mean one or more than one. As used herein "another" may mean at least a second or more. In specific embodiments, aspects of the subject matter may "consist essentially of" or "consist of" one or more elements or steps of the subject matter, for example. Some embodiments of the subject matter may consist of or consist essentially of one or more elements, method steps, and/or methods of the subject matter. It is contemplated that any method or composition described herein can be implemented with respect to any other method or composition described herein.

In keeping with long-standing patent law convention, the words "a" and "an" when used in the present specification in concert with the word comprising, including the claims, denote "one or more." Some embodiments of the disclosure may consist of or consist essentially of one or more elements, method steps, and/or methods of the disclosure. It is contemplated that any method or composition described herein can be implemented with respect to any other method or composition described herein and that different embodiments may be combined.

As used herein, the terms "or" and "and/or" are utilized to describe multiple components in combination or exclusive of one another. For example, "x, y, and/or z" can refer to "x" alone, "y" alone, "z" alone, "x, y, and z," "(x and y) or z," "x or (y and z)," or "x or y or z." It is specifically contemplated that x, y, or z may be specifically excluded from an embodiment.

Throughout this application, the term "about" is used according to its plain and ordinary meaning in the area of cell and molecular biology to indicate that a value includes the standard deviation of error for the device or method being employed to determine the value.

Reference throughout this specification to "one embodiment," "an embodiment," "a particular embodiment," "a related embodiment," "a certain embodiment," "an additional embodiment," or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments.

A variety of aspects of this disclosure can be presented in a range format. It should be understood that the description in range format is merely for convenience and brevity and should not be construed as an inflexible limitation on the scope of the present disclosure. Accordingly, the description of a range should be considered to have specifically disclosed all the possible subranges as well as individual numerical values within that range as if explicitly written out. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. When ranges are present, the ranges may include the range endpoints.

The term "subject," as used herein, may be used interchangeably with the term "individual" and generally refers to an individual in need of a therapy. The subject can be a mammal, such as a human, dog, cat, horse, pig or rodent. The subject can be a patient, e.g., have or be suspected of having or at risk for having a disease or medical condition related to excessive uric acid levels, for example. For subjects having or suspected of having a medical condition directly or indirectly associated with excessive uric acid levels, the medical condition may be of one or more types. The subject may have a disease or be suspected of having the disease. The subject may be asymptomatic. The subject may be of any gender. The subject may be of a certain age, such as at least 30, 35, 40, 45, 50, 55, 60, 65, 70, 75, 80, 85, 90, 95, or 100 or more.

The compositions of the present disclosure may be suitable for the treatment of diseases in a human or animal patient. In one embodiment, the patient is a mammal including a human, horse, dog, cat, sheep, cow, or primate. In one embodiment the patient is a human. In a further embodiment, the patient is not a human. The individual may be receitivng one or more compounds through the world wide web.

As used herein, the term "effective amount" means that amount of a compound, drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

As used herein the term "treatment" refers to defending against or inhibiting a symptom, treating a symptom, delaying the appearance of a symptom, reducing the severity of the development of a symptom, and/or reducing the number or type of symptoms suffered by an individual, as compared to not administering a pharmaceutical composition of the disclosure. The term treatment encompasses the use in a palliative setting

Embodiments of the disclosure include one or more compositions that encompass one or more herb extracts and optionally folic acid and/or one or more derivatives. Any of the elements listed herein may be combined with any one or more other elements as part of a composition. One or more components within any composition may impart an additive or synergistic effect. It is emphasized that the composition of the present invention is, specifically, a composition comprising both (a) Japanese Thistle extract and (b) one or more of: Grape Seed Extract; Amla Fruit Extract; Pine Bark Extract; and Chinese Mint Extract.

In specific embodiments, the composition(s) can be utilized for treating or preventing a variety of medical conditions, including those in which it would be beneficial to reduce xanthine oxidase (XO) activity. Examples of medical conditions include one or more of direct or indirect hyperuricemic conditions, such as gout, hypertension, atherosclerosis, coronary artery disease, heart failure, left ventricular hypertrophy, atrial fibrillation, periphery artery disease, vascular restenosis, vascular thrombosis, stroke, diabetes, insulin resistance, metabolic syndrome, chronic kidney disease, psoriatic arthritis, micro-albuminuria, erectile dysfunction, preeclampsia, cancer (of any kind), immune disorders, or an inflammatory disease. When the medical condition is cancer, it may be of the lung, breast, brain, liver, colon, skin, stomach, prostate, uterus, endometrium, ovary, testes, bone, spleen, thyroid, blood, gall bladder, kidney, and so forth. It is emphasized that the composition for use of the present invention is use in a method of treating or preventing a hyperuricemic condition in an individual.

In some embodiments, there is a composition comprising Euryale (Seed) Extract; Rice Bean Extract; Wheat (Immature Fruit) Extract; Houttuynia (Aboveground Parts) Extract; Citron (Fruit) Extract; Sacred Lotus (Seed) Extract; Chinese Mint Extract; Fennel (Fruit) Extract; Canadian Thistle (Aboveground Parts) Extract; White Hyacinth Bean Extract; Sea-ear Shell Extract; Fructus Hordei Germinatus Extract; Sterculia (Seed) Extract; Haizhou Elsholtzia (Aboveground Parts) Extract; Sharp-Leaf Galangal (Seed) Extract; Radish (Seed) Extract; Sacred Lotus (Plumule) Extract; Fermented Soybean Extract; Jujube (Seed) Extract; Butcher's Broom; Oolong Tea (leaf) Extract; Senna Leaf Extract; Yohimbe bark Extract; Japanese Thistle extract; Butterbur Extract; Caralluma bark (wild) Extract; Echinacea root Extract; Horse Chestnut fruit Extract; Kola Nut Extract; Magnolia Bark Extract; Muira Puama Bark Extract; or a combination thereof. In such cases, the composition may be formulated as a pharmaceutical, medicinal food, supplement, food, food supplement, nutritional or dietary supplement, food for special dietary use, or medical food, or combination thereof.

Any composition of the disclosure may include one or more of Clove (Flower Bud) Extract; Vietnamese Sophora (Root) Extract; Flowering Quince (Fruit) Extract; Fringed Pink (Aboveground Parts) Extract; Honeysuckle (Flower Bud) Extract; Lesser Galangal Extract (*Alpinia officinarum)*; or Wild Chrysanthemum (Flower) Extract; Fructose Crataegi (Charred) Extract; Chinese Licorice (Root & Rhizome) Extract; Ginkgo Leaf Extract; Sickle-pod Senna (Seed) Extract; Fructus Aurantii Processed Extract; Bitter Orange (Young Fruit) Extract; Chinese Smilax (Rhizome) Extract; White Mulberry (Young Twig) Extract; White Mulberry (Leaf) Extract; Sacred Lotus (Leaf) Extract; Chrysanthemum (Flower) Extract; Perilla (Leaf) Extract; Job's Tears Extract; Palm-leaf Raspberry Extract; or Patchouli (Aboveground Parts) Extract; African Mango seed Extract; Amla Fruit Powder; Grape Seed Extract; Green Tea Pure Extract; Milk Thistle Extract; Olive Leaf Extract; Pine Bark; Pomegranate Extract; Spearmint Leaf Extract; St John's Wort Extract; Aloe Vera Leaf Extract; Catuaba bark Bark Extract; Gynostemma Extract; Hawthorn Leaf Extract; Kudzu Root Extract; Lemon Balm Extract; Licorice Root Extract; Moringa Extract; Papaya Fruit Extract; Papaya Seed; Red Clover (steamleaf) Extract; Rosemary Extract; or a combination thereof.

In particular embodiments, the composition comprises Euryale (Seed) Extract; Rice Bean Extract; Wheat (Immature Fruit) Extract; Houttuynia (Aboveground Parts) Extract; Citron (Fruit) Extract; Sacred Lotus (Seed) Extract; Chinese Mint Extract; Butcher's Broom Extract; Oolong Tea (leaf) Extract; Senna Leaf Extract; Yohimbe bark Extract; Japanese Thistle extract; Butterbur Extract; Caralluma bark (wild) Extract; Echinacea root Extract; Horse Chestnut fruit Extract; Kola Nut Extract; Magnolia Bark Extract; Muira Puama Bark Extract; or a combination thereof. In specific cases, the composition comprises Euryale (Seed) Extract and one or more other compounds. In specific cases, the composition comprises Rice Bean Extract and one or more other compounds. In specific cases, the composition comprises Wheat (Immature Fruit) Extract and one or more other compounds. In specific cases, the composition comprises Houttuynia (Aboveground Parts) Extract and one or more other compounds. In specific cases, the composition comprises Citron (Fruit) Extract and one or more other compounds. In specific cases, the composition comprises Sacred Lotus (Seed) Extract and one or more other compounds. In specific cases, the composition comprises Chinese Mint Extract and one or more other compounds. In specific cases, the composition comprises Butcher's Broom Extract and one or more other compounds. In specific cases, the composition comprises Oolong Tea (leaf) Extract and one or more other compounds. In specific cases, the composition comprises Senna Leaf Extract and one or more other compounds. In specific cases, the composition comprises Yohimbe bark Extract and one or more other compounds. In specific cases, including the specific composition of the present invention, the composition comprises Japanese Thistle extract and one or more other compounds. In specific cases, the composition comprises Butterbur Extract and one or more other compounds. In specific cases, the composition comprises Caralluma bark (wild) Extract and one or more other compounds. In specific cases, the composition comprises Caralluma bark (wild) Extract and one or more other compounds. In specific cases, the composition comprises Caralluma bark (wild) Extract and one or more other compounds. In specific cases, the composition comprises Echinacea root Extract and one or more other compounds. In specific cases, the composition comprises Horse Chestnut fruit Extract and one or more other compounds. In specific cases, the composition comprises Kola Nut Extract and one or more other compounds. In specific cases, the composition comprises Magnolia Bark Extract and one or more other compounds. In specific cases, the composition comprises s. Muira Puama Bark Extract and one or more other compound.

In particular embodiments, the composition comprises 1, 2, 3, 4, 5, 6, 7, 8, 9, 10, 11, 12, 13, 14, 15, 16, 17, or all of Euryale (Seed) Extract; Rice Bean Extract; Wheat (Immature Fruit) Extract; Houttuynia (Aboveground Parts) Extract; Citron (Fruit) Extract; Sacred Lotus (Seed) Extract; Chinese Mint Extract; Butcher's Broom Extract; Oolong Tea (leaf) Extract; Senna Leaf Extract; Yohimbe bark Extract; Japanese Thistle extract; Butterbur Extract; Caralluma bark (wild) Extract; Echinacea root Extract; Horse Chestnut fruit Extract; Kola Nut Extract; Magnolia Bark Extract; and Muira Puama Bark Extract.

In some embodiments, the composition comprises Lesser Galangal Extract (*Alpinia officinarum),* Honeysuckle (Flower Bud) Extract, Chinese Mint Extract, Clove (Flower Bud) Extract, Citron (Fruit) Extract or a combination thereof. In specific cases, the composition comprises Lesser Galangal Extract (*Alpinia officinarum*) and one or more other compounds. In specific cases, the composition comprises Honeysuckle (Flower Bud) and one or more other compounds. In specific cases, the composition comprises Chinese Mint Extract and one or more other compounds. In specific cases, the composition comprises Clove (Flower Bud) Extract and one or more other compounds. In specific cases, the composition comprises Citron (Fruit) Extract and one or more other compounds. In particular embodiments, the composition comprises 1, 2, 3, or all of Lesser Galangal Extract (*Alpinia officinarum)*, Honeysuckle (Flower Bud) Extract; Chinese Mint Extract; Clove (Flower Bud) Extract; and Citron (Fruit) Extract.

In specific embodiments, the composition comprises Japanese thistle extract; Grape seeds extract; Amla extract; Pine bark extract; Chinese mint extract; or a combination thereof. In specific cases, including the specific composition of the present invention, the composition comprises Japanese thistle extract and one or more other compounds. In specific cases, the composition comprises Grape seeds extract and one or more other compounds. In specific cases, the composition comprises Amla extract and one or more other compounds. In specific cases, the composition comprises Pine bark extract and one or more other compounds. In specific cases, the composition comprises Chinese mint extract and one or more other compounds. In particular embodiments, the composition comprises 1, 2, 3, 4, or all of Japanese thistle extract; Grape seeds extract; Amla extract; Pine bark extract; and Chinese mint extract. In specific embodiments, the composition comprises at least Japanese thistle extract and Grape seeds extract.

In some embodiments, one or more compositions are included with folic acid and/or one or more derivatives thereof. One can utilize folic acid and its one or more of its three derivatives, dihydrofolic acid, tetrahydrofolic acid and 5-methyl tetrahydrofolate (5-MTHF) in employing the XO inhibition assay. IC₅₀ for folic acid and 5-MTHF are 0.75 uM and 35.6 uM, respectively. Dihydrofolic acid and tetrahydrofolic acid are more potent than folic acid, in at least some embodiments. Importantly, the XO inhibitory effect was studied of combinations of single herb medicine extract with folic acid or 5-MTHF. Nine out of 14 extracts tested showed an additive XO inhibitory effect with folic acid; and 10 out of 14 extracts tested showed an additive XO inhibitory effect with 5-MTHF.

One can characterize the additive or synergistic effect of different combinations of extracts and folic acid or 5-MTHF at different experimental conditions. One can select a few lead combination formulations of herb extracts with or without folic acid or 5-MTHF and determine those that have additive and/or synergistic effects on XO inhibitions, in some cases while dose of each component in the combination is relatively small. These lead combination recipes may be characterized in animal model tests and ultimately for the treatment and/prevention of patients with both symptomatic and asymptomatically hyperuricemia conditions, which are a significant and independent risk factor for cardiovascular disease and many other diseases. In addition, these herb extracts/folic acid-based health supplements also have antioxidant capability, reduce oxidative stress, and decrease serum homocysteine levels, thereby providing multiple health benefits.

### I. Pharmaceutical and Other Compositions

In accordance with this disclosure, the term "composition" or "pharmaceutical composition" relates to a composition for administration to an individual. In a particular embodiment, the pharmaceutical or other composition comprises a composition for parenteral, transdermal, intraluminal, intra-arterial, intrathecal or intravenous administration, for example. It is in particular envisaged that the pharmaceutical composition is to be administered to the individual orally. Administration of the suitable composition(s) may be effected by different ways, e.g., by oral, intravenous, subcutaneous, intraperitoneal, intramuscular, topical, intradermal administration, *via* infusion, injection, or rectal, including as using suppositories, for example.

The pharmaceutical or other composition of the present disclosure may further comprise an acceptable carrier, such as a pharmaceutically acceptable carrier. Examples of suitable carriers are well known in the art and include phosphate buffered saline solutions, water, emulsions, such as oil/water emulsions, various types of wetting agents, sterile solutions, *etc.* Compositions comprising such carriers can be formulated by well-known conventional methods. These compositions can be administered to the subject at a suitable dose.

The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A preferred dosage for administration might be in the range of 0.24 µg to 48 mg, preferably 0.24 µg to 24 mg, more preferably 0.24 µg to 2.4 mg, even more preferably 0.24 µg to 1.2 mg and most preferably 0.24 µg to 240 mg units per kilogram of body weight per day. Progress can be monitored by periodic assessment.

The compositions of the disclosure may be administered locally or systemically. Administration will generally be parenteral, *e.g*., intravenous; DNA may also be administered directly to the target site, *e.g*., by biolistic delivery to an internal or external target site or by catheter to a site in an artery. In a particular embodiment, the pharmaceutical composition is administered subcutaneously and in an even more preferred embodiment intravenously. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishes, electrolyte replenishers (such as those based on Ringer's dextrose), and the like. Preservatives and other additives may also be present such as, for example, antimicrobials, anti-oxidants, chelating agents, and inert gases and the like. In addition, the pharmaceutical composition of the present disclosure might comprise proteinaceous carriers, like, *e.g*., serum albumin or immunoglobulin, preferably of human origin. It is envisaged that the pharmaceutical composition of the disclosure might comprise, in addition to the proteinaceous bispecific single chain antibody constructs or nucleic acid molecules or vectors encoding the same (as described in this disclosure), further biologically active agents, depending on the intended use of the composition.

In some embodiments, any of the compositions encompassed herein may be to be consumed or otherwise utilized by an individual once or more than once. When the composition is to be consumed or otherwise utilized more than once, the duration between administrations may be on the order of 1-24 hours, 1-7 days, 1-4weeks, or 1-12 months or more. The individual may or may not be consuming or utilizing the compositions following a diagnosis indicating the individual was in need thereof. In particular aspects, the individual is diagnosed as having excessive uric acid levels, for example with respect to the general population or as a control, and the compositions are then consumed or utilized thereafter. The individual may obtain the compositions through commercial sources, including through the world wide web.

When there is more than one element (for example, type of extract) listed herein comprised within a composition as encompassed herein, the elements may or may not be in a specific ratio. For example, in some cases two different elements of the composition may be formulated within the composition to be in a 1:1 ratio, a 1:2 ratio, a 1:5 ratio, a 1:10 ratio, a 1:25 ratio, a 1:50 ratio, a 1:100 ratio, a 1:500 ratio, a 1:1000 ratio, a 1:10000 ratio and so forth. In cases wherein the composition comprises three elements (for example, types of extract), the ratio may be a 1:1:1 ratio, 1:2:1 ratio, 1:5:1 ratio, a 1:10:1 ratio, a 1:50:1 ratio, a 1:100:1 ratio, a 1:500:1 ratio, a 1:1000:1 ratio, a 1:1:2 ratio, a 1:1:5 ratio, a 1:1:10 ratio, a 1:1:50 ratio, a 1:1:100 ratio, a 1:1:500 ratio, a 1:1:1000 ratio, a 2:1:1 ratio, a 5:1:1 ratio, a 10:1:1 ratio, a 50:1:1 ratio, a 100:1:1 ratio, a 500:1:1 ratio, a 1000:1:1 ratio, and so forth.

In some embodiments, the concentration of one or more particular elements (for example, type of extract) may be within a range. For example, the one or more extracts may be within a range of concentration of 0.1 to 100 mg/kg in the composition. In specific embodiments, the one or more extracts may be within a range of concentration of 0.1 to 100 mg/kg; 0.1 to 75 mg/kg; 0.1 to 50 mg/kg; 0.1 to 25 mg/kg; 0.1 to 10 mg/kg/ 0.1 to 5 mg/kg; 0.1 to 1 mg/kg; 0.1 to 0.75 mg/kg/ 0.1 to 0.5 mg/kg; 0.5 to 100 mg/kg; 0.5 to 75 mg/kg; 0.5 to 50 mg/kg; 0.5 to 25 mg/kg; 0.5 to 10 mg/kg; 0.5 to 5 mg/kg; 0.5 to 1 mg/kg; 1 to 100 mg/kg; 1 to 75 mg/kg; 1 to 50 mg/kg; 1 to 25 mg/kg; 1 to 10 mg/kg; 1 to 5 mg/kg; 5 to 100 mg/kg; 5 to 75 mg/kg; 5 to 50 mg/kg; 5 to 25 mg/kg; 5 to 10 mg/kg; 10 to 100 mg/kg/ 10 to 75 mg/kg; 10 to 50 mg/kg/ 10 to 25 mg/kg; 25 to 100 mg/kg; 25 to 75 mg/kg; 25 to 50 mg/kg; 50 to 100 mg/kg; 50 to 75 mg/kg; or 75 to 100 mg/kg in the composition, as examples.

Any of the compositions described herein may be comprised in a kit. In a nonlimiting example, one or more components of the composition(s) and/or the reagents to extract the herbs may be comprised in a kit. The kit components are provided in suitable container means.

Some components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits also will typically include a means for containing the components in close confinement for commercial sale. Such containers may include injection or blow molded plastic containers into which the desired vials are retained.

When the components of the kit are provided in one and/or more liquid solutions, the liquid solution is an aqueous solution, with a sterile aqueous solution being particularly useful. In some cases, the container means may itself be a syringe, pipette, and/or other such like apparatus, from which the formulation may be applied to an infected area of the body, injected into an animal, and/or even applied to and/or mixed with the other components of the kit.

However, the components of the kit may be provided as dried powder(s). When reagents and/or components are provided as a dry powder, the powder can be reconstituted by the addition of a suitable solvent. It is envisioned that the solvent may also be provided in another container means. The kits may also comprise a second container means for containing a sterile, pharmaceutically acceptable buffer and/or other diluent.

In particular embodiments, the compositions are provided in a kit, and in some cases the compositions are essentially the sole component of the kit. The kit may comprise reagents and materials to make the desired extract or composition. In particular embodiments, there are one or more apparatuses in the kit suitable for extracting one or more samples from an individual. The apparatus may be a syringe, scalpel, and so forth.

In some cases, the kit, in addition to disclosed embodiments, also includes a second therapy, such as one or more uricosurics and/or one or more xanthine oxidase inhibitors other than those described in the disclosure, for example.

### II. Dietary or Health Food Supplements

Any herbal components and non-herbal components of this disclosure can be used in the form of a dietary supplement or health food supplement or as a medicinal preparation, for example, in solid, semi-solid or liquid form that comprises the composition of the present disclosure, as an active ingredient, including in at least some cases in admixture with an organic or inorganic carrier or excipient suitable for external, enteral or parenteral applications. The active ingredient(s) may be compounded, for example, with the usual non-toxic pharmaceutically acceptable carriers for tablets, pellets, capsules, suppositories, solutions, emulsions, suspensions, and any other form suitable for use. Formulations of the present disclosure encompass those that include an exemplified carrier, including carriers such as water, glucose, lactose, gum acacia, gelatin, mannitol, starch paste, magnesium trisilicate, corn starch, keratin, colloidal silica, potato starch, urea and other carriers suitable for use in manufacturing preparations, in solid, semisolid or liquid form and in addition auxiliary, stabilizing, thickening and coloring agents and perfumes may be used.

For preparing solid compositions such as tablets or capsules, the principal active ingredient(s) may be mixed with a carrier (*e.g*., conventional tableting ingredients such as corn starch, lactose, sucrose, sorbitol, talc, stearic acid, magnesium stearate, dicalcium phosphate or gums) and other diluents (*e.g*., water) to form a solid preformulation composition comprising a substantially homogeneous mixture of one or more compositions of the present disclosure, or a non-toxic pharmaceutically acceptable salt thereof. In cases when referring to the preformulation compositions as substantially homogenous, it is meant that the active ingredients are dispersed evenly throughout the composition so that the composition may be readily subdivided into equally effective unit dosage forms such as tablets, pills and capsules. This solid preformulation composition may then be subdivided into unit dosage forms of the type described above containing 0.4 mg of the composition of the present disclosure, for example in capsules. The tablets or pills of the novel composition can be coated or otherwise compounded to provide a dosage form affording the advantage of prolonged action, in some cases. For example, the tablet or pill can comprise an inner dosage an outer dosage component, the latter being in the form of an envelope over the former. In some cases, the components can be separated by an enteric layer that serves to resist disintegration in the stomach and permits the inner component to pass intact into the duodenum or to be delayed in release. A variety of materials can be used for such enteric layers or coatings such materials, including a number of polymeric acids and mixtures of polymeric acids with such materials as shellac, cetyl alcohol and cellulose acetate.

In cases wherein liquid forms are utilized, in which the novel composition of the present disclosure may be incorporated for administration orally or by injection, for example, they may include an aqueous solution, suitably flavored syrups, aqueous or oil suspensions, and flavored emulsions with edible oils such as cottonseed oil, sesame oil, coconut oil, or peanut oil as well as elixirs and similar pharmaceutical vehicles. Suitable dispersing or suspending agents for aqueous suspensions include synthetic natural gums, such as tragacanth, acacia, alginate, dextran, sodium carboxymethyl cellulose, methylcellulose, polyvinylpyrrolidone or gelatin.

In one embodiment, the composition is formulated as a powder to be mixed with a liquid, such as with a drink or broth.

Liquid preparations for oral administration may take the form of, for example, solutions, syrups or suspensions, or they may be presented as a dry product for reconstitution with water or other suitable vehicles before use. Such liquid preparations may be prepared by conventional means with pharmaceutically acceptable additives such as suspending agents (*e.g*., sorbitol syrup, methyl cellulose or hydrogenated edible fats); emulsifying agents (*e.g*., lecithin or acacia); non-aqueous vehicles (*e.g*., almond oil, oily esters or ethyl alcohol); preservatives (*e.g*., methyl or propyl p-hydroxybenzoates or sorbic acid); and artificial or natural colors and/or sweeteners.

For buccal administration, the composition may take the form of tablets or lozenges formulated in conventional manners.

The active compounds may be formulated for parenteral administration by injection, which includes using conventional catheterization techniques or infusion. Formulations for injection may be presented in unit dosage form, *e.g.,* in ampules, or in multi-dose containers, with an added preservative. The composition(s) may take such forms as suspensions, solutions or emulsions in oily or aqueous vehicles, and may contain formulating agents such as suspending, stabilizing, and/or dispersing agents. Alternatively, the active ingredient(s) may be in powder form for reconstitution with a suitable vehicle, *e.g.*, sterile pyrogen-free water, before use.

### III. Methods of Preventing or Treating a Medical Condition

In particular embodiments, a medical condition is to be prevented or treated using one or more compositions of the disclosure. A medical condition may, in general, be of any kind, and the medical condition may or may have not been diagnosed by a medical practitioner of any kind, including of Eastern or Western medicine. In specific embodiments, the medical condition is one that is directly or indirectly related to oxidative stress. The medical condition may be related to excessive levels of uric acid in the individual, such as compared to a standard or the general population. The compositions and methods of the disclosure may completely inhibit the medical condition, delay the onset of the medical condition or reduce the severity of one or more symptoms of the medical condition. The medical condition may be gout, hypertension, atherosclerosis, coronary artery disease, heart failure, left ventricular hypertrophy, atrial fibrillation, periphery artery disease, vascular restenosis, vascular thrombosis, stroke, diabetes, insulin resistance, metabolic syndrome, chronic kidney disease, psoriatic arthritis, micro-albuminuria, erectile dysfunction, preeclampsia, cancers, immune disorders, or an inflammatory disease. In the composition for use of the present invention, the use is in a method of treating or preventing a hyperuricemic condition in an individual.

In particular embodiments, the composition(s) may be provided to an individual in need thereof once or more than once. When provided more than once, the duration in time between administrations may be of any duration such as 1-60 minutes, -24, days, 1-4 weeks or 1-12 or more months. The dosage with multiple administrations may or may not be the same.

### EXAMPLES

The following examples are presented in order to more fully illustrate the preferred embodiments of the disclosure.

### EXAMPLE 1 (REFERENCE EXAMPLE)

### EXAMPLES OF MATERIALS AND METHODS

The inventors have carefully selected and ordered standard extracts from 72 traditional Chinese herb medicines (Table 1).

All 72 herb extracts (100 g) are in the powder form. Each extract was dissolved in water or DMSO respectively with strong votex, at stock concentration of 50 mg/ml. The solution was kept at room temperature for 30 min before centrifugation at 14k rpm. The clear solution was used for assay of XO inhibitory activity. XO activity was determined using the method of continuous spectrophotometric rate measurements (60). The reaction mixture contained xanthine in 67 mM phosphate buffer (pH 7.4) and 20 nM XO with an activity of 5 uU/mL, with or without extract solution. After pre-incubating the extract (at 0-10 ul) with XO for 1 min at 25°C, 50 uM xanthine was added to initiate the formation of uric acid, and the increase of absorption of uric acid at 295 nm was monitored in a dynamic mode. Initial rate of XO activity was recorded. 0-10 ul of DMSO was assayed and found that it has no inhibitory effect on XO activity. For initial screen of herb extracts, final concentration of each extract was 163 ug/ml. Percentage of inhibition is calculated: Percentage of inhibition = (1 - test OD/blank OD) x 100

Folic acid, dihydrofolic acid and tetrahydrofolic acid were purchased from Alfa Aesar (Tewksbury, MA); 5-methyl tetrahydrofolate (5-MTHF) was ordered from Sigma-Aldrich (St. Louis, Missouri); and 6-formylpterin was obtained from Cayman Chemical (Ann Arbor, Michigan). XO inhibition assay for folic acid related molecules is similar to that for herb extracts. Folic acids were dissolved in DMSO. Dose-dependent of folic acids and photolysis effect were studied. FDA-approved OX-inhibitor drug, Allopurinol, and small molecule XO inhibitor, DHNB, were included as a positive control.

### EXAMPLE 2 (REFERENCE EXAMPLE)

### DEVELOPMENT OF HEALTH FOOD SUPPLEMENTS/ANTIOXIDANTS FOR CONTROLLING HYPERURICEMIA AND OXIDATIVE STRESS

Provided herein are xanthine oxidase (XO) inhibition assays for 72 standard extracts of Chinese traditional herb medicines (single dose experiment: 163 ug/ml with two factions). There are 14 herb extracts that showed a strong effect of OX inhibition (> 40% either in water-soluble fraction or in DMSO-soluble fraction). There are 27 herb extracts, which have an XO inhibition rate from 20% to 40%. The rest of 31 herb extracts had a weak effect of XO inhibition (< 20%). Per preliminary literature search, 7 out of 14 extracts (>40% XO inhibition) are not previously reported, and 12 out of 27 extracts (20-40% OXI) are not previously reported. DMSO-soluble fractions of these extracts are more potent XO inhibitors than their water-soluble fractions. The inventors performed a dose dependent study of XO inhibition for each of these 14 extracts (DMSO-soluble fractions). All 14 extracts showed a nice dose-dependent XO inhibition curve. IC₅₀ is an operational parameter defined as the concentration of inhibitor required for achieving 50% inhibition of the enzyme. The smaller IC₅₀, the more potent the inhibitor is. IC₅₀ for each extract is calculated. Top five potent XO inhibitors are Alpinia officinarum ( 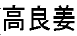 , IC₅₀ 28.5 ug/ml), Honeysuckle ( 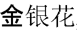 , IC₅₀ 33.5 ug/ml), Chinese Mint ( 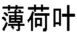 , IC₅₀ 34 ug/ml), Clove (Flower Bud)( 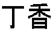 , IC₅₀ 63 ug/ml), and Citron fruit ( 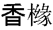 , IC₅₀ 71 ug/ml).

### 1. Data of 72 standard herb medicine extracts

Standard extracts of 72 traditional Chinese herb medicines in both water-soluble and DSMO-soluble fractions at a final concentration of 163 ug/ml were mixed with 20 nM XO, the initial rate of uric acid formation in the reaction system was recorded; and XO inhibition rate of each herb extract was calculated. All data are shown in Table 1. There are 14 herb extracts that showed a strong effect of XO inhibition (> 40% either in water-soluble fraction or in DMSO-soluble fraction). There are 27 herb extracts, which have an XO inhibition rate between 20% to 40%. The rest of 31 herb extracts had a weak effect of XO inhibition (< 20%).

### 72 items:

14 extracts: >40% XO inhibition (7/14 are not previously reported per our preliminary keyword search)
27 extracts: 20-40% XO inhibition (12 out of 27 are not previously reported per our preliminary keyword search)
31 extracts: <20% XO inhibition

**Table 1. XO inhibitory effects of 72 traditional Chinese herb medicines (standard extracts)**

| Herb extract and Description | Code | DMSO % of XO inhibition¹ | H₂O, % Of XO inhibition² | Previous report |
|---|---|---|---|---|
| #1, 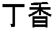 Extract 5:1 100g \| Ding Xiang Extract 5:1 \| Clove (Flower Bud) Extract 5:1 | EXD016 | 61 | 35 | Ref 61,62,63 |
| #2, 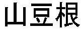 Extract 5:1 100g \| Shan Dou Gen Extract 5:1 \| Vietnamese Sophora (Root) Extract 5:1 | EXS007 | 71 | 22 | Ref 64,65 |
| 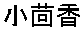 Extract 5:1 100g \| Xiao Hui Xiang Extract 5:1 \| Fennel (Fruit) Extract 5:1 | EXX009 | 39 | 19 | NA |
| 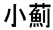 Extract 5:1 100g \| Xiao Ji Extract 5:1 \| Canadian Thistle (Aboveground Parts) Extract 5:1 | EXX010 | 30 | 35 | NA |
| 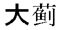 Extract 5:1 100g \| Da Ji Extract 5:1 \| Japanese Thistle Extract 5:1 | EXD003 | NA | 15 | |
| 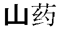 Extract 5:1 100g \| Shan Yao Extract 5:1 \| Chinese Yam (Rhizome) Extract 5:1 | EXS008 | 9 | 7 | |
| 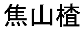 Extract 5:1 100g \| Shan Zha (Jiao) Extract 5: 11 Fructose Crataegi (Charred) Extract 5:1 | EXS009 | 30 | 0 | Ref 66 |
| 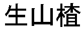 Extract 5:1 \| Shan Zha (Sheng) Extract 5:1 \| Fructus Crataegi Extract 5:1 | EXS010 | 5 | 0 | |
| 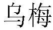 Extract 5:1 100g \| Wu Mei Extract 5:1 \| Smoked Plum / Fructus Mume Extract 5:1 | EXW007 | 5 | 4 | |
| #3, 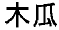 Extract 5:1 100g \| Mu Gua Extract 5:1 \| Flowering Quince (Fruit) Extract 5:1 | EXM009 | 52 | 28 | Ref 61,67,68 |
| 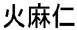 Extract 5:1 100g \| Huo Ma Ren Extract 5:1 \| Hemp Seed Extract 5:1 | EXH017 | 5 | 9 | |
| 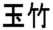 Extract 5:1 100g \| Yu Zhu Extract 5:1 \| Aromatic Solomon's Seal (Rhizomme) Extract 5:1 | EXY014 | 4 | 9 | |
| 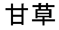 Extract 5:1 100g \| Gan Cao Extract 5:1 \| Chinese Licorice (Root & Rhizome) Extract 5:1 | EXG001 | 28 | 22 | Ref 66,69 |
| 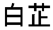 Extract 5:1 100g \| Bai Zhi Extract 5:1 \| Fragrant Angelica Extract 5:1 | EXB 11 | 0 | | |
| 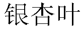 Extract 5:1 100g \| Yin Xing Ye Extract 5:1 \| Ginkgo Leaf Extract 5:1 | EXY008 | 31 | 22 | Ref 70 |
| 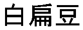 Extract 5:1 100g \| Bai Bian Dou Extract 5:1 \| White Hyacinth Bean Extract 5:1 | EXB002 | 30 | 19 | NA |
| 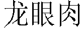 / 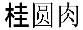 Extract 5:1 100g \| Gui Yuan Rou Extract 5:1 \| Longan Extract 5:1 | EXG013 | 10 | 8 | |
| 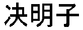 Extract 5:1 100g \| Jue Ming Zi Extract 5:1 \| Sickle-pod Senna (Seed) Extract 5:1 | EXJ010 | 27 | 15 | Ref 71 |
| 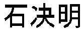 Extract 5:1 100g \| Shi Jue Ming Extract 5:1 \| Sea-ear Shell Extract 5:1 | EXJ023 | 30 | 22 | NA |
| 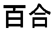 Extract 5:1 \| Bai He Extract 5:1 \| Bulbus Lilii Extract 5:1 | EXB004 | 7 | 10 | |
| 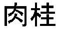 Extract 20:1 100g \| Rou Gui Extract 20:1 \| Cortex Cinnamomi Extract 20: 1 | EXR004 | 18 | 7 | |
| 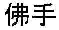 Extract 5:1 100g \| Fo Shou Extract 5: 11 Flesh-figer Citron Extract 5:1 | EXF004 | 0 | 0 | |
| 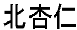 / 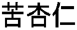 Extract 5:1 100g \| Bei Xing Ren Extract 5:1 \| Bitter Apricot Seed Extract 5:1 | EXB019 | 0 | | |
| 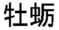 Extract 5:1 100g \| Mu Li Extract 5:1 \| Oyster Shell Extract 5:1 | EXM010 | 0 | | |
| #4, 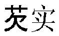 Extract 5:1 100g \| Qian Shi Extract 5:1 \| Euryale (Seed) Extract 5:1 | EXQ002 | 42 | 30 | NA |
| #5, 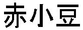 Extract 5:1 100g \| Chi Xiao Dou Extract 5:1 \| Rice Bean Extract 5:1 | EXC010 | 44 | 27 | NA |
| 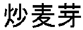 Extract 5:1 100g \| Chao Mai Ya Extract 5:1 \| Fructus Hordei Germinatus Extract 5:1 | EXC005 | 38 | 22 | NA |
| 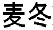 Extract 5:1 100g \| Mai Dong Extract 5:1 \| Ophiopogon Extract 5:1 | EXM003 | 0 | | |
| #6, 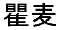 Extract 5:1 100g \| Qu Mai Extract 5:1 \| Fringed Pink (Aboveground Parts) Extract 5:1 | EXQ009 | 45 | 18 | Ref 66 |
| #7, 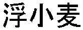 Extract 10:1 100g \| Fu Xiao Mai Extract 10:1 \| Wheat (Immature Fruit) Extract 10:1 | EXF008 | 40 | 25 | NA |
| 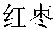 / 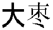 Extract 5:1 100g \| Da Zao Extract 5:1 \| Dried Dates Extract 5:1 | EXD006 | 0 | | |
| 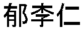 Extract 5:1 100g \| Chinese Dwwarf Cherry Seed Extract 5:1 \| Semen Pruni | EXY012 | 12 | 0 | |
| #8, 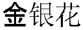 Extract 5:1 100g \| Jin Yin Hua Extract 5:1 \| Honeysuckle (Flower Bud) Extract 5:1 | EXJ006 | 73 | 55 | Ref 66,72,73,74 |
| #9, 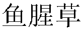 Extract 5:1 100g \| Yu Xing Cao Extract 5:1 \| Houttuynia (Aboveground Parts) Extract 5:1 | EXY013 | 40 | 3 | NA |
| 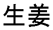 Extract 10:1 100g \| Sheng Jiang Extract 10: 1 \| Ginger (Fresh Rhizome) Extract 10: 1 | EXY018 | 5 | | |
| 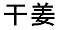 Extract 10:1 100g \| Gan Jiang Extract 5:1 \| Rhizome Zingi Extract 5:1 | EXG002 | 0 | 0 | |
| 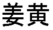 Extract 5:1 100g\| Jiang Huang Extract 5:1 \| Turmeric (Rhizome) Extract 5:1 | EXJ003 | 13 | 17 | |
| 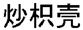 Extract 5:1 100g \| Zhi Ke Extract 5:1 \| Fructus Aurantii Processed Extract 5:1 | EXZ009 | 23 | 17 | Ref 75 |
| 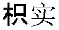 Extract 5:1 100g \| Zhi Shi Extract 5:1 \| Bitter Orange (Young Fruit) Extract 5:1 | EXZ012 | 10 | 20 | Ref 76 |
| 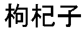 Extract 10:1 100g \| Gou Qi Extract 10:1 \| Lycium (Fruit) 10:1 | EXG007 | 0 | 0 | |
| 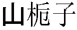 Extract 5:1 100g \| Shan Zhi Zi Extract 5:1 \| Gardenia (Fruit) Extract 5:1 | EXS011 | 0 | | |
| 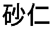 Extract 5:1 100g \| Sha Ren Extract 5:1 \| Chinese Amomum (Fruit) Extract 5:1 | EXS005 | 10 | 20 | |
| 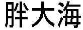 / 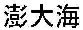 Extract 5:1 100g \| Pang Da Hai Extract 5:1 \| Sterculia (Seed) Extract 5:1 | EXP001 | 22 | 4 | NA |
| 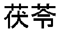 Extract 5:1 100g \| Fu Ling Extract 5:1 \| Poria (Sclerotium) Extract 5:1 | EXP005 | 10 | 10 | |
| 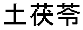 Extract 5:1 100g \| Tu Fu Ling Extract 5:1 \| Chinese Smilax (Rhizome) Extract 5:1 | EXT007 | 37 | 11 | Ref 66,76,77 |
| #10, 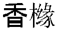 Extract 5:1 100g \| Xiang Yuan Extract 5:1 \| Citron (Fruit) Extract 5:1 | EXX008 | 50 | 21 | NA |
| Extract 5:1 100g \| Zhen Xiang Ru Extract 5:1 \| Haizhou Elsholtzia (Aboveground Parts) Extract 5:1 | EXZ005 | 38 | 26 | NA |
| 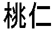 Extract 5:1 100g \| Tao Ren Extract 5:1 \| Peach (Seed) Extract 5:1 | EXT002 | 10 | 0 | |
| 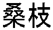 Extract 5:1 100g \| Sang Zhi Extract 5:1 \| White Mulberry (Young Twig) Extract 5:1 | EXS004 | 30 | 15 | Ref 66,78 |
| 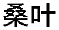 Extract 5:1 100g \| Sang Ye Extract 5:1 \| White Mulberry (Leaf) Extract 5:1 | EXS003 | 22 | 7 | Ref 66,79 |
| 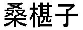 Extract 5:1 100g \| Sang Shen Zi Extract 5:1 \| White Mulberry (Fruit) Extract 5:1 | EXS002 | 0 | | |
| 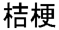 Extract 5:1 100g \| Jie Geng Extract 5:1 \| Platycodon grandiflorus (Jacq.)A. DC. Extract 5:1 | EXJ004 | 0 | | |
| 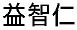 Extract 5:1 100g \| Yi Zhi Ren Extract 5:1 \| Sharp-Leaf Galangal (Seed) Extract 5:1 | EXY006 | 32 | 18 | NA |
| 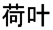 Extract 5:1 100g \| He Ye Extract 5:1 \| Sacred Lotus (Leaf) 5:1 | EXH005 | 31 | 17 | Ref 80 |
| 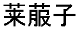 Extract 5:1 100g \| Lai Fu Zi Extract 5:1 \| Radish (Seed) Extract 5:1 | EXI,001 | 20 | 20 | NA |
| #11, 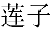 Extract 5:1 100g \| Lian Zi Extract 5:1 \| Sacred Lotus (Seed) Extract 5:1 | EXL005 | 46 | 16 | NA |
| 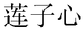 Extract 5:1 100g \| Lian Zi Xin Extract 5:1 \| Sacred Lotus (Plumule) Extract 5:1 | EXL006 | 29 | 8 | NA |
| #12, 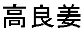 Extract 5:1 100g \| Gao Liang Jiang Extract 5:1 \| Lesser Galangal (also called *Alpinia officinarum*) Extract 5:1 | EXG004 | 65 | 23 | Ref 81.82.83 |
| 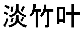 Extract 5:1 100g \| Dan Zhu Ye Extract 5:1 \| Lophatherum Extract 5:1 | EXD009 | 10 | 0 | |
| 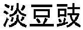 Extract 5:1 100g \| Dan Dou Chi Extract 5:1 \| Fermented Soybean Extract 5:1 | EXD007 | 24 | 13 | NA |
| #13, 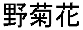 Extract 5:1 100g \| Ye Ju Hua Extract 5:1 \| Wild Chrysanthemum (Flower) Extract 5:1 | EXY003 | 48 | 34 | Ref 66,84,85,86.87 |
| 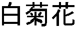 Extract 5:1 100g \| Bai Ju Hua Extract 5:1 \| Chrysanthemum (Flower) Extract 5:1 | EXB008 | 27 | 16 | Ref 66,84,85,86,87 |
| 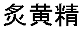 Extract 5:1 100g \| Zhi Huang Jing Extract 5:1 \| Polygonatum (Rhizome, Cured) Extract 5:1 | EXZ008 | 0 | | |
| 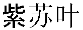 Extract 5:1 100g \| Zi Su Ye Extract 5:1 \| Perilla (Leaf) Extract 5:1 | EXZ018 | 32 | 17 | Ref 88,89 |
| 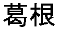 Extract 5:1 100g \| Ge Gen Extract 5:1 \| Kudzu 5:1 | EXG005 | 0 | | |
| 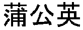 Extract 5:1 100g \| Pu Gong Ying Extract 5:1 \| Herba Taraxaci Extract 5:1 | EXP004 | 15 | 15 | |
| () Extract 5:1 100g \| Suan Zao Ren Extract 5:1 \| Jujube (Seed) Extract 5:1 | EXS027 | 32 | 21 | NA |
| 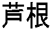 Extract 5:1 100g \| Lu Ge Extract 5:1 \| Phragmites (Rhizome) Extract 5:1 | EXL010 | 17 | 10 | |
| #14, 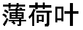 Extract 5:1 100g \| Bo He Extract 5: 1 \| Chinese Mint Extract 5: 1 | EXB020 | 80 | 71 | NA |
| 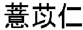 / 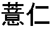 Extract 5:1 100g \| Yi Ren Extract 5:1 \| Job's Tears Extract 5:1 | EXYOO5 | 37 | 15 | Ref 66,90 |
| 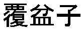 Extract 5:1 100g \| Fu Pen Zi Extract 5:1 \| Palm-leaf Raspberry Extract 5:1 | EXF006 | 10 | 30 | Ref 91 |
| 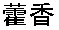 Extract 5:1 100g \| Huo Xiang Extract 5:1 \| Patchouli (Aboveground Parts) Extract 5:1 | EXH018 | 35 | 20 | Ref 92 |

| | | | | |
|---|---|---|---|---|
| NA means: the extract was not previously reported for XO inhibition Blank means: the extract has no XO inhibition as tested, and no literature search was performed ¹ DMSO fraction for the extract ² Water fraction for the extract | | | | |

In general, most DMSO-soluble fractions of many herb extracts have a stronger effect of XO inhibition than their water-soluble fractions. These data indicates that potent XO inhibitory compounds are hydrophobic, in specific embodiments.

### 2. Data of dose-dependent of 14 standard herb medicine extracts

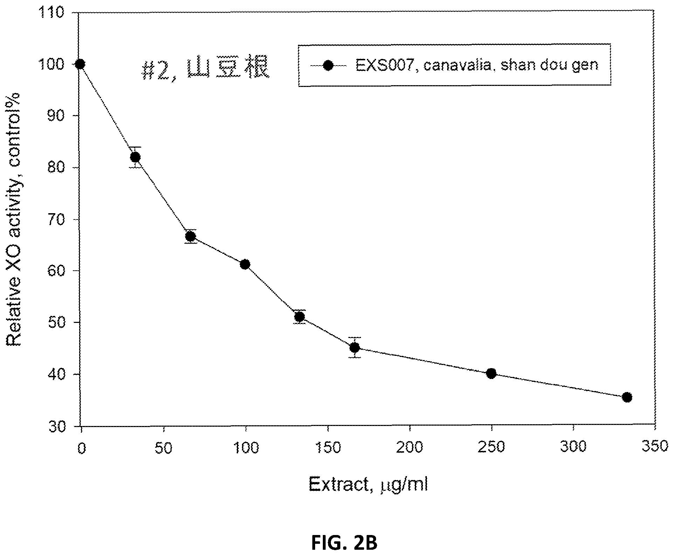

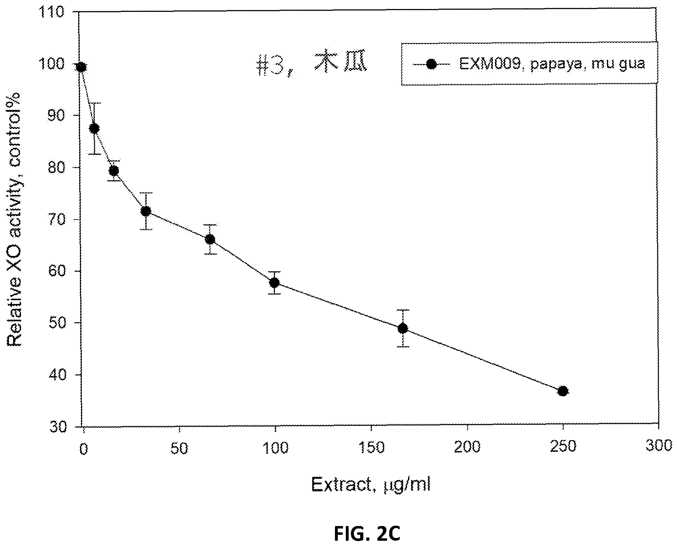

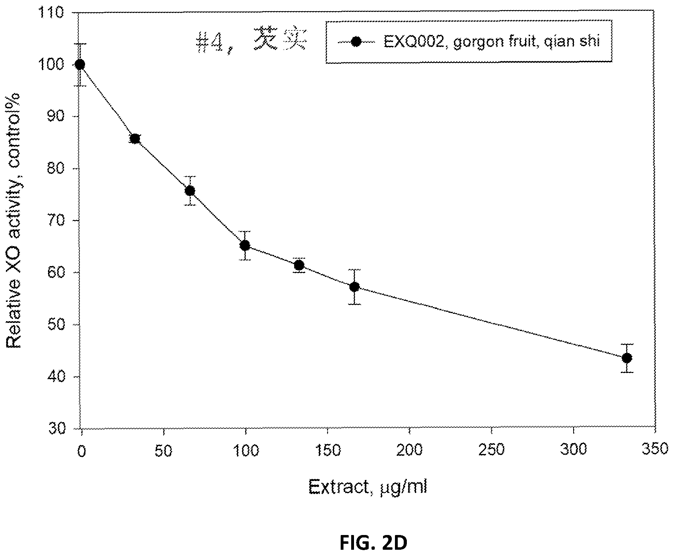

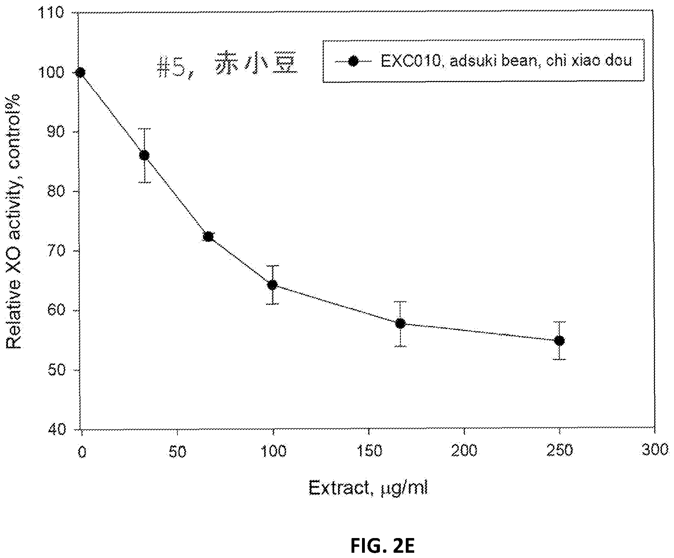

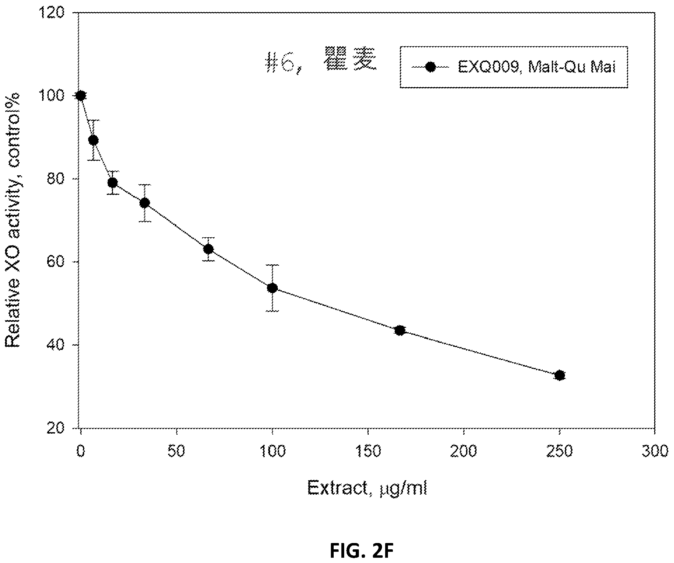

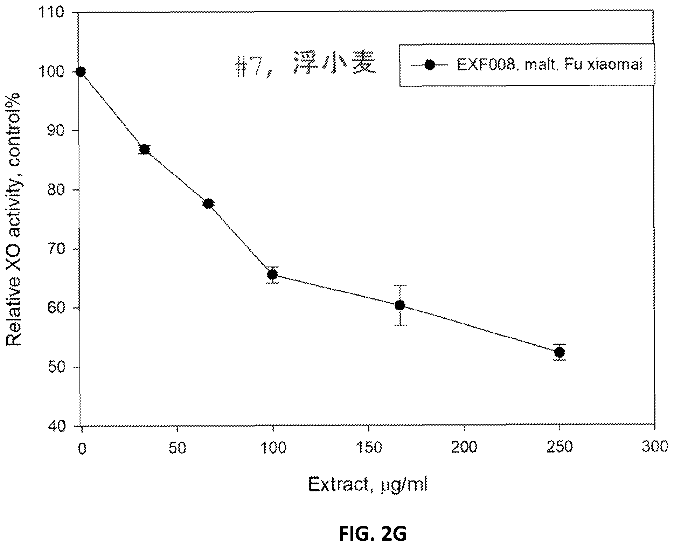

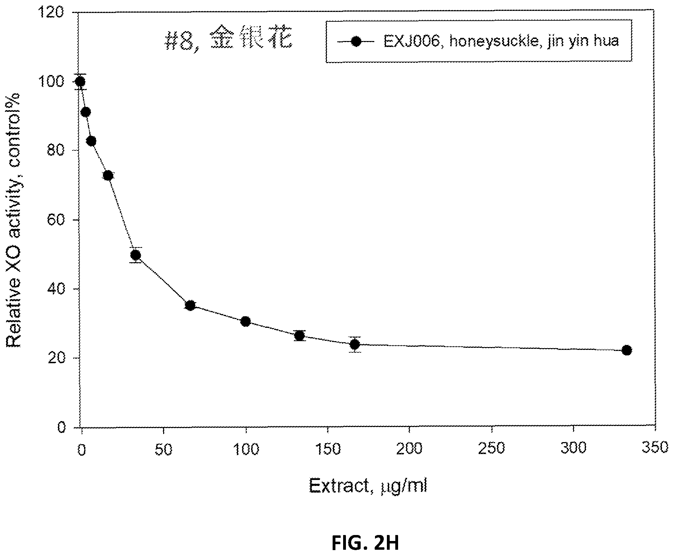

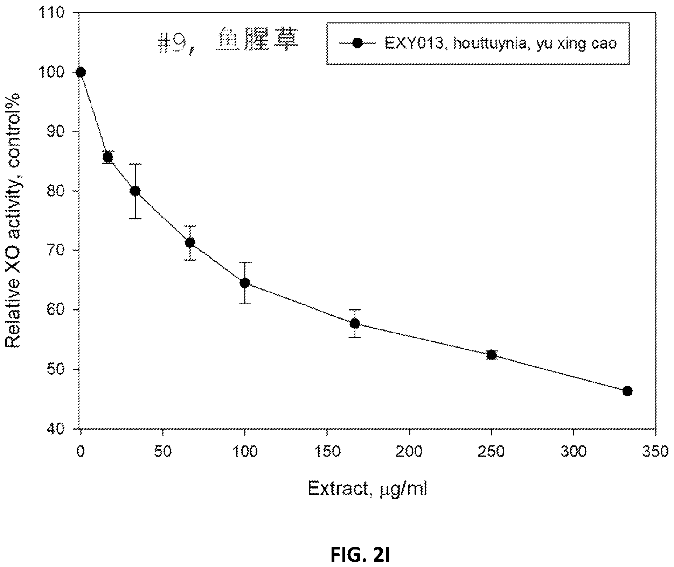

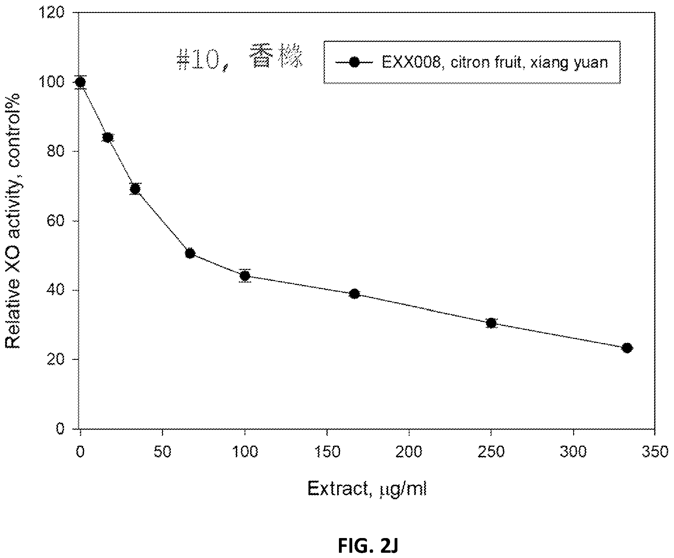

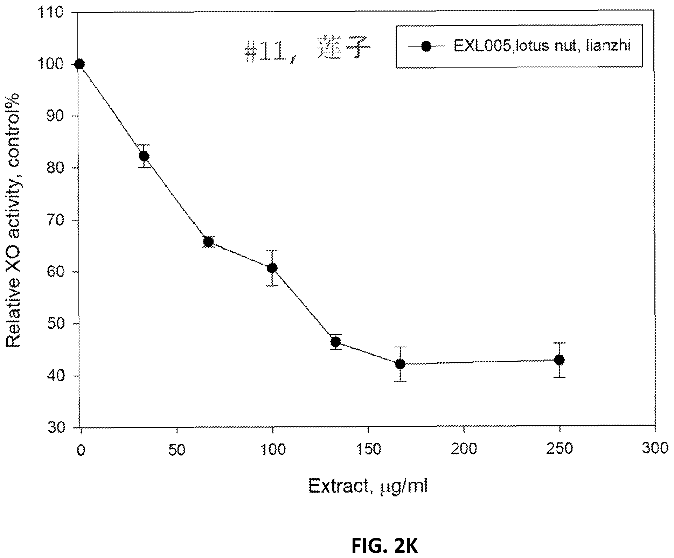

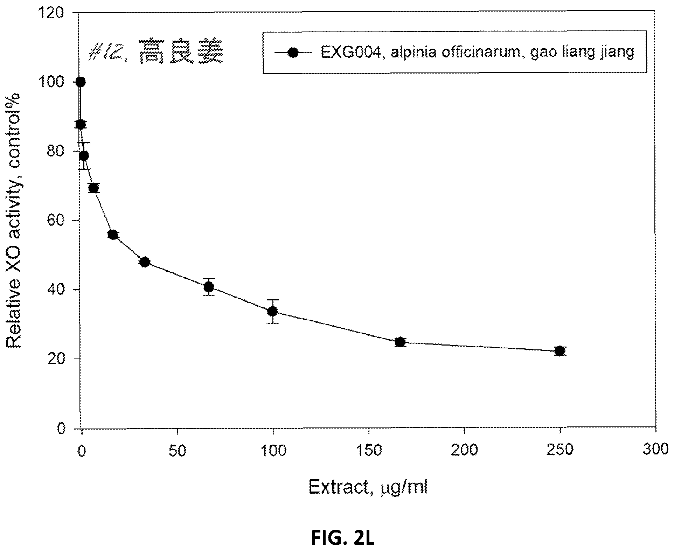

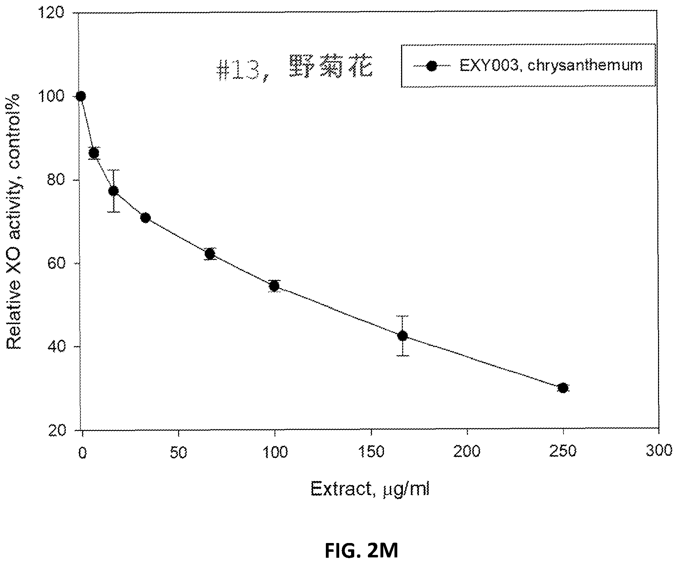

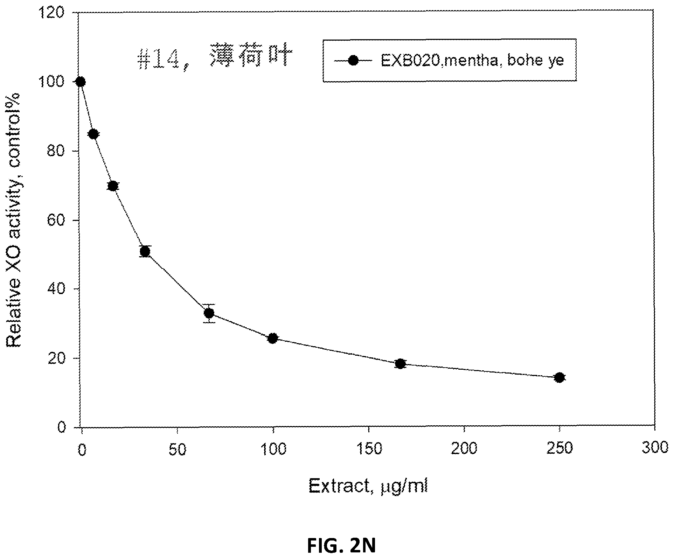

From initial screen of XO inhibitory effects of 72 traditional Chinese herb standard extracts with single dose (163 ug/ml), 14 extracts had more than 40% inhibition of XO activity. DMSO-soluble fractions of these extracts are more potent XO inhibitors than their water-soluble fractions (FIG. 1). Furthermore, we performed a dose dependent XO inhibition assay for each of these 14 extracts (DMSO-soluble fractions). Concentration range of each extract from 0 to 250, or 333 ug/ml (8 to 10 doses was studied). All 14 extracts showed a nice dose-dependent XO inhibition curve (FIG. 2). IC₅₀ is an operational parameter defined as the concentration of inhibitor required for achieving 50% inhibition of the enzyme. The smaller IC₅₀, the more potent the inhibitor is. IC₅₀ for each extract is calculated (Table 2). Top five potent XO inhibitors are Alpinia officinarum ( 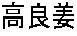 , IC₅₀ 28.5 ug/ml), Honeysuckle ( 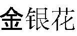 , IC₅₀ 33.5 ug/ml), Chinese Mint ( 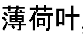 , IC₅₀ 34 ug/ml), Clove ( 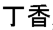 , IC₅₀ 63 ug/ml), and Citron fruit ( 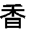 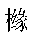 , IC₅₀ 71 ug/ml). These data are useful for understanding the potency of these extracts and provides rational design for combination assay of each extract with folic acid and 5-MTHF (for example) or multiple extracts with folic acid and 5-MTHF (for example).

**Table 2. IC₅₀ (XO inhibitory effect) of 14 traditional Chinese herb medicines (standard extracts)**

| Herb extract and Description | Code | DMSO % of XO inhibition | H₂O, % Of XO inhibition | IC50 (ug/ml) | Previous report |
|---|---|---|---|---|---|
| #1, 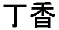 Extract 5:1 100g \| Ding Xiang Extract 5:1 \| Clove (Flower Bud) Extract 5:1 | EXD016 | 61 | 35 | 63 | Ref 61,62,63 |
| #2, 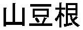 Extract 5:1 100g \| Shan Dou Gen Extract 5:1 \| Vietnamese Sophora (Root) Extract 5:1 | EXS007 | 71 | 22 | 136 | Ref 64,65 |
| #3, 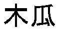 Extract 5:1 100g \| Mu Gua Extract 5:1 \| Flowering Quince (Fruit) Extract 5:1 | EXM009 | 52 | 28 | 154 | Ref 61,67,68 |
| #4, 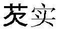 Extract 5:1 100g \| Qian Shi Extract 5:1 \| Euryale (Seed) Extract 5:1 | EXQ002 | 42 | 30 | 246 | NA |
| #5, 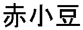 Extract 5:1 100g \| Chi Xiao Dou Extract 5:1 \| Rice Bean Extract 5:1 | EXC010 | 44 | 27 | >300 | NA |
| #6, 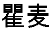 Extract 5:1 100g \| Qu Mai Extract 5:1 \| Fringed Pink (Aboveground Parts) Extract 5:1 | EXQ009 | 45 | 18 | 120 | Ref 66 |
| #7, 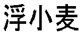 Extract 10:1 100g \| Fu Xiao Mai Extract 10:1 \| Wheat (Immature Fruit) Extract 10:1 | EXF008 | 40 | 25 | 270 | NA |
| #8, 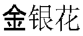 Extract 5:1 100g \| Jin Yin Hua Extract 5:1 \| Honeysuckle (Flower Bud) Extract 5:1 | EXJ006 | 73 | 55 | 33.5 | Ref 66,72,73,74 |
| #9, 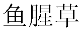 Extract 5:1 100g \| Yu Xing Cao Extract 5:1 \| Houttuynia (Aboveground Parts) Extract 5:1 | EXY013 | 40 | 3 | 285 | NA |
| #10, 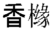 Extract 5:1 100g \| Xiang Yuan Extract 5:1 \| Citron (Fruit) Extract 5:1 | EXX008 | 50 | 21 | 71 | NA |
| #11, 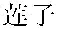 Extract 5:1 100g \| Lian Zi Extract 5:1 \| Sacred Lotus (Seed) Extract 5:1 | EXL005 | 46 | 16 | 123 | NA |
| #12, 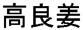 Extract 5:1 100g \| Gao Liang Jiang Extract 5:1 \| Lesser Galangal Extract 5:1 | EXG004 | 65 | 23 | 28.5 | Ref 81.82.83 |
| #13, 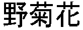 Extract 5:1 100g \| Ye Ju Hua Extract 5:1 \| Wild Chrysanthemum (Flower) Extract 5:1 | EXY003 | 48 | 34 | 123 | Ref 66,84,85,86.87 |
| #14, 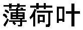 Extract 5:1 100g \| Bo He Extract 5:1 \| Chinese Mint Extract 5:1 | EXB020 | 80 | 71 | 34 | NA |

| | | | | | |
|---|---|---|---|---|---|
| NA means: the extract was not previously reported for XO inhibition Blank means: the extract has no XO inhibition as tested, and no literature search was performed | | | | | |

### Data of folic acid and its derivatives

Folic acid and its two derivatives, dihydrofolic acid and tetrahydrofolic acid, were employed in the XO inhibition assay. When 20 nM XO was mixed with increasing concentrations of folic acid, dihydrofolic acid and tetrahydrofolic acid. The initial rate of uric acid formation showed a concentration-dependent decrease compared to the negative control, reflecting the decrease of XO activity (FIG. 3). Folic acid, dihydrofolic acid and tetrahydrofolic acid significantly inhibited XO activity with a dose range within 2 uM. Potency of XO inhibition shows dihydrofolic acid > tetrahydrofolic > folic acid. As compared to Allopurinol and DHNB, folic acid had an XO inhibitory effect stronger than Allopurinol and DHNB (FIG. 4). The half maximal inhibitory concentration (IC₅₀) for folic acid, allopurinol and DNHB is 0.75 uM, 1.8 uM and 3.0 uM, respectively.

### Significance of Certain Embodiments (Folic acid is a XO inhibitor)

Folate and folic acid are forms of a water-soluble B vitamin. It is essential for the body to make DNA, RNA, and metabolize amino acids, which are required for cell division and many physiological functions. As humans cannot make folic acid, it is required from the diet, making it an essential vitamin. Folate is the common form of vitamin B9 present in many whole foods, including leafy greens, beans, eggs, citrus fruit, avocados, and beef liver. While folic acid is a synthesized version of vitamin B9 that is added to processed foods and the common version used in supplements. Folic acid is an oxidized form of folate, and has a molecular structure that is nearly identical to folate (a salt form). Folic acid is more stable than physiological types of folate. Since 1998, folic acid has been added to cold cereals, flour, breads, pasta, bakery items, cookies, and crackers, as required by US federal law. More than 50 countries are doing this. The goal of this regulation is an attempt to reduce the prevalence of Neural Tube Defects (NTDs), a common birth defect that had shown some connection to the mother's vitamin B9 intake. Besides NTDs, folate deficiency can lead to many health problems such as slowed growth, megaloblastic anemia, weight loss, digestive disorders, leukopenia, thrombocytopenia, cracking/redness of tongue/mouth, diarrhea, liver disease, cancers, cardiovascular disease, depression and other behavior changes (93-96). Thus, folic acid supplement has enormous impact on the improvement and maintenance of human health including prevention and treatment of many diseases.

Before folic acid can be utilized by the body, it must undergo two conversions to dihydrofolate (DHF) first and then tetrahydrofolate (THF). THF can be converted to 5,10-methylenetetrahydrofolate (5,10-MTHFR) and 10-formyl tetrahydrofolate (10-FTHF), and 5-methyl tetrahydrofolate (5-MTHF, levomefolic acid), which is the end form of folate that the body requires for many key functions (FIG. 5). One of the most important folate-dependent reactions is the methylation of deoxyuridylate to thymidylate in the formation of DNA, is required for proper cell division. An impairment of this reaction initiates a process that can lead to megaloblastic anemia, one of the hallmarks of folate deficiency. Another folate-dependent reaction is the conversion of homocysteine to methionine in the synthesis of S-adenosyl-methionine. Hyperhomocyteinemia (Hcy) is toxic, well known risk factor for cardiovascular diseases and many other diseases (97). The chemical reactions that are necessary for degradation of Hcy require the presence of the folic acid, vitamins B6 and B12. Fold acid is also important for methylation of DNA, phospholipids and proteins for their functional regulations as well as synthesis of several important neurotransmitters such as serotonin, epinephrine and dopamine (FIG. 6). Furthermore, there is evidence that folic acid exerts both direct and indirect antioxidant effects, such as free radical scavenging (98).

Methylenetetrahydrofolate reductase (MTHFR) is the key enzyme that regulates this conversion process. However, it is estimated that up to 60% of Americans have genetic variations that reduce their ability to convert 5-MTHF from folic acid on their own. For those who have this genetic variation, supplementing with folic acid may lead to varying amounts of the converted 5-MTHF form, potentially leaving the body with less than it requires. Furthermore, it could lead to a build-up of the folic acid form in the body. This is why supplementing with 5-MTHF can be considered superior to using folic acid for those that may not get enough from the diet. THF is active only in its (unstable) reduced form and thus not suitable for oral supplementation. In general, supplements of folic acid and 5-MTHF at a recommended amount is safe and beneficial. The risk of toxicity from folic acid is low because folate is a water-soluble vitamin and is regularly removed from the body through urine (99).Folic acid intake below the established tolerable upper intake level (LTL) of 1000 µg/day for the general population is not associated with any adverse health outcomes (100). However, excessive folic acid intake may be correlated with significant health risks including cancer (101-103); this risk issue is still under debate, not confirmed yet. In fact, folate deficiency increases the initiation of carcinogenesis by causing genome instability as well as alteration of methylation patterns in the genome, leading to altered expression of oncogenes and tumor suppressor genes (100).

The effect of folic acid on XO inhibition was first reported in 1948 (104); subsequently, the same research team reported that this inhibition was due to pterine-6-aldehyde (2-NH,-4-OH-pteridine-6-aldehyde), a photolytic breakdown product of folic acid in 1950 (105). This argument continued in 1980s (106, 107). Up to 1989, further experiments with different methods confirm that uric acid and its derivatives indeed are potent XO inhibitors (108). XO inhibitory effects of THF and DHF are stronger than that of folic acid (107, 109). Studies have shown that 5-MTHF can effectively inhibit XO activity in vitro and in vivo (110). Folic acid is known to be sensitive to ultraviolet (UV) radiation (111-116); it can be cleaved into p-aminobenzoyl-L-glutamic acid and 6-formyl pterin when exposed to ultraviolet radiation. When the irradiation continues, 6-formyl pterin is degraded to pterin-6-carboxylic acid (117). 6-formyl pterin is a very potent XO inhibitor in vitro; while pterin-6-carboxylic acid has no XO inhibitory effect (117).

Like humans, birds such as chicken and quail lack the enzyme uricase; their blood uric acid levels are usually higher than that in mice or rats that have the enzyme uricase, metabolizing uric acid to 5-hydroxyisourate (118). In fact, gout is a common disease in chicken, and their blood levels of uric acid can be as high as 44 mg/dL as compared to 5-7 mg/dL in a normal bird. Dietary folic acid supplementation significantly inhibits liver XO activities in the chick (119,120). A recent study showed that dietary folic acid supplementation significantly reduced serum uric acid in the older laying hens (121). Intraperitoneal administration of folic acid and allopurinol (12.5-50 mg/kg) significantly inhibited radiation-induced activation of XO in a mouse model (122). Daily oral supplement of folic acid significantly reduced serum uric acid levels and its associated renal pathology in a rat model (123). In addition, folic acid supplementation can prevent cardiovascular disease (124-126) and renal disease (127), inhibit tumorigenesis (128) and enhance the efficacy of antitumor chemotherapy (129) in animal models.

Studies of dietary supplement of folic acid for patients with hyperuricemia and/or gout have been reported. A case-control study showed that high folate intake may protect against gout (130). In adult hypertensive patients, the administration of a daily dose of 10 mg of Enalapril combined with 0.8 mg of folic acid had a greater beneficial effect on the serum UA levels than did that of 10 mg of Enalapril alone in patients with either an elevated UA concentration or baseline hyperuricemia (54,55). Furthermore, folic acid supplementation can reduce risks for cardiovascular disease and chronic kidney disease in clinical trials (56-59, 131).

One can confirm the efficacy of folic acid or 5-MTHF on control of hyperuricemia and in specific embodiments include it into one or more combination recipes with traditional Chinese herb medicines or other botanical ingredients as of dietary health supplements for long term management of hyperuricemia, as one example. Folic acid may be one of the components of any composition encompassed herein, in certain embodiments, and in specific embodiments it enhances the therapeutic efficacy of the combination therapy.

### 4. Data of combination of single herb medicine extract with folic acid

Based on the result of dose-dependent XO inhibition experiments for herb extracts and folic acid, a relatively small concentration of each extract (a total of 14 extracts) and folic acid were utilized for a combination assay. Initially, Chinese mint leave extract (6.67 ug/ml) and folic acid (0.167 uM) inhibited XO activity by 25.85% and 16.49%, respectively. The combination of Chinese mint leave extract and folic acid at these concentrations showed XO inhibition by 34.4% (FIG. 7). It is clear that the combination of Chinese mint leave extract and folic acid shows an additive effect on XO inhibition. One of the important advantages of such combination supplements is enhancement of XO inhibitory function of the supplement; while reducing the dose of each component, thereby reducing potential side effects of the supplement for long-term use.

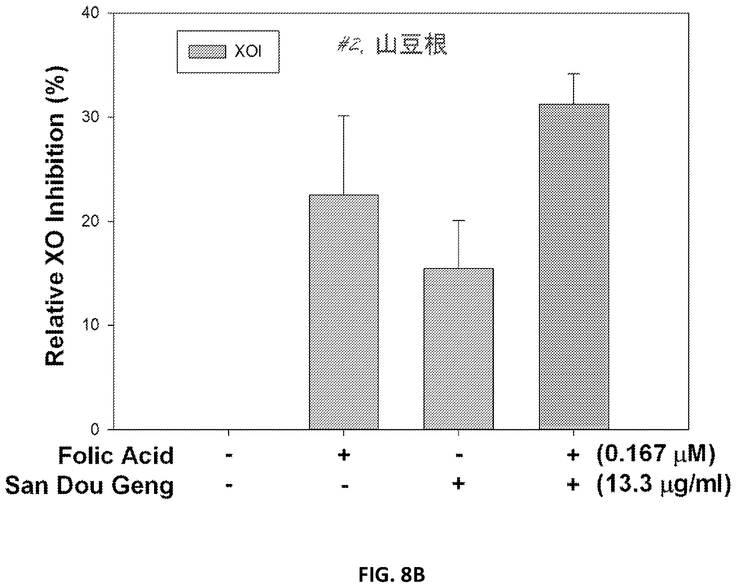

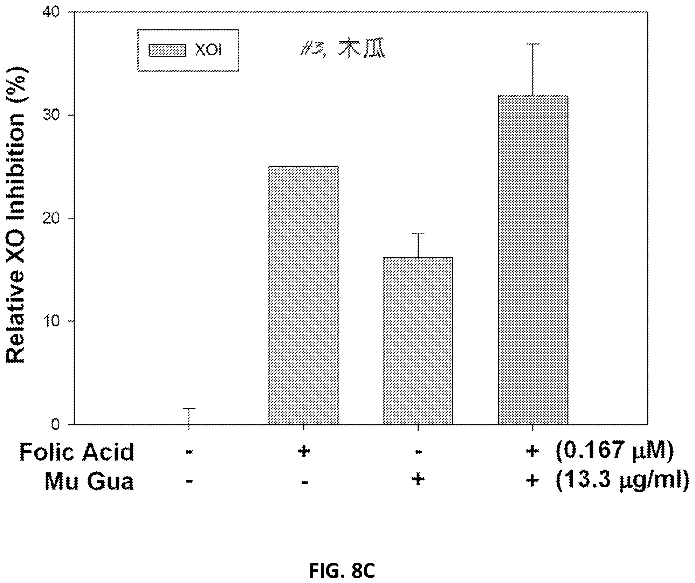

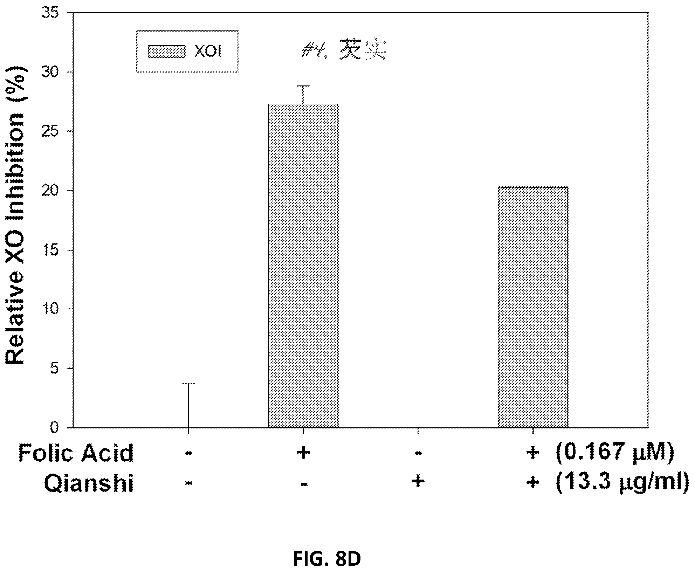

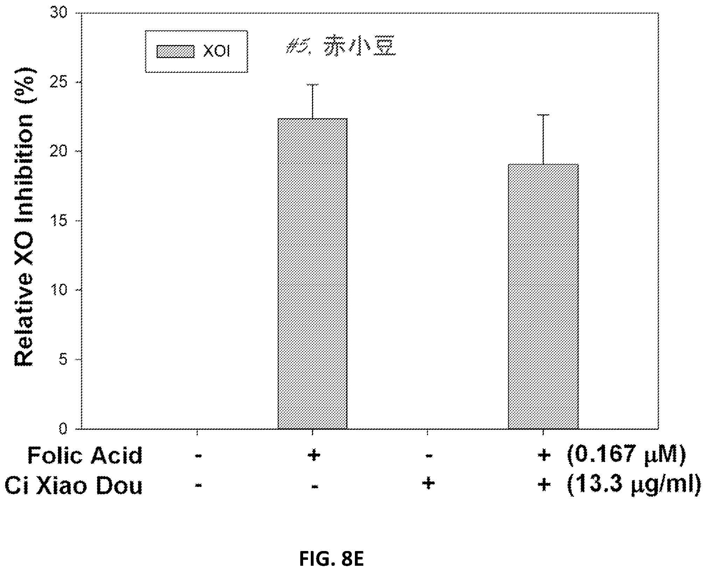

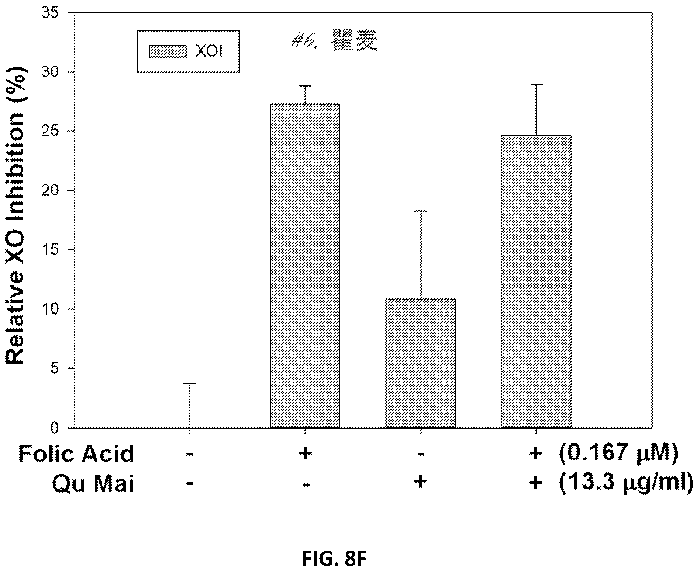

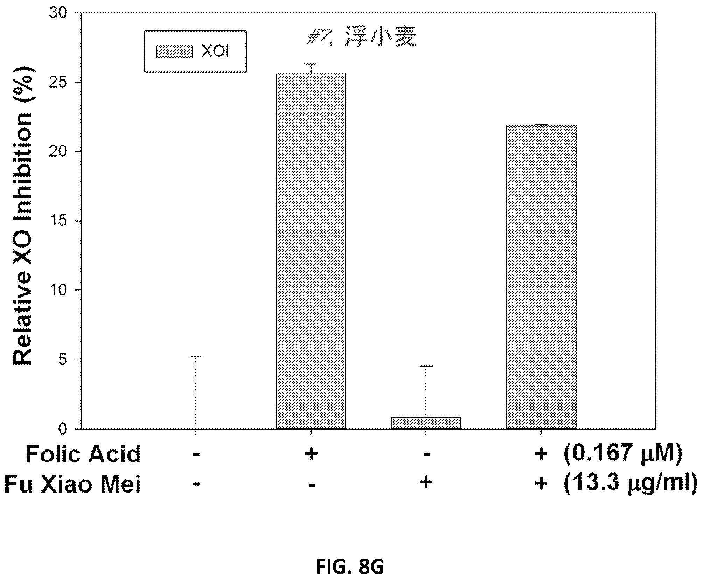

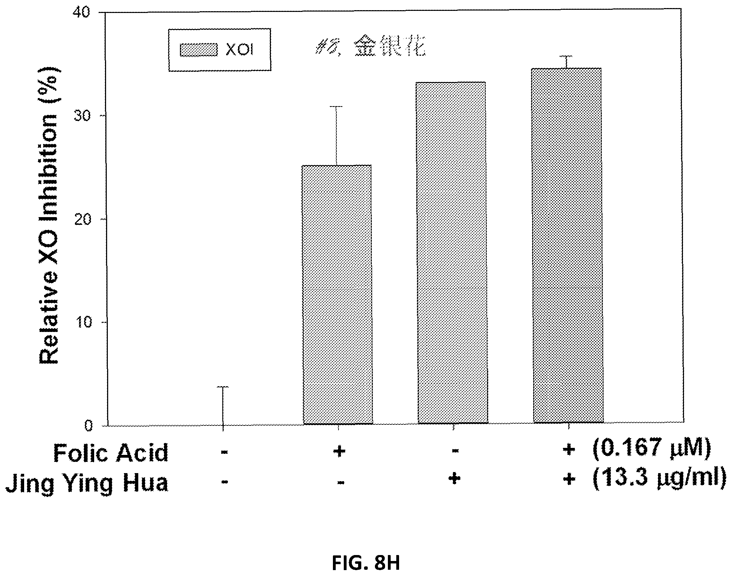

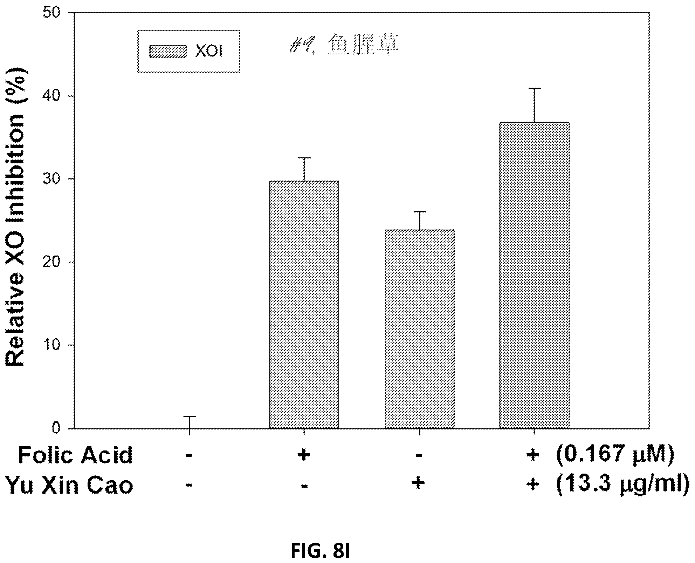

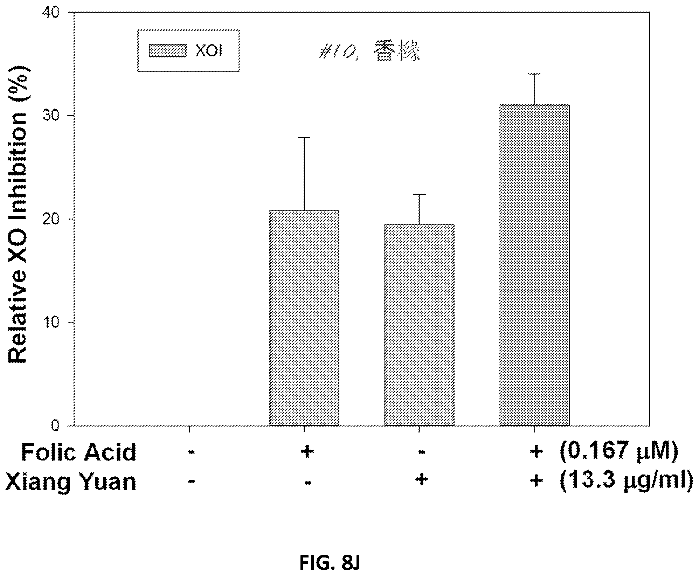

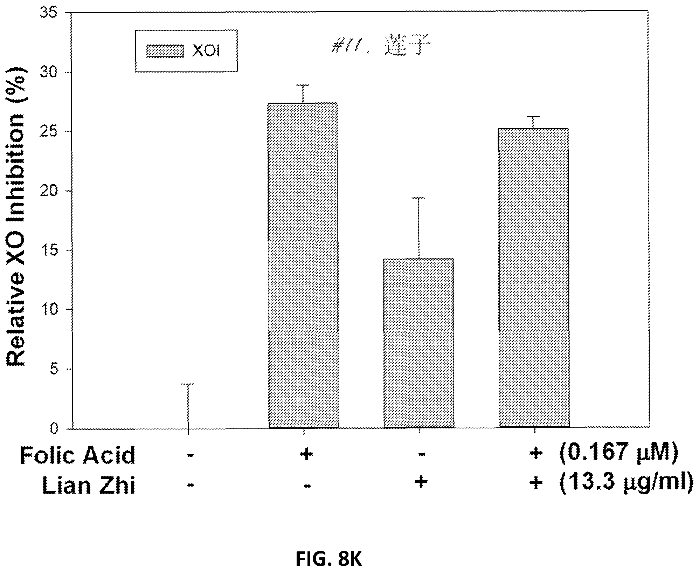

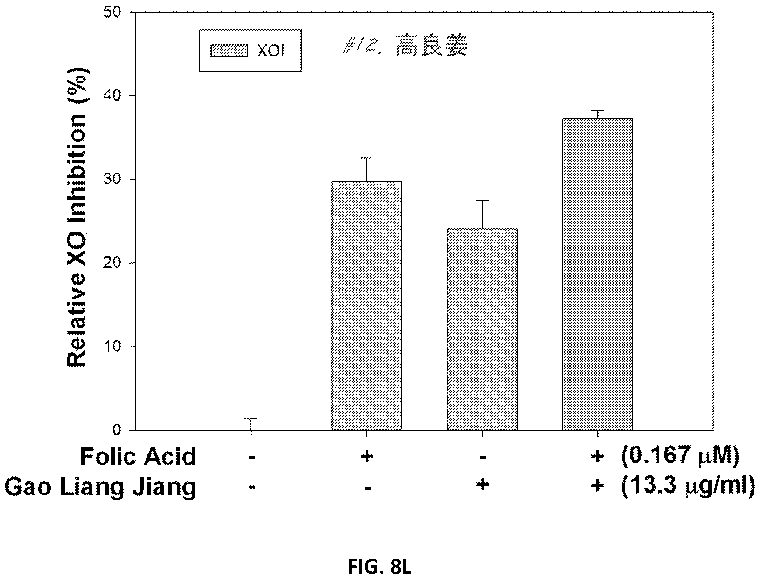

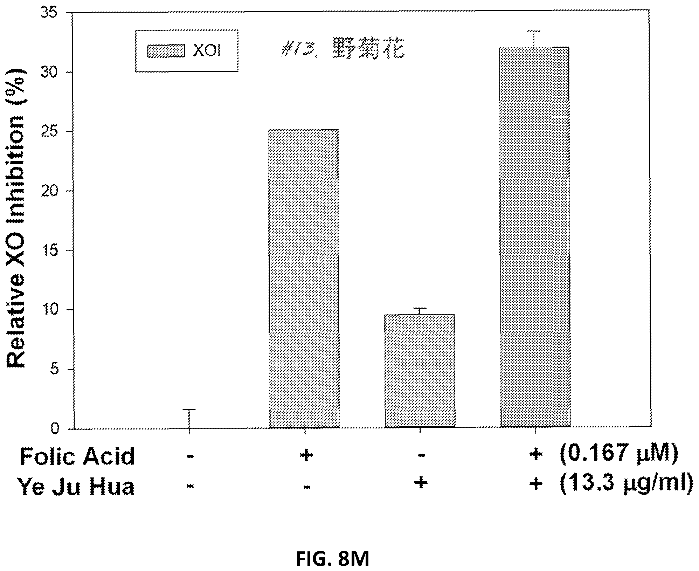

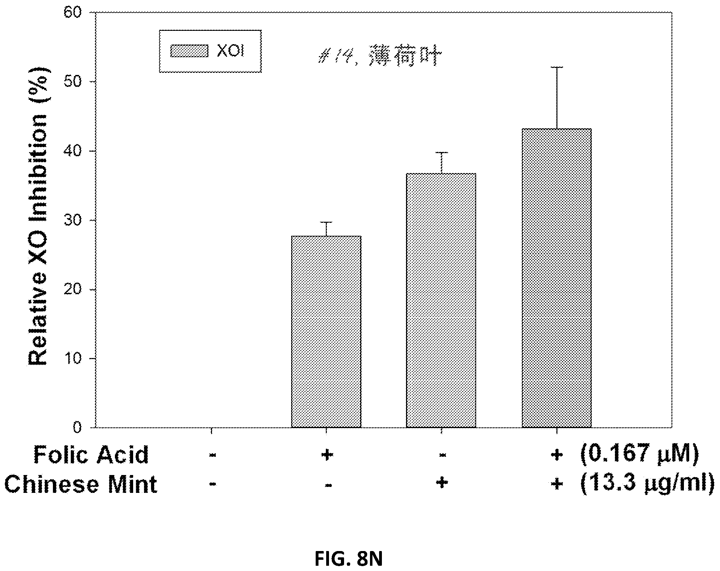

Furthermore, a combination essay was performed for folic acid and each of all 14 traditional Chinese medicine herb extracts including Chinese mint extract. Folic acid (0.167 uM) and extract (13.3 ug/ml) were separately added into the XO reaction system without pre-mixing. XO activity was measured. 9 out of 14 extracts showed an additive XO inhibitory effect with folic acid (FIG. 8). These effective extracts are Chinese mint extract ( 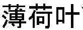 ); | Clove (Flower Bud) Extract (Ding xiang 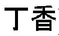 ); Vietnamese Sophora (Root) Extract (San Dou Geng 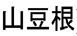 ); Flowering Quince (Fruit) Extract (Mu Gua, 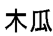 ); Honeysuckle (Flower Bud) Extract (Jing Ying Hua, 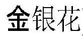 ); Houttuynia (Aboveground Parts) Extract (Yu Xin Cao 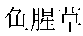 ); Citron (Fruit) Extract (Xiang Yuan 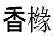 ); Lesser Galangal Extract (Gao Liang Jiang 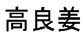 ); and Wild Chrysanthemum (Flower) Extract (Ye Ju Hua 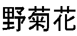 ).

### 5. Data of combination of herb medicine extracts with 5-methyl tetrahydrofolate (5-MTHF)

5-methyl tetrahydrofolate (5-MTHF) is considered a superior form of folic acid supplementing. Studies have shown that 5-MTHF can effectively inhibit XO activity in vitro and in vivo (90). The inventors have confirmed that 5-MTHF effectively inhibited XO activity in vitro in a concentration-dependent manner (FIG. 9). IC₅₀ for 5-MTHF is about 35.6 uM. However, the XO inhibitory effect of 5-MTHF is weaker than folic acid (IC₅₀ 0.75 uM). The inventors also tested the combination of 5-MTHF with different traditional Chinese medicine herb extracts for their effect on XO activity in vitro (FIG. 10). 5-MTHF had an additive effect on XO inhibition with 10 out of 14 herb extracts: Flowering Quince (Fruit) Extract (Mu Gua 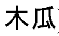 ); Euryale (Seed) Extract (Gian Shi 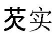 ); Fringed Pink (Aboveground Parts) Extract (Qu Mai, 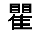 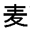 ), Honeysuckle (Flower Bud) Extract (Jing Ying Hua, 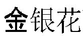 ), Houttuynia (Aboveground Parts) (Yu Xin Cao 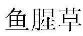 ); Citron (Fruit) Extract (Xiang Yuan 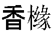 ); Sacred Lotus (Seed) Extract (Lian Zhi, 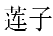 ); Lesser Galangal Extract (Gao Liang Jiang, 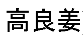 ); Wild Chrysanthemum (Flower) Extract (Ye Ju Hua ( 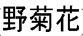 ); and Chinese mint (Bo He Yie, 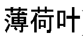 ) (Figure 10), but not with Clove (Flower Bud) Extract (Ding xiang 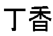 ); Vietnamese Sophora (Root) Extract (San Dou Geng 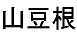 ); Rice Bean Extract (Ci Xiao Dou 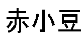 ); and Wheat (Immature Fruit) Extract (Fu Xiao Mei 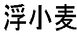 ).

In some embodiments, pre-mixing is utilized of folic acid or 5-methyl tetrahydrofolate and individual extracts on XO inhibition. After completion of studies of combinations of single extracts with folic acid, one can further characterize the combination of multiple herb extracts with folic acid. One can also characterize the combination of multiple herb extracts without folic acid and/or 5-methyl tetrahydrofolate for the XO inhibition.

One can extend XO activity assay to more Chinese herb extracts and other botanical ingredients and study their additive or synergistic effect among these agents with or without folic acid and/or 5-methyl tetrahydrofolate. Regarding XO inhibition studies, one can screen and characterize a few lead combination recipes of herb extracts with or without folic acid or 5-MTHF for animal model tests and ultimately for the treatment and/prevention of patients with both symptomatic and asymptomatically hyperuricemia conditions, which are a significant and independent risk factor for cardiovascular disease and many other diseases.

### EXAMPLE 3 (REFERENCE EXAMPLE)

### PARTICULAR EMBODIMENTS OF DIETARY HEALTH SUPPLEMENTS/ANTIOXIDANTS FOR CONTROLLING HYPERURICEMIA AND OXIDATIVE STRESS

Embodiments of the disclosure encompass compositions (such as health food supplements or functional food) that are able to reduce blood uric acid, to reduce oxidative stress, and/or to lower the risks of gout and cardiovascular disease, for example. In such embodiments, one can select, characterize, optimize, synergize and formulate extracts of certain Chinese herbs and/or regular food items as well as vitamins, minerals and nutrients for the purpose of developing new, safe health food supplements or functional food for controlling hyperuricemia and oxidative stress, especially in its long-term use for the prevention and treatment of gout and hyperuricemia-induced cardiovascular disease. One can utilize a cell free system of XO enzyme activity assay and mouse models for this embodiment, for example.

One can employ *in vitro* screening of effective raw materials of dietary health supplement products and develop more effective recipes/formulas for the inhibition of xanthine oxdase (XO) activity and oxidative stress (a variety of substrates and combinations, additive effects, synergistic effects). One can use acute (short-term) animal studies to characterize the effectiveness and toxicity of dietary health supplement recipes/formulas for controlling hyperuricemia. One can also use chronic (relatively long-term) animal studies to characterize the effectiveness and toxicity of dietary health supplement recipes/formulas for controlling hyperuricemia.

In specific embodiments, some traditional Chinese herb medicines have potent XO-inhibitory activities as well as antioxidant activities; individual herb activities may have additive or synergistic effects with other herb medicines and/or with folic acid and/or one or more of its derivatives, for example (folic acid and its derivatives are XO inhibitors (52,53). In specific aspects, the functional food/dietary supplement formulation(s) of the present disclosure including traditional Chinese herb extract and (in some cases) folic acid and/or one or more of its derivatives provide a new preventative and/or treatment for asymptomatic hyperuricemia is utilized as a preventative and/or treatment. In specific cases, the compositions of the disclosure provide for long-term management of asymptomatic hyperuricemia, an independent risk factor for CVD, and therefore impacts CVD prevention.

Specific embodiments of the disclosure provide for confirmation of the efficacy of folic acid or 5-MTHF on control of hyperuricemia and may be included with traditional Chinese herb medicines and/or other botanical ingredients as dietary health supplements, for example for the long term management of hyperuricemia. Folic acid may be one of components, and it may enhance the therapeutic efficacy of the combination therapy.

Examples of herbal extracts that may be included in the compositions include one or more of the following:

**Table 3: Herbal Extracts**

| Name |
|---|
| Acai Berry Extract Powder |
| African Mango Extract Powder |
| Ajuga Turkest Extract Powder |
| Alfalfa Extract Powder |
| Aloe Vera Extract Powder |
| American Ginseng Extract Powder |
| Amla Extract Powder |
| Andrographis Extract Powder |
| Aronia Extract Powder |
| Artichoke Extract Powder |
| Ashwagandha Extract Powder |
| Astragalus Extract Powder |
| Bamboo Extract Powder |
| Barley Extract Powder |
| Bearberry Leaf Extract (Uva Ursi) Powder |
| Bilberry Extract Powder |
| Bitter Melon Extract Powder |
| Black Cohosh Extract Powder |
| Black Garlic Extract Powder |
| Blood Orange Extract Powder |
| Boswellia Serrata Extract Powder |
| Broccoli Extract Powder |
| Buckthorn Bark Extract Powder |
| Burdock Root Extract Powder |
| Butcher's Broom Extract Powder |
| Butterbur Extract Powder |
| Caralluma Extract Powder |
| Cascara Sagrada Extract Powder |
| Cassia Seed Extract Powder |
| Cat's Claw Extract Powder |
| Catuaba Extract Powder |
| Cayenne Extract Capsules |
| Cayenne Extract Powder |
| Celery Seed Extract Powder |
| Chasteberry Extract Powder |
| Chitosan Extract Powder |
| Cinnamon Bark Extract Capsules |
| Cinnamon Bark Extract Powder |
| Cnidium Fruit Extract Powder |
| Cocoa Extract Powder |
| Coriolus Versicolor Extract (Turkey Tail) Powder |
| Corn Silk Extract Powder |
| Corydalis Extract Powder |
| Creek Yellow Grass Extract Powder |
| Curcumin 95% Natural Turmeric Extract Powder |
| Dandelion Root Extract Powder |
| Devil's Claw Extract Powder |
| Dragon Fruit Extract Powder |
| Echinacea Extract Powder |
| Elderberry Extract Powder |
| Eyebright Extract Powder |
| Flaxseed Extract Powder |
| Fo-Ti Extract Powder |
| Garlic Extract Powder |
| Ginger Root Extract Powder |
| Ginkgo Biloba Leaf Extract Powder |
| Ginseng Root Extract Powder |
| Grape Seed Extract Powder |
| Grapefruit Seed Extract Powder |
| Green Tea Extract (50% EGCG) Powder |
| Guanabana Extract Powder |
| Gymnema Extract Powder |
| Gynostemma Extract Powder |
| Hawthorn Berry Extract Powder |
| Hawthorn Leaf Extract Powder |
| Hibiscus Flower Extract Powder |
| Hoodia Extract Powder |
| Hops Extract Powder |
| Horse Chestnut Extract Powder |
| Horsetail Extract Powder |
| Kava Kava Extract Powder |
| Kelp Extract Powder |
| Kola Nut Extract Powder |
| Kudzu Root Extract Powder |
| Lemon Balm Extract Powder |
| Licorice Root Extract Powder |
| LongJack Extract (Tongkat Ali) Powder |
| Maca Root Extract Powder |
| Magnolia Extract Powder |
| Marshmallow Root Extract Powder |
| Milk Thistle Extract Powder |
| Moringa Extract Powder |
| Motherwort Extract Powder |
| Mucuna Pruriens Extract Powder |
| Muira Puama Extract Powder |
| Nettle Extract Powder |
| Oat Straw Extract Powder |
| Olive Leaf Extract Powder |
| Oolong Tea Extract Powder |
| Oyster Extract Powder |
| Papaya Fruit Extract Powder |
| Parsley Extract Powder |
| Passion Flower Extract Powder |
| Peppermint Extract Powder |
| Pine Bark Extract Powder |
| Pomegranate Extract Powder - (40% Ellagic Acid) |
| Portulaca Oleracea (Purslane) Extract Powder |
| Pumpkin Seed Extract Powder |
| Red Clover Extract Powder |
| Red Yeast Rice Extract (RYR) Powder |
| Reishi Mushroom Extract Powder |
| Rosemary Extract Powder |
| Sage Extract Powder |
| Saw Palmetto Extract Powder |
| Senna Leaf Extract Powder |
| Slippery Elm Bark Extract Powder |
| Spearmint Extract Powder |
| Spinach Extract Powder |
| St John's Wort Extract Powder |
| Tart Cherry Extract Powder |
| Taxillus Chinensis Danser Extract Powder |
| Turkey Rhubarb Extract Powder |
| Valerian Root Extract Powder |
| White Mulberry Fruit Extract Powder |
| White Willow Bark Extract 15% Capsules |
| White Willow Bark Extract Powder |
| Wild Yam Extract Powder |
| Yohimbe Extract Powder |

In addition, one or more of the following compositions may be utilized in any composition(s) in the disclosure. These vitamins may have additive or synergistic effects with composition(s) of the disclosure, such as for controlling hyperuricemia, oxidative stress and/or hyperhomocysteinemia, for example, which are risk factors for cardiovascular diseases and many other diseases. Therefore, any composition of the disclosure, including those that employ the following, may be utilized as a preventative composition.

**Table 4: Selected vitamin supplements**

| Names |
|---|
| Vitamin A |
| Vitamin D3 |
| Vitamin B1 |
| Vitamin B2 |
| Vitamin B6 |
| Vitamin B12 |
| Nicotinic acid (B3) |
| Folic acid (B9) |
| Methyl Folate (5-MTHF) |
| Biotin (B7) |
| Choline |
| Vitamin C |
| Vitamin K |
| Pantothenic acid |
| Vitamin E |

The following items in Table 5 may be utilized in any composition(s) of the present disclosure.

**Table 5: Additional herb extracts**

| Description |
|---|
| 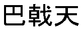 Extract 5:1100g \| Ba Ji Tian Extract 5:1 \| Morinda Extract 5:1 |
| 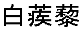 Extract 5:1100g \| Bai Ji Li Extract 5:1 \| Tribulus (Fruit) Extract 5:1 |
| 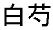 Extract 5:1100g \| Bai Shao Extract 5:1 \| Chinese Peony Extract 5:1 |
| 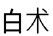 Extract 10:1 100g \| Bai Zhu Extract 10:1 \| Bai-Zhu Atractylodes (Rhizome) 10:1 |
| 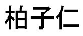 Extract 10:1 100g \| Bo Zi Ren Extract 10:1 \| Platycladi Seed Extract 10:1 |
| 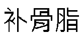 Extract 5:1100g \| Bu Gu Zhi Extract 5:1 \| Psoraleae Extract 5:1 |
| 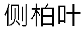 Extract 10:1100g \| Ce Bai Ye Extract 10:1 \| Oriental Arborvitae (Leaf & Branch Tip) Extract 10:1 |
| 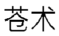 Extract 5:1100g \| Chang Zhu Extract 5:1 \| Cang-Zhu Atractylodes (Rhizome) Extract 5:1 |
| 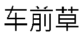 Extract \| Che Qian Cao Extract 5:1 \| Asian Plantain Extract 5:1 |
| Extract 5:1100g \| Che Qian Zi Extract 5:1 \| Asian Plantain (Seed) Extract 5:1 |
| 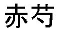 Extract 5:1100g \| Chi Shao Extract 5:1 \| Chinese Peony Extract 5:1 |
| 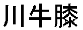 Extract 5:1100g \| Chuan Niu Extract 5:1 \| Cyathula Extract 5:1 |
| 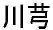 Extract Extract 5:1100g \| Chuan Xiong Extract 5:1 \| Sichuan Lovage |
| Extract 5:1 |
| 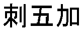 Extract 5:1100g \| Ci Wu Jia Cha Extract 5:1 \| Siberian Ginseng Extract 5:1 |
| 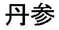 Extract Extract 5:1100g \| Dan Shen Extract 5:1 \| Chinese Salvia (Root & Rhizome) Extract 5:1 |
| 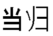 Extract 5:1100g \| Dang Gui Extract 5:1 \| Radix Angelicae Sinensis Extract 5:1 |
| 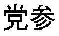 Extract 5:1100g \| Dang Shen Extract 5:1 \| Radix Codonopsis Extract 5:1 |
| 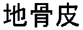 Extract 5:1100g \| Di Gu Pi Extract 5:1 \| Lycium Extract 5:1 |
| 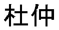 Extract 5:1100g \| Du Zhong Extract 5:1 \| Eucommia Extract 5:1 |
| 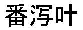 Extract 5:1100g \| Fan Xie Ye Extract 5:1 \| Senna (Leaf) Extract 5:1 |
| 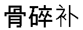 Extract 5:1100g \| Gu Sui Bu Extract 5:1 \| Drynaria (Rhizome) Extract 5:1 |
| 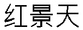 Extract 10:1 100g \| Hong Jing Tian Extract 10:1 \| Rhodiola rosea linn Extract 10:1 |
| 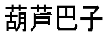 Extract 5:1 100g \| Hu Lu Ba Zi Extract 5:1 \| Common Fenugreek Seed Extract 5:1 |
| 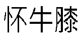 Extract 5:1100g \| Huai Niu Xi Extract 5:1 \| Achyranthes (Root) Extract 5:1 |
| 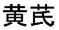 Extract 10:1100g \| Huang Qi Extract 10:1 \| Astragalus (Root) Extract 10:1 |
| 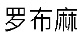 Extract 5:1 100g \| Luo Bu Ma Extract 5:1 \| Dogbane Leaf Extract 5:1 |
| 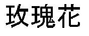 Extract 5:1100g \| Mei Gui Hua Extract 5:1 \| Rugose Rose (Flower Bud) Extract 5:1 |
| 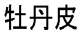 Extract 5:1100g \| Mu Dan Pi Extract 5:1 \| Tree Peony (Root Bark) Extract 5:1 |
| 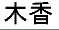 Extract 5:1100g \| Mu Xiang Extract 5:1 \| Radix Aucklandiae 5:1 |
| 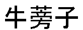 Extract 5:1100g \| Niu Bang Zi Extract 5:1 \| Burdock (Fruit) Extract 5:1 |
| 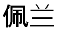 Extract 5:1100g \| Pei Lan Extract 5:1 \| Chinese Eupatorium (Aboveground Parts) Extract 5:1 |
| 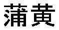 Extract 5:1100g \| Pu Huang Extract 5:1 \| Cattail (Pollen) Extract 5:1 |
| 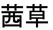 Extract 5:1100g \| Qian Cao Extract 5:1 \| Indian Madder (Root & Rhizome) Extract 5:1 |
| 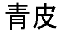 Extract 5:1100g \| Qing Pi Extract 5:1 \| Chinese Ash (Stem Bark) Extract 5:1 |
| 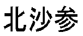 Extract 5:1100g \| Bei Sha Shen Extract 5:1 \| Glehnia (Root) Extract 5:1 |
| 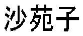 Extract 5:1100g \| Shan Yuan Zi Extract 5:1 \| Flatstem Milkvetch Seed Extract 5:1 |
| 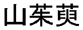 Extract 100g \| Shan Zhu Yu Extract 5:1 \| Asiatic Dogwood (Fruit without Seed) Extract 5:1 |
| 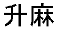 Extract 5:1100g \| Sheng Ma Extract 5:1 \| Chinese Cimicifuga (Rhizome) Extract 5:1 |
| 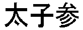 Extract 5:1100g \| Tai Zi Shen Extract 5:1 \| Pseudostellaria (Root Tuber) Extract 5:1 |
| 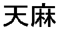 Extract 5:1100g \| Tian Ma Extract 5:1 \| Gastrodia (Rhizome) Extract 5:1 |
| 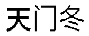 Extract 5:1100g \| Tian Men Dong Extract 5:1 \| Chinese Asparagus (Root Tuber) Extract 5:1 |
| 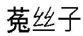 Extract 5:1100g \| Tu Si Zi Extract 5:1 \| Cuscutae Semen Extract 5:1 |
| 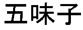 Extract 5:1 100g \| Wu Wei Zi Extracts 5:1 \| Schisandra (Fruit) Extract 5:1 |
| 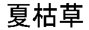 Extract 5:1100g \| Xia Ku Cao Extract 5:1 \| Heal All (Fruit Spike) Extract 5:1 |
| 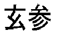 Extract 5:1100g \| Xuan Shen Extract 5:1 \| Scrophularia (Root) Extract 5:1 |
| 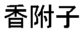 Extract 5:1 \| Xiang Fu Zi Extract 5:1 \| Rhizoma Cyperi / Nutgrass Galingale Rhizome Extract 5:1 |
| 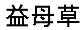 Extract 5:1100g \| Yi Mu Cao Extract 5:1 \| Chinese Motherwort (Aboveground Parts) Extract 5:1 |
| 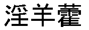 Extract 5:1100g \| Yin Yang Huo Extract 5:1 \| Epimedium (Aboveground Parts) Extract 5:1 |
| 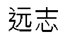 Extract 10:1100g \| Yuan Zhi Extract 10:1 \| Radix Polygalae Extract 10:1 |
| 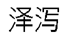 Extract 5:1100g \| Ze Xie Extract 5:1 \| Asian Water Plantain (Rhizome) Extract 5:1 |
| 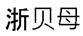 Extract 5: 100g \| Zhe Bei Mu Extract 5:1 \| Bulbus Fritillariae Thunbergii Extract 5:1 |
| 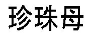 Extract 10:1 100g \| Zhen Zhu Mu Extract 10:1 \| Nacre (Calcined) Extract 10:1 |
| 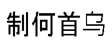 Extract 5:1100g \| He Shou Wu (Zhi) Extract 5:1 \| Processed Fo-Ti (Cured Root Tuber) Extract 5:1 |
| 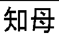 Extract 5:1100g \| Zhi Mu Extract 5:1 \| Anemarrhena (Rhizome) Extract 5:1 |
| 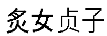 Extract 5:1100g \| Zhi Nu Zhen Zin Extract 5:1 \| Ligustrum (Fruit, Cured) Extract 5:1 |
| 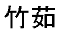 Extract 5:1100g \| Zhu Ru Extract 5:1 \| Caulis Bambusae In Taenia Extract 5:1 |

An example of an experimental design is as follows. For initial screening individual extracts for XO inhibitory and antioxidant effects, one may employ any suitable method include at least the following:

### Method (1):

Xanthine oxidase (XO) inhibition assay (using one or more substrate concentrations for screening). The detailed method is described in a previous publication (132).

### Method (2):

DPPH (2,2-diphenyl-1-picrylhydrazyl) scavenging assay (using one substrate concentration for screening). The abilities of the tested compounds to scavenge DPPH radicals are measured optically by monitoring the decrease of the absorption at 429 nm. The compounds' antioxidant activities are compared to those of vitamin C and vitamin E. The detailed method is described in our previous publication (132).

One may search and develop effective recipes/formulas based on selected effective health supplement raw materials (a variety of substrates and combinations, additive effects, synergistic effects can be performed). If initial screening results from raw materials identify a group of extracts that inhibit XO activity, one can utilize the effective standard extracts for further characterization studies. The concentration range of these selected materials is expanded and mixed with other materials. In some cases, effects of different combinations of individual herb extract with folic acid, 5-MTHF or other herb extracts on XO inhibition and antioxidant potential may be tested in *in vitro* assays. Any additive or synergistic effects of these combinations is characterized. If additive or synergistic effects of components exist, one can utilize such component(s) for combinational formulations (for example, with small amounts of each components in the combination) to achieve the maximal therapeutic effects, while reducing potential side effects of each component and reducing the potential risk of drug resistance for long-term management of hyperuricemia, in at least some cases.

### Use of acute (short-term) animal experiments to characterize the effectiveness and toxicity of dietary health supplement recipes/formulas for controlling hyperuricemia

### Materials for Studies:

According to the results of *in vitro* experiments, effective recipes/formulas of combinational herb extracts with or without folic acid/5-MTHF are characterized in animal studies. In specific embodiments, each formulation may contain 3 to 5 individual extracts or natural compounds/vitamin supplements. One may or may not test individual ingredients in animal experiments prior to testing them in the combination formulations in animal experiments. In specific embodiments, only recipes/formulas as a whole are characterized in animal experiments.

### Example of a Study Design:

A. Formulation and characterization of selected combination recipes for animal studies

Based on initial studies, DMSO-soluble factions of herb extracts had stronger XO inhibitory effects than their water-soluble fractions. DMSO dissolves both polar and non-polar compounds in the extract. DMSO is effective for getting a wide range of compounds into solution. Each herb extract selected from initial studies are further extracted in DMSO and freeze-dried (lyophilized). XO inhibitory effect and antioxidant potential of each DMSO extract are reconfirmed by *in vitro* XO activity assay and DPPH scavenging assay, respectively. One or more oils, such as one or more cooking oils (for example, oleic acid, isopropyl myristate, medium chain triglyceride, olive oil, castor oil, peanut oil, corn oil, sesame oil, soybean oil, almond oil, linseed oil, rapeseed oil, sunflower oil, coconut oil, groundnut oil, and/or palm oil) may be selected to mix these extracts for animal administration (oral gavage).

### B. Acute model of hyperuricemia and toxicity in mice

One can determine the hypouricemic effect of the formulation in allantoxanamide-treated mice. Intraperitoneal (i.p.) injection of uricase inhibitor allantoxanamide can effectively block the conversion of uric acid to 5-hydroxyisourate and thus cause a marked increase in serum uric acid levels in mice, providing a hyperuricemic animal model. One can characterize the hypouricemic effect of selected formulations in a well characterized mouse model (Table 6). Selected formulations will administered to the mouse by oral gavage. The detailed method is described in a previous publication (132).

**Table 6: Examples of animal assignments for studying the hypouricemic effect of XO inhibitors**

| **Group** | **Allantoxan** | **Treatment** | **End Point** |
|---|---|---|---|
| 1 | - | PEG400 (Control) | Serum uric acid |
| 2 | + | PEG400 (Control) | Serum uric acid |
| 3 | + | Formulation 1, low dose | Serum uric acid |
| 4 | + | Formulation 1, high dose | Serum uric acid |
| 5 | + | Formulation 2, low dose | Serum uric acid |
| 6 | + | Formulation 2, high dose | Serum uric acid |
| 7 | + | Allopurinol, 50 mg/kg | Serum uric acid |
| 8 | + | Allopurinol, 100 mg/kg | Serum uric acid |

Toxicity in mice. One can investigate the potential toxicity of selected formulations in mice, focusing on low dose and high dose (oral administration). Negative control and Allopurinol control may be included. Formulated health supplements may be administrated into mice by oral garage once a day for 15 days. Major end points include general health conditions and body weight, blood counts, blood chemistry and enzymes (including liver, heart and kidney functional panels), and organ histology (Table 7).

**Table 7. Animal assignments for studying the potential toxicity of XO inhibitors**

| **Group** | **Treatment** | **Frequency** | **Sacrific** | **End Points** |
|---|---|---|---|---|
| 1 | PEG400 (Control) | Once daily | 15 days | Mortality |
| 2 | Formulation 1, low | Once daily | 15 days | General health |
| 3 | Formulation 1, | Once daily | 15 days | body weight |
| 4 | Formulation 2, low | Once daily | 15 days | Blood counts |
| 5 | Formulation 2, | One time | 15 days | Blood chemistry |
| 6 | Allopurinol, 50 | One time | 15 days | Organ histology |
| 7 | Allopurinol, 100 | One time | 15 days | |

### Use of chronic (relatively long-term) animal experiments to test the effectiveness and toxicity of dietary health supplement recipes/formulas for controlling hyperuricemia

### Study Design:

Chronic model of hyperuricemia in uricase knockout mice: In mice and many other mammals, normal blood levels of uric acid are relatively low (1-2 mg/dL) because uric acid is further oxidized to allantoin by uricase (Chen, Lu, *et al.*, 2016). The loss of uricase in humans and higher primates occurred about 15 million years ago, resulting in a relatively higher serum uric acid (SUA) level than that in these lower animals. In order to establish an animal model of chronic hyperuricemia, scientists have created uricase (Uox) gene knockout mice that show severe hyperuricemia and urate nephropathy (133). Under the maintenance of Allopurinol, adult uricase-/- mice show relatively low SUA (2-4 mg/dL). However, once Allopurinol is discontinued, uricase-/- mice will have high SUA levels (6-10 mg/dL) in one week. The long-term hypouricemia effect of selected formulations can be determined by using uricase knockout mice. Selected formulations will be administrated through oral gavage once a day for 2 weeks or through drinking water for 2 months. Serum uric acid level will be tested every two days or once a week. During the study, general health conditions including body weight will be monitored. Blood counts, blood chemistry and enzymes (including liver, heart and kidney functional panels), and organ histology will be studied when the mice will be sacrificed (Table 6). It is estimated that about 500 g of each material would be needed for the current experiments. Each herb extract selected from PLAN I study will be further extracted in DMSO and freeze-dried. XO inhibitory effect and antioxidant potential of each DMSO extract will be reconfirmed by in vitro XO activity assay and DPPH scavenging assay, respectively. One or more oils, such as one or more cooking oils (for example, oleic acid, isopropyl myristate, medium chain triglyceride, olive oil, castor oil, peanut oil, corn oil, sesame oil, soybean oil, almond oil, linseed oil, rapeseed oil, sunflower oil, coconut oil, groundnut oil, and/or palm oil) may be selected to mix these extracts for animal administration (oral gavage).

**Table 8. Uricase-/- mice assignments for studying hypouricemic effect and the potential toxicity of XO inhibitors**

| Group (n=10) | Treatment (10 mg/kg) | Frequency | Sacrifice | End Points |
|---|---|---|---|---|
| 1 | Oral gavage, PEG400 (Control) | Once daily | 15 days | Mortality Serum uric acid |
| | | | | General health conditions and body weight |
| 2 | Oral gavage formulation | Once daily | 15 days | |
| | | | | Blood counts Blood chemistry Organ histology |
| 3 | Oral gavage Allopurinol | Once daily | 15 days | |
| 4 | Drinking water (Control) | Continuously | 60 days | |
| 5 | Drinking water, formulation | Continuously | 60 days | |
| 6 | Drinking water, Allopurinol | Continuously | 60 days | |

### References for Examples 1-3

1. Benjamin EJ, Virani SS, Callaway CW, Chamberlain AM, et al. American Heart Association Council on Epidemiology and Prevention Statistics Committee and Stroke Statistics Subcommittee. Heart Disease and Stroke Statistics-2018 Update: A Report From the American Heart Association. Circulation. 2018 Mar 20;137(12):e67-e492.
2. Galassi FM, Borghi C. A brief history of uric acid: from gout to cardiovascular risk factor. Eur J Intern Med. 2015;26:373.
3. Borghi C, Desideri G. Urate-lowering drugs and prevention of cardiovascular disease: The emerging role of xanthine oxidase inhibition. Hypertension, 2016; 67(3): 496-98
4. Li M, Hu X, Fan Y et al: Hyperuricemia and the risk for coronary heart disease morbidity and mortality a systematic review and dose-response meta-analysis. Sci Rep, 2016; 6: 19520
5. Storhaug HM, Norvik JV, Toft I et al: Uric acid is a risk factor for ischemic stroke and all-cause mortality in the general population: A gender specific analysis from The Tromsø Study. BMC Cardiovasc Disord, 2013; 13: 115
6. Tian Zuo, Xuehui Liu et al. Hyperuricemia and coronary heart disease mortality: a meta-analysis of prospective cohort studies. BMC Cardiovascular Disorders, 2016: 16:207.
7. Loeffler LF, Navas-Acien A, Brady TM, et al. Uric acid level and elevated blood pressure in US adolescents: national health and nutrition examination survey, 1999-2006. Hypertension, 2012, 59(4): 811-817.
8. Fan Y, Wei F, Lang Y, et al. Losartan treatment for hypertensive patients with hyperuricaemia in Chinese population: a meta-analysis. J Hypertens, 2015, 33(4): 681-688; discussion 689.
9. Kuwabara M, Niwa K, Hisatome I. Hyperuricemia is an independent risk factor of atrial fibrillation. Journal of the American College of Cardiology 2014:63(12) Supplement. DOI: 10.1016/S0735-1097(14)60469-1
10. Alderman MH: Serum uric acid as a cardiovascular risk factor for heart disease. Curr Hypertens Rep 2001; 3: 184-189.
11. Kuwabara M, Niwa K, Nishi Y, et al: Relationship between serum uric acid levels and hypertension among Japanese individuals not treated for hyperuricemia and hypertension. Hypertens Res 2014; 37: 785-789.
12. Grayson PC, et al. Hyperuricemia and incident hypertension: a systematic review and meta-analysis. Arthritis Care Res (Hoboken). 2011;63(1): 102-10.
13. Mazzali M, Hughes J, Kim YG, et al. Elevated uric acid increases blood pressure in the rat by a novel crystal-independent mechanism. Hypertension, 2001, 38(5): 1101-1106.
14. Biscaglia S, Ceconi C, Malagù M, et al. Uric acid and coronary artery disease: an elusive link deserving further attention. Int J Cardiol, 2016, 213: 28-32.
15. Lv Q, et al. High serum uric acid and increased risk of type 2 diabetes: a systemic review andmeta-analysis of prospective cohort studies. PLoS One. 2013;8(2):e56864.
16. Zhu Y, et at. Prevalence of gout and hyperuricemia in the U.S. general population: The National Health and Nutrition Examination Survey 2007-2008. Arthritis Rheum. 2011, 63, 3136-3141.
17. Liu R, Han C, Wu D et al: Prevalence of hyperuricemia and gout in mainland China from 2000 to 2014: A systematic review and meta-analysis. Biomed Res Int, 2015; 2015: 762820
18. Smith E, et al. Global Prevalence of Hyperuricemia: A Systematic Review of Population-Based Epidemiological Studies. Arthritis Rheumatol. 2015; 67 (suppl 10).
19. Chou CT, Lai JS. The epidemiology of hyperuricaemia and gout in Taiwan aborigines. Br J Rheumatol, 1998;37(3):258-62.
20. Chen C, Lü JM, Yao Q. Hyperuricemia-Related Diseases and Xanthine Oxidoreductase (XOR) Inhibitors: An Overview. Med Sci Monit. 2016 Jul 17;22:2501-12.
21. MacIsaac RL, Salatzki J, Higgins P, Walters MR, Padmanabhan S, Dominiczak AF, et al. Allopurinol and cardiovascular outcomes in adults with hypertension. Hypertension 2016;67, 535-540.
22. Chen JH, Lan JL, Cheng CF, et al. Effect of urate-lowering therapy on the risk of cardiovascular disease and all-cause mortality in patients with gout: a case-matched cohort study. J Rheumatol. 2015;42(9):1694-701.
23. Chen JH, Lan JL, Cheng CF, et al. Effect of urate-lowering therapy on all-cause and cardiovascular mortality in hyperuricemic patients without gout: a case-matched cohort study. PLoS One. 2015;10(12):e0145193.
24. Luk AJ, Levin GP, Moore EE, et al. Allopurinol and mortality in hyperuricaemic patients. Rheumatology (Oxford) 2009;48:804-6
25. Dubreuil M, Zhu Y, Zhang Y, et al. Allopurinol initiation and all cause mortality in the general population. Ann Rheum Dis 2014;74:1368-7.
26. Sluijs I, Beulens JW, van der A DL, Spijkerman AM, Schulze MB, van der Schouw YT. Plasma uric acid is associated with increased risk of type 2 diabetes independent of diet and metabolic risk factors. J Nutr. 2013 Jan;143(1):80-5. doi: 10.3945/jn.112.167221. Epub 2012 Nov 21.
76. Krishnan E, Pandya BJ, Chung L, Hariri A, Dabbous O. Hyperuricemia in young adults and risk of insulin resistance, prediabetes, and diabetes: a 15-year follow-up study. Am J Epidemiol. 2012 Jul 15;176(2):108-16. doi: 10.1093/aje/kws002. Epub 2012 Jul 2.
28. Li C, Hsieh MC, Chang SJ. Metabolic syndrome, diabetes, and hyperuricemia. Curr Opin Rheumatol. 2013 Mar;25(2):210-6. doi: 10.1097/BOR.0b013e32835d951e. Review.
29. Chang HY, Tung CW, Lee PH, Lei CC, Hsu YC, Chang HH, Yang HF, Lu LC, Jong MC, Chen CY, Fang KY, Chao YS, Shih YH, Lin CL. Hyperuricemia as an independent risk factor of chronic kidney disease in middle-aged and elderly population. Am J Med Sci. 2010 Jun;339(6):509-15. doi: 10.1097/MAJ.0b013e3181db6e16.
30. Tsuruta N, Imafuku S, Narisawa Y. Hyperuricemia is an independent risk factor for psoriatic arthritis in psoriatic patients. J Dermatol. 2017 Dec;44(12):1349-1352. doi: 10.1111/1346-8138.13968. Epub 2017 Jul 10.
31. Chang H-Y, Lee P-H, Lei C-C, Tung C-W, Hsu Y-C, Huang T-J, et al. (2013) Hyperuricemia Is an Independent Risk Factor for New Onset Micro-Albuminuria in a Middle-Aged and Elderly Population: A Prospective Cohort Study in Taiwan. PLoS ONE 8(4): e61450. https://doi.org/10.1371/journal.pone.0061450
32. Long H, Jiang J, Xia J, Jiang R, He Y, Lin H, Fan Z, Zeng T. Hyperuricemia Is an Independent Risk Factor for Erectile Dysfunction. J Sex Med. 2016 Jul;13(7):1056-62. doi: 10.1016/j.jsxm.2016.04.073. Epub 2016 May 18.
33. Doherty A, Carvalho JC, Drewlo S, El-Khuffash A, Downey K, Dodds M, Kingdom J. Altered hemodynamics and hyperuricemia accompany an elevated sFlt-1/PIGF ratio before the onset of early severe preeclampsia. J Obstet Gynaecol Can. 2014 Aug;36(8):692-700. doi: 10.1016/S1701-2163(15)30511-9.
34. Springer J, Tschirner A, Hartman K, Palus S, Wirth EK, Ruis SB, Möller N, von Haehling S, Argiles JM, Köhrle J, Adams V, Anker SD, Doehner W. Inhibition of xanthine oxidase reduces wasting and improves outcome in a rat model of cancer cachexia. Int J Cancer. 2012 Nov 1;131(9):2187-96. doi: 10.1002/ijc.27494. Epub 2012 Jul 9.
35. Zhou FL, Zhang WG, Wei YC, Meng S, Bai GG, Wang BY, Yang HY, Tian W, Meng X, Zhang H, Chen SP. Involvement of oxidative stress in the relapse of acute myeloid leukemia. J Biol Chem. 2010 May 14;285(20):15010-5. doi: 10.1074/jbc.M110.103713. Epub 2010 Mar 16.
36. Honorat JA, Kinoshita M, Okuno T, Takata K, Koda T, Tada S, Shirakura T, Fujimura H, Mochizuki H, Sakoda S, Nakatsuji Y. Xanthine oxidase mediates axonal and myelin loss in a murine model of multiple sclerosis. PLoS One. 2013 Aug 8;8(8):e71329. doi: 10.1371/journal.pone.0071329. eCollection 2013.
37. Peglow S, Toledo AH, Anaya-Prado R, Lopez-Neblina F, Toledo-Pereyra LH. Allopurinol and xanthine oxidase inhibition in liver ischemia reperfusion. J Hepatobiliary Pancreat Sci. 2011 Mar;18(2):137-46. doi: 10.1007/s00534-010-0328-7. Review.
38. Kim NH, Choi S, Han EJ, Hong BK, Choi SY, Kwon HM, Hwang SY, Cho CS, Kim WU. The xanthine oxidase-NFAT5 pathway regulates macrophage activation and TLR-induced inflammatory arthritis. Eur J Immunol. 2014 Sep;44(9):2721-36. doi: 10.1002/eji.201343669. Epub 2014 Aug 11.
39. Fang J, Yin H, Liao L, Qin H, Ueda F, Uemura K, Eguchi K, Bharate GY, Maeda H. Water soluble PEG-conjugate of xanthine oxidase inhibitor, PEG-AHPP micelles, as a novel therapeutic for ROS related inflammatory bowel diseases. J Control Release. 2016 Feb 10;223:188-196. doi: 10.1016/j.jconrel.2015.12.049. Epub 2015 Dec 29.
40. CHMP Assessment Report For Adenuric (International Nonproprietary Name: Febuxostat), Procedure No. EMEA/H/C/777, European Medicines Agency (EMEA) Evaluation of Medicines for Human Use. EMEA/258531/2008
41. Bohm M, Vuppalanchi R, Chalasani N, Drug-Induced Liver Injury Network (DILIN): Febuxostat-induced acute liver injury. Hepatology, 2016; 63(3): 1047-49
42. Reinders MK, Jansen TL (2010) Management of hyperuricemia in gout: focus on febuxostat. ClinInterv Aging, 2010;5: 7-18.
43. Gray CL, Walters-Smith NE (2011). Febuxostat for treatment of chronic gout. Am J Health Syst Pharm 2011; 68: 389-398.
44. Chen C, Lü JM, Yao Q. Hyperuricemia-Related Diseases and Xanthine Oxidoreductase (XOR) Inhibitors: An Overview. Med Sci Monit. 2016 Jul 17;22:2501-12.
45. Chohan S: Safety and efficacy of febuxostat treatment in subjects with gout and severe allopurinol adverse reactions. J Rheumatol, 2011; 38: 1957-59
46. Abeles AM: Febuxostat hypersensitivity. J Rheumatol, 2012; 39: 659
47. Schumacher HR Jr., Becker MA, Wortmann RL et al: Effects of febuxostat versus allopurinol and placebo in reducing serum urate in subjects with hyperuricemia and gout: A 28-week, phase III, randomized, double-blind, parallel-group trial. Arthritis Rheum, 2008; 59(11): 1540-48
48. www.accessdata.fda.gov. Accessed April 27, 2018
49. Mauck M, Taintor A, Jha P: Cross-sensitivity of allopurinol and febuxostat-induced drug rash with eosinophilia and systemic symptoms (DRESS) syndrome. J Gen Intern Med, 2010; 25: 5504-5
50. Bardin T, Chalès G, Pascart T et al: Risk of cutaneous adverse events with febuxostat treatment in patients with skin reaction to allopurinol. A retrospective, hospital-based study of 101 patients with consecutive allopurinol and febuxostat treatment. Joint Bone Spine, 2016; 83(3): 314-17
51. Kang Y, Kim MJ, Jang HN et al: Rhabdomyolysis associated with initiation of febuxostat therapy for hyperuricaemia in a patient with chronic kidney disease. J Clin Pharm Ther, 2014; 39(3): 328-30
52. Lewis AS, Murphy L, McCalla C, Fleary M, Purcell S. Inhibition of mammalian xanthine oxidase by folate compounds and amethopterin. J Biol Chem 1984;259:12-5.
53. Williams JN Jr., Nichol CA, Elvehjem CA. Relation of dietary folic acid and vitamin B12 to enzyme activity in the chick. J Biol Chem 1949;180:689-94.
54. Qin X, Li Y, He M, Tang G, Yin D, Liang M, Wang B, Nie J, Huo Y, Xu X, Hou FF. Folic acid therapy reduces serum uric acid in hypertensive patients: a substudy of the China Stroke Primary Prevention Trial (CSPPT). Am J Clin Nutr. 2017 Apr;105(4):882-889.
55. Li H, Qin X, Xie D, Tang G, Zhang Y, Li J, Hou F, Wang X, Huo Y, Xu X. Effects of combined enalapril and folic acid therapy on the serum uric acid levels in hypertensive patients: a multicenter, randomized, double-blind, parallel-controlled clinical trial. Intern Med. 2015;54(1):17-24.
56. Qin X, Xu M, Zhang Y, Li J, Xu X, Wang X, Xu X, Huo Y. Effect of folic acid supplementation on the progression of carotid intima-media thickness: a meta-analysis of randomized controlled trials. Atherosclerosis. 2012 Jun;222(2):307-13.
57. Huo Y, Li J, Qin X, Huang Y, Wang X, Gottesman RF, Tang G, et al. Efficacy of folic acid therapy in primary prevention of stroke among adults with hypertension in China: the CSPPT randomized clinical trial. JAMA. 2015 Apr 7;313(13):1325-35.
58. Huo Y, Qin X, Wang J, Sun N, Zeng Q, Xu X, Liu L, Xu X, Wang X. Efficacy of folic acid supplementation in stroke prevention: new insight from a meta-analysis. Int J Clin Pract. 2012 Jun;66(6):544-51.
59. Li Y, Huang T, Zheng Y, Muka T, Troup J, Hu FB. Folic Acid Supplementation and the Risk of Cardiovascular Diseases: A Meta-Analysis of Randomized Controlled Trials. J Am Heart Assoc. 2016 Aug 15;5(8).
60. Horiuchi H, Ota M, Kobayashi M, Kaneko H, Kasahara Y, Nishimura S, Kondo S, Komoriya K.A comparative study on the hypouricemic activity and potency in renal xanthine calculus formation of two xanthine oxidase/xanthine dehydrogenase inhibitors: TEI-6720 and allopurinol in rats. Res Commun Mol Pathol Pharmacol. 1999;104(3):307-19.
61. 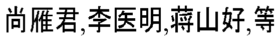 . Acteoside 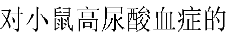 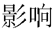 . 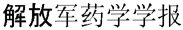 , 2004,22(1):30 32
62. Lopes Galeno DM, Carvalho RP, Boleti AP, Lima AS, Oliveira de Almeida PD, Pacheco CC, Pereira de Souza T, Lima ES. Extract from Eugenia punicifolia is an antioxidant and inhibits enzymes related to metabolic syndrome. Appl Biochem Biotechnol. 2014 Jan;172(1):311-24.
63. Schmeda-Hirschmann G, Theoduloz C, Franco L, Ferro E, de Arias AR. Preliminary pharmacological studies on Eugenia uniflora leaves: xanthine oxidase inhibitory activity. J Ethnopharmacol. 1987 Nov;21(2):183-6.
64. Suzuki R, Hasuike Y, Hirabayashi M, Fukuda T, Okada Y, Shirataki Y. Identification of a xanthine oxidase-inhibitory component from Sophora flavescens using NMR-based metabolomics. Nat Prod Commun. 2013 Oct;8(10):1409-12.
65. 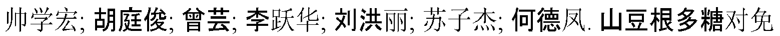 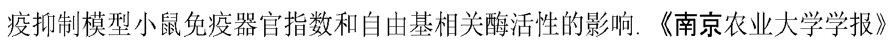 [ISSN:1000-2030/CN:32-1148/S] 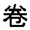 : 32 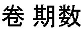 : 2009 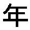 2 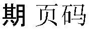 : 170-172.
66. Kong LD, Cai Y, Huang WW, Cheng CH, Tan RX. Inhibition of xanthine oxidase by some Chinese medicinal plants used to treat gout. J Ethnopharmacol. 2000 Nov;73(1-2):199-207.
67. 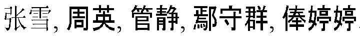 . 5 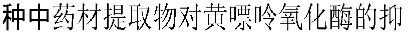 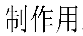 . 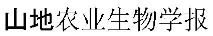 ; 2015 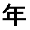 04 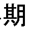 (2015/12/11), P51 - 54
68. 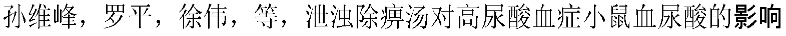 . 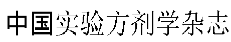 , 2006, 12 (9) : 36-38. 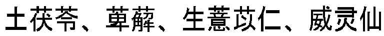 , 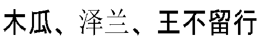
69. Liu L, Shi S, Zhao H, Yu J, Jiang X, Chen X. Selective fishing and analysis of xanthine oxidase binders from two Fabaceae species by coupling enzyme functionalized core-shell magnetic nanoparticles with HPLC-MS. J Chromatogr B Analyt Technol Biomed Life Sci. 2014 Jan 15;945-946:163-70.
70. Hudaib MM, Tawaha KA, Mohammad MK, Assaf AM, Issa AY, Alali FQ, Aburjai TA, Bustanji YK. Xanthine oxidase inhibitory activity of the methanolic extracts of selected Jordanian medicinal plants. Pharmacogn Mag. 2011 Oct;7(28):320-4. doi: 10.4103/0973-1296.90413.
71. 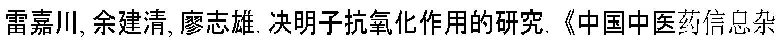 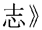 2006 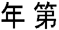 11 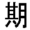
72. Peng MJ, Shi SY, Chen L, Zhang SH, Cai P, Chen XQ. Online coupling solid-phase ligand-fishing with high-performance liquid chromatography-diode array detector-tandem mass spectrometry for rapid screening and identification of xanthine oxidase inhibitors in natural products. Anal Bioanal Chem. 2016 Sep;408(24):6693-701.
73. Chien SC, Yang CW, Tseng YH, Tsay HS, Kuo YH, Wang SY. Lonicera hypoglauca inhibits xanthine oxidase and reduces serum uric acid in mice. Planta Med. 2009 Mar;75(4):302-6.
74. Nguyen MT, Awale S, Tezuka Y, Tran QL, Watanabe H, Kadota S. Xanthine oxidase inhibitory activity of Vietnamese medicinal plants. Biol Pharm Bull. 2004 Sep;27(9):1414-21.
75. Liu K, Wang W, Guo BH, Gao H, Liu Y, Liu XH, Yao HL, Cheng K. Chemical Evidence for Potent Xanthine Oxidase Inhibitory Activity of Ethyl Acetate Extract of Citrus aurantium L. Dried Immature Fruits. Molecules. 2016 Mar 2;21(3):302.
76. Hou PY, Mi C, He Y, et al. Pallidifloside D from Smilax riparia enhanced allopurinol effects in hyperuricemia mice. Fitoterapia, vol. 105, no. 1, pp. 43-48, 2015.
77. 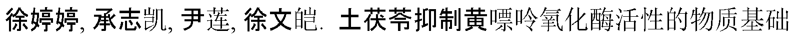 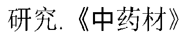 2012 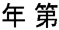 4 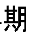
78. 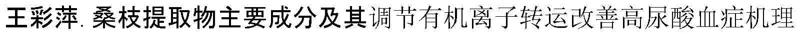 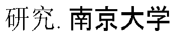 . 2009 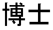 Thesis. 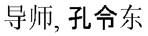
79. Hunyadi A, Liktor-Busa E, Márki A, Martins A, Jedlinszki N, Hsieh TJ, Báthori M, Hohmann J, Zupk6 I. Metabolic effects of mulberry leaves: exploring potential benefits in type 2 diabetes and hyperuricemia. Evid Based Complement Alternat Med. 2013;2013:948627.
80. Sang M, Du G, Hao J, Wang L, Liu E, Zhang Y, Wang T, Gao X, Han L. Modeling and optimizing inhibitory activities of Nelumbinis folium extract on xanthine oxidase using response surface methodology. J Pharm Biomed Anal. 2017 May 30; 139:37-43.
81. Tu PT, Tawata S. Anti-Oxidant, Anti-Aging, and Anti-Melanogenic Properties of the Essential Oils from Two Varieties of Alpinia zerumbet. Molecules. 2015 Sep 14;20(9): 16723-40.
82. 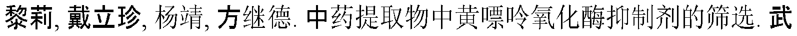 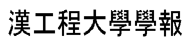 ; 32 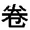 3 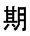 (2010/03 /30), P44 - 46
83. 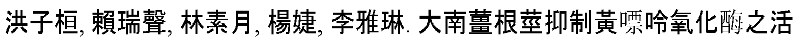 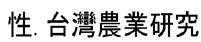 ; 66 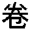 4 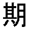 (2017 112/31), P333 - 342
84. Nguyen MT, Awale S, Tezuka Y, Ueda JY, Tran Ql, Kadota S. Xanthine oxidase inhibitors from the flowers of Chrysanthemum sinense. Planta Med. 2006 Jan;72(1):46-51.
85. Song HP, Zhang H, Fu Y, Mo HY, Zhang M, Chen J, Li P. Screening for selective inhibitors of xanthine oxidase from Flos Chrysanthemum using ultrafiltration LC-MS combined with enzyme channel blocking. J Chromatogr B Analyt Technol Biomed Life Sci. 2014 Jun 15;961:56-61.
86. Honda S, Kawamoto S, Tanaka H, Kishida H, Kitagawa M, Nakai Y, Abe K, Hirata D. Administered chrysanthemum flower oil attenuates hyperuricemia: mechanism of action as revealed by DNA microarray analysis. Biosci Biotechnol Biochem. 2014;78(4):655-61.
87. Lee YS, Son E, Kim SH, Lee YM, Kim OS, Kim DS. Synergistic Uric Acid-Lowering Effects of the Combination of <i>Chrysanthemum indicum</i> Linne Flower and <i>Cinnamomum cassia</i> (L.) J. Persl Bark Extracts. Evid Based Complement Alternat Med. 2017;2017:9764843.
88. Wang Z, Kwon SH, Hwang SH, Kang YH, Lee JY, Lim SS. Competitive binding experiments can reduce the false positive results of affinity-based ultrafiltration-HPLC: A case study for identification of potent xanthine oxidase inhibitors from Perilla frutescens extract. J Chromatogr B Analyt Technol Biomed Life Sci. 2017 Mar 24;1048:30-37.
89. Huo LN, Wang W, Zhang CY, Shi HB, Liu Y, Liu XH, Guo BH, Zhao DM, Gao H. Bioassay-Guided Isolation and Identification of Xanthine Oxidase Inhibitory Constituents from the Leaves of Perilla frutescens. Molecules. 2015 Sep 25;20(10):17848-59.
90. 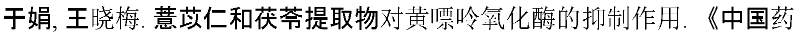 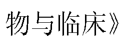 2014 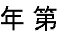 1 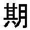 30-32 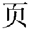
91. Costantino L, Albasini A, Rastelli G, Benvenuti S. Activity of polyphenolic crude extracts as scavengers of superoxide radicals and inhibitors of xanthine oxidase. Planta Med. 1992 Aug;58(4):342-4.
92. Liu F, Deng C, Cao W, Zeng G, Deng X, Zhou Y. Phytochemicals of Pogostemon Cablin (Blanco) Benth. aqueous extract: Their xanthine oxidase inhibitory activities. Biomed Pharmacother. 2017 May;89:544-548.
93. Kim Yi. Will mandatory folic acid fortification prevent or promote cancer? Am J Clin Nutr 80: 1123-1128, 2004.
94. Choi SW and Mason JB: Folate status: Effects on pathways of colorectal carcinogenesis. J Nutr 132 (Suppl 8): S2413-S2418, 2002.
95. Varela-Moreiras G, Murphy MM, Scott JM. Cobalamin, folic acid, and homocysteine. Nutr Rev. 2009 May. 67 Suppl 1: S69-72.
96. Reynolds EH, Rothfeld P, Pincus JH. Neurological disease associated with folate deficiency. Br Med J. 1973 May 19;2(5863):398-400.
97. Zaric BL, Obradovic M, Bajic V, Haidara MA, Jovanovic M, Isenovic ER. Homocysteine and Hyperhomocysteinaemia. Curr Med Chem. 2018 Mar 12. doi: 10.2174/0929867325666180313105949. [Epub ahead of print]
98. Joshi RAdhikari SPatro BSet al.. Free radical scavenging behavior of folic acid: evidence for possible antioxidant activity. Free Radic Biol Med . 2001;30:1390-1399.
99. Hathcock JN. Vitamins and minerals: efficacy and safety. Am J Clin Nutr. 1997 Aug;66(2):427-37.
100. Field MS, Stover PJ. Safety of folic acid. Ann N Y Acad Sci. 2018 Feb;1414(1):59-71.
101. Kim Yi. Will mandatory folic acid fortification prevent or promote cancer? Am J Clin Nutr 2004;80(5): 1123-1128.
102. Wien TN, Pike E, Wisloff T, Staff A, Smeland S, Klemp M. Cancer risk with folic acid supplements: a systematic review and meta-analysis. BMJ Open. 2012 Jan 12;2(1):e000653.
103. Ebbing M, Bønaa KH, Nygård O, Arnesen E, Cleland PM, Nordrehaug JE, et al. Cancer incidence and mortality after treatment with folic acid and vitamin B12. JAMA 2009;302(19): 2119-2126.
104. Kalckar HM, Klenow H. Milk xanthopterin oxidase and pteroylglutamic acid. J. Biol. Chem. 1948;172,349-52.
105. Kalckar HM, Kjeldgaard NO, Klenow H. 2-Amino-4hydroxy-6-formylpteridine, an inhibitor of purine and pterine oxidases. Biochim Biophys Acta. 1950 Jun;5(3/4):586-94.
106. Lewis AS, Murphy L, McCalla C, Fleary M, Purcell S. Inhibition of mammalian xanthine oxidase by folate compounds and amethopterin. J Biol Chem. 1984 Jan 10;259(1):12-5.
107. Spector T, Ferone R. Folic acid does not inactivate xanthine oxidase. J Biol Chem. 1984 Sep 10;259(17):10784-6.
108. Nishino T, Tsushima K. Interaction of milk xanthine oxidase with folic acid. Inhibition of milk xanthine oxidase by folic acid and separation of the enzyme into two fractions on Sepharose 4B/folate gel. J Biol Chem. 1986 Aug 25;261(24):11242-6.
109. Maciel ME, Castro GD, Castro JA. Inhibition of the rat breast cytosolic bioactivation of ethanol to acetaldehyde by some plant polyphenols and folic acid. Nutr Cancer. 2004;49(1):94-9.
110. Verhaar MC, Wever RM, Kastelein JJ, van Dam T, Koomans HA, Rabelink TJ. 5-methyltetrahydrofolate, the active form of folic acid, restores endothelial function in familial hypercholesterolemia. Circulation. 1998 Jan 27;97(3):237-41.
111. Akhtar MJ, Khan MA, Ahmad I. Photodegradation of folic acid in aqueous solution, J. Pharm. Biomed. Anal. 19 (1999) 269-275.
112. Branda RF, Eaton JW. Skin color and nutrient photolysis: an evolutionary hypothesis, Science 201 (1978) 625-626.
113. Hirakawa K, Suzuki H, Oikawa S, Kawanishi S. Sequencespecific DNA damage induced by ultraviolet A-irradiated folic acid via its photolysis product, Arch. Biochem. Biophys. 410 (2003) 261-268.
114. Jamil AM, Ataullah KM, Ahmad I. Identification of photoproducts of folic acid and its degradation pathways in aqueous solution, J. Pharm. Biomed. Anal. 31 (2003) 579-588.
115. Lucock M, Yates Z, Glanville T, Leeming R, Simpson N, Daskalakis I. A critical role for B-vitamin nutrition in human developmental and evolutionary biology, Nutrit. Res. 23 (2003) 1463-1475.
116. Thomas AH, Suarez G, Cabrerizo FM, Martino R, Capparelli AL. Study of the photolysis of folic acid and 6-formylpterin in acid aqueous solutions, Photochem. Photobiol. A: Chem. 135 (2000) 147-154.
117. Off MK, Steindal AE, Porojnicu AC, Juzeniene A, Vorobey A, Johnsson A, Moan J. Ultraviolet photodegradation of folic acid. J Photochem Photobiol B. 2005 Jul 1;80(1):47-55.
118. Keebaugh AC, Thomas JW. The Evolutionary Fate of the Genes Encoding the Purine Catabolic Enzymes in Hominoids, Birds, and Reptiles. Mol Biol Evol. 2010 Jun; 27(6): 1359-1369.
119. Keith CK, Broach WJ, et al. Xanthine oxidase and tyrosinase in the livers of chicks receiving graded levels of dietary pteroylglutamic acid. J Biol Chem. 1948 Dec;176(3):1095-1101.
120. Williams JN Jr, Nichol CA, Elvehjem CA. Relation of dietary folic acid and vitamin B12 to enzyme activity in the chick. J Biol Chem. 1949 Sep;180(2):689-94.
121. Jing M, Munyaka PM, Tactacan GB, Rodriguez-Lecompte JC, O K, House JD. Performance, serum biochemical responses, and gene expression of intestinal folate transporters of young and older laying hens in response to dietary folic acid supplementation and challenge with Escherichia coli lipopolysaccharide. Poult Sci. 2014 Jan;93(1):122-31.
122. Srivastava M, Chandra D, Kale RK. Modulation of radiation-induced changes in the xanthine oxidoreductase system in the livers of mice by its inhibitors. Radiat Res. 2002 Mar;157(3):290-7.
123. Wu X, Liu J, Zhang J, et al. Folic acid reverses uric acid crystal-induced surface OAT1 internalization by inhibiting RhoA activity in uric acid nephropathy. Molecular Medicine Reports. 2016;13(3):2385-2392.
124. Cui S, Li W, Lv X, Wang P, Gao Y, Huang G. Folic Acid Supplementation Delays Atherosclerotic Lesion Development by Modulating MCP1 and VEGF DNA Methylation Levels. In Vivo and In Vitro.Int J Mol Sci. 2017 May 5;18(5).
125. Camicer R, Navarro MA, Arbonés-Mainar JM, Acín S, Guzmán MA, Surra JC, Arnal C, de Las Heras M, Blanco-Vaca F, Osada J. Folic acid supplementation delays atherosclerotic lesion development in apoE-deficient mice. Life Sci. 2007 Jan 23;80(7):638-43.
126. Qipshidze N, Tyagi N, Sen U, Givvimani S, Metreveli N, Lominadze D, Tyagi SC. Folic acid mitigated cardiac dysfunction by normalizing the levels of tissue inhibitor of metalloproteinase and homocysteine-metabolizing enzymes postmyocardial infarction in mice. Am J Physiol Heart Circ Physiol. 2010 Nov;299(5):H1484-93.
127. Hwang SY, Siow YL, Au-Yeung KK, House J, O K. Folic acid supplementation inhibits NADPH oxidase-mediated superoxide anion production in the kidney. Am J Physiol Renal Physiol. 2011 Jan;300(1):F189-98.
128. Lu R, Wang X, Sun DF, Tian XQ, Zhao SL, Chen YX, Fang JY. Folic acid and sodium butyrate prevent tumorigenesis in a mouse model of colorectal cancer. Epigenetics. 2008 Nov;3(6):330-5.
129. Ishiguro L, Yang M, Sohn KJ, Streutker CJ, Grin A, Croxford R, Kim YI.,Folic Acid Supplementation Adversely Affects Chemosensitivity of Colon Cancer Cells to 5-fluorouracil. Nutr Cancer. 2016 Jul;68(5):780-90.
130. Lyu LC, Hsu CY, Yeh CY, Lee MS, Huang SH, Chen CL. A case-control study of the association of diet and obesity with gout in Taiwan. Am J Clin Nutr 2003;78:690-701.
131. Xu X, Qin X, Li Y, Sun D, Wang J, Liang M, Wang B, Huo Y, Hou FF. Efficacy of Folic Acid Therapy on the Progression of Chronic Kidney Disease: The Renal Substudy of the China Stroke Primary Prevention Trial. JAMA Intern Med. 2016 Oct 1;176(10):1443-1450.
132. Lü JM, Yao Q, Chen C. 3,4-Dihydroxy-5-nitrobenzaldehyde (DHNB) is a potent inhibitor of xanthine oxidase: a potential therapeutic agent for treatment of hyperuricemia and gout. Biochem Pharmacol. 2013 Nov 1;86(9):1328-37.
133. Wu X, Wakamiya M, Vaishnav S, Geske R, Montgomery C Jr, Jones P, Bradley A, Caskey CT. Hyperuricemia and urate nephropathy in urate oxidase-deficient mice. Proc Natl Acad Sci USA. 1994 Jan 18;91(2):742-6.

### EXAMPLE 4

### DEVELOPMENT OF HEALTH FOOD SUPPLEMENTS/ANTIOXIDANTS FOR CONTROLLING HYPERURICEMIA AND OXIDATIVE STRESS

As described herein, the inventors determined the effect of xanthine oxidase (XO) inhibition for 112 standard herbal extracts obtained from Bulk Supplements.com (111) and Trustworthy Herbs Inc (1) by an *in vitro* enzyme activity assay. All extracts in a DSMO-soluble fraction at a final concentration of 166.7 ug/ml except for a few extracts at lower concentrations due to a solubility issue was used in the assay. There are 15 herb extracts that showed a strong effect of XO inhibition (> 40%). There are 19 herb extracts, which have an XO inhibition rate between 20% to 40%. The rest of 78 herb extracts had a weak effect of XO inhibition (< 20%). 5 out of 15 extracts (>40% XO inhibition) are not previously reported; 7 out of 19 extracts (20-40% OXI) are not previously reported. In addition, a dose dependent XO inhibition assay was performed for each of these 15 extracts and 6 extracts, which showed a 20-40% XO inhibitory effect at a single dose (166.7 ug/ml). Concentration range of each extract from 0 to 250, or 333 ug/ml (8 to 10 doses was studied). All 21 extracts showed a good dose-dependent XO inhibition curve. IC₅₀ is an operational parameter defined as the concentration of inhibitor required for achieving 50% inhibition of the enzyme. The smaller IC₅₀, the more potent the inhibitor is. IC₅₀ for each extract is calculated. Top ten potent XO inhibitors are Milk Thistle ( 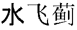 , IC₅₀ 3.3 ug/ml), Japanese Thistle extract ( 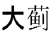 from 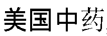 , IC₅₀ 3.8 ug/ml), Amla Fruit ( 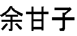 , IC₅₀ 5.6 ug/ml), Grape Seed ( 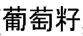 , IC₅₀ 8 ug/ml), Pomegranate (40% Ellagic Acid, 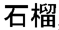 , IC₅₀ 10 ug/ml), Green Tea (50% EGCG, 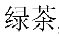 , IC₅₀ 11.5 ug/ml), Pine Bark ( 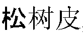 , IC₅₀ 12 ug/ml), St John's Wort ( 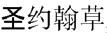 , IC₅₀ 14 ug/ml), African Mango seed ( 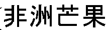 , IC₅₀ 35 ug/ml), and Butcher's Broom ( 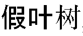 , IC₅₀ 82 ug/ml). Furthermore, a relatively small concentration was selected of each of 8 herbal extracts and folic acid for a combination assay. Folic acid (0.167 uM) and extract at a defined concentration (ug/ml) were tested. Three out of 8 extracts (Grape Seed, Milk Thistle and Japanese Thistle extract) showed a synergistic XO inhibitory effect with folic acid; 3 out of 8 extracts (Amla Fruit, Green Tea and Pine Bark) showed an additive XO inhibitory effect with folic acid.

The inventors designed a dietary health supplement recipe, which contains five herbal extracts (Japanese thistle extract, Grape seeds extract, Amla extract, Pine bark extract, Chinese mint extract) and folic acid at a clinically relevant dose for animal testing. Allopurinol was used a positive control. Each mouse was I.P. injected with allatoxanamide, and then the mice were then oral gavaged with allopurinol or extract recipe, respectively. Blood was taken from facial vein at 1.5 hours and 3 hours after the treatment. Serum uric acid level was measured. Uricase inhibitor Allantoxanmide treatment (I.P. injection) significantly increased serum uric acid levels at 1.5 hours and 3 hours. Allopurinol at a clinically relevant dose significantly reduced serum uric acid levels in Allantoxanmide-treated mice at both 1.5 hours and 3 hours. Although clinically relevant dose of dietary health supplement recipe was less potent than Allopurinol, it also significantly reduced serum uric acid levels in Allantoxanmide-treated mice at both 1.5 hours and 3 hours. This effect showed a dose-dependent manner.

### 1. Data of 112 standard herb medicine extracts

Standard herbal extracts (111) were obtained from Bulk Supplements.com and 1 herbal extract obtained from Trustworthy Herbs Inc ( 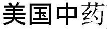 ). Bulk Supplements Inc is located in Henderson, Nevada. Per the company's website, 1) it is a FDA-registered cGMP manufacturing and distribution facility; 2) the company is dedicated to maintaining all health code and government regulations, and 3) each supplement is tested in their in-house laboratory before distribution, ensuring that all products are safe for consumption. The standard herbal extracts (100 g) were in powder form and were packaged in a seal in the alumina bag. Daily serving dose of each extract is provided on the bag.

All extracts were in a DSMO-soluble fraction at a final concentration of 166.7 ug/ml except for a few extracts at lower concentrations because of solubility issues; these were mixed with 20 nM XO. The initial rate of uric acid formation in the reaction system was recorded; and XO inhibition rate of each herb extract was calculated. All data are shown in Table 9 and FIG. 11 (and the numbering on the x-axis in FIG. 11 corresponds to the order of the list of compounds in Table 9). There are 15 herb extracts that showed a strong effect of XO inhibition (> 40%). There are 19 herb extracts, which have an XO inhibition rate between 20% to 40%. The rest of 78 herb extracts had a weak effect of XO inhibition (< 20%).

The inventors performed a preliminary literature search (herb name, xanthine oxidase, English and Chinese) in both the Medline and Google scholar search engines, and it was found that:
- 5 out of 15 extracts (>40% XO inhibition) are not previously reported: Butcher's Broom Extract ( 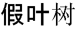 , 64% XOI), Oolong Tea ( 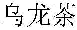 , 60% XOI), Senna Leaf ( 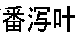 , 45% XOI), Yohimbe bark( 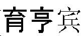 , 62% XOI) and Japanese Thistle extract ( 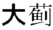 from 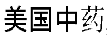 , 80% XOI at 33.3 ug/ml). XO inhibitory effects of other 10 extracts are previously reported: African Mango seed ( 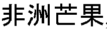 , 78% XOI) (1,2), Amla Fruit ( 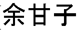 , 100% XOI) (5), Grape Seed ( 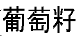 , 78% XOI) (7,8,9), Green Tea (50% EGCG, 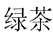 , 77% XOI (10,11,12), Milk Thistle ( 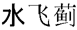 , 87% XOI at 33.3 ug/ml) (19), Olive Leaf ( 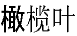 , 65%) (21,22), Pine Bark ( 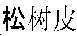 , 87% XOI) (25), Pomegranate (40% Ellagic Acid, 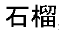 , 90% XOI) (26,27,28), Spearmint ( 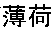 ,63.6%) (15, 32) and St John's Wort ( 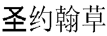 ,87% XOI) (33).
- 7 out of 19 extracts (20-40% OXI) are not previously reported: Butterbur ( 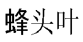 or 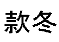 , 20% XOI), Caralluma bark ( 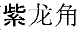 , 30% XOI), Echinacea root ( 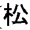 5R M, 21% XOI), Horse Chestnut fruit ( 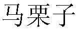 , 23.5% XOI), Kola Nut ( 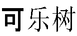 , 20% XOI), Magnolia Bark ( 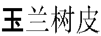 , 22% XOI) and Muira Puama Bark ( 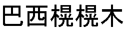 , 31.6% XOI).

112 items (111 from Bulk Supplements.com and 1 from Trustworthy Herbs Inc 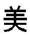 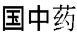 ): single dose (166.7 ug/ml, DMSO-fraction):
15 extracts: >40% XO inhibition (5 out of 15 are not previously reported per our preliminary keyword search)
19 extracts: 20-40% XO inhibition (7 out of 19 are not previously reported per our preliminary keyword search)
78 extracts: <20% XO inhibition

**Table 9.**

| XO inhibitory effects of 112 herbal extracts (111 from Bulk Supplements.com and 1 from Trustworthy Herbs Inc ( 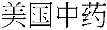 ) | | | | |
|---|---|---|---|---|
| **Herb extract and Description** | **Code** | **Chinese** | **XOI%** (166.7 ug/ml) | **Previous** report |
| Acai Berry Extract 4:1 Powder | ACAI100 | 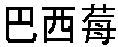 | 0 | |
| #1, African Mango seed Extract Powder | AFMAN100 | 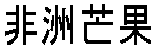 | 78 | Ref 1,2 |
| Ajuga Turkest Extract Powder | AJTU100 | 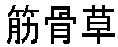 | 16 | |
| Alfalfa Extract Powder | ALFA100 | 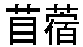 | 0 | |
| Aloe Vera Leaf Extract Powder | ALOE100 | 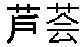 | 27 | Ref 3,4 |
| American Ginseng Extract Powder | AMGINEX100 | 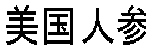 | 0 | |
| #2, Amla Fruit Powder | AMLA100 | 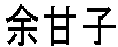 | 100 | Ref 5. |
| Andrographis Extract Powder | ANPA100 | 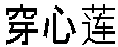 | 14 | |
| Aronia Extract Powder | ARON100 | 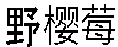 | 0 | |
| Artichoke Extract Powder | ART100 | 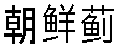 | 0 | |
| Astragalus Extract Powder | ASTR100 | 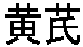 | 0 | |
| Bamboo Extract Powder | BAMB100 | 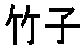 | 0 | |
| Bearberry Leaf Extract (Uva Ursi) | BEAR100 | 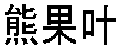 | 10 | |
| Bilberry Extract Powder | BIL100 | 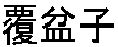 | 0 | |
| Bitter Melon Extract Powder | MELON100 | 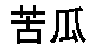 | 0 | |
| Black Cohosh Powder | BLKC100 | 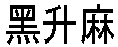 | 0 | |
| Black Garlic Extract Powder | BKGRLC250 | 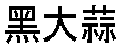 | 0 | |
| Blood Orange Extract Powde | BLDOR100 | 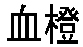 | 0 | |
| Boswellia Serrata Extract Powder | BOSW100 | 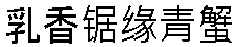 | 0 | |
| Broccoli Extract Powder | BROC100 | 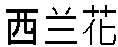 | 0 | |
| Buckthorn Bark Extract Powder | BTHRN100 | 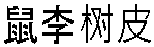 | 0 | |
| Burdock Extract Powder | BURD100 | 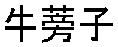 | 7 | |
| #3, Butcher's Broom Extract Powder | BROOM100 | 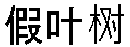 | 64 | NA |
| Butterbur Extract Powder | BTRBR25 | 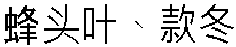 | 20 | NA |
| Caralluma bark (wild) Extract Powder | CARFIM100 | 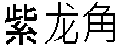 | 30 | NA |
| Cascara Sagrada Extract Powder | CASSA100 | 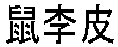 | 18 | |
| Cassia Seed Extract Powder | CASSS100 | 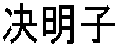 | 6 | |
| Cat's Claw Extract Powder | CTCLW100 | 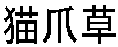 | 6 | |
| Catuaba bark Bark Extract Powder | CAT100 | 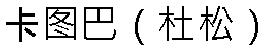 | 26 | Ref 6. |
| Cayenne Extract Powder | CAYN100 | 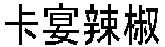 | 0 | |
| Celery Seed Extract 4:1 Powder | CLYSD100 | 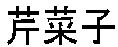 | 17 | |
| Chasteberry Extract | CBE-100 | 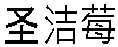 | 10 | |
| Chitosan Extract Powder | CHLOR100 | 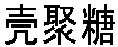 | 0 | |
| Chlorella Blue-Green Algae | CHLOR100 | 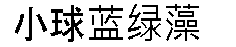 | 0 | |
| Cinnamon Bark Powder Extract | CBE100 | 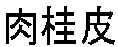 | 0 | |
| Cissus Quadrangularis Powder | CISSQ100 | 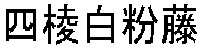 | 0 | |
| Cocoa Extract | COCOA100 | 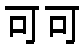 | 10 | |
| Cordyceps Powder | CORDY100 | 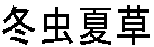 | 0 | |
| Coriolus Versicolor (Turkey Tail) Powder | COVER100 | 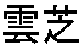 | 0 | |
| Corydalis Extract Powder | CRYDLS100 | 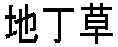 | 0 | |
| Creek Yellow Grass Powder | CYG100 | 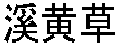 | | |
| Curcumin 95% Natural Turmeric Extract | CURC25 | 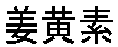 | 0 | |
| Dandelion Root Extract Powder | DAND100 | 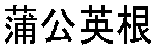 | 0 | |
| Devil's Claw Extract Powder | DEVCLW100 | 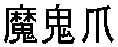 | 0 | |
| Dragon Fruit Extract Powder | DGNFRT100 | 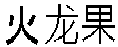 | 0 | |
| Echinacea root Extract Powder | EYEB100 | 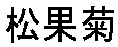 | 21 | NA |
| Eyebright Extract Powder | EYEB100 | 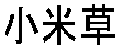 | 0 | |
| Flaxseed Extract Powder | FLXPDR100 | 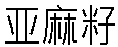 | 0 | |
| Fo-Ti Extract Powder | FOTI100 | 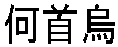 | 8 | |
| Garlic Extract | GARLIC100 | 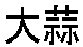 | 0 | |
| Ginkgo Biloba Leaf Extract | GINK100 | 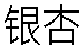 | 10 | |
| Ginseng Root Extract Powder | GINEX50 | 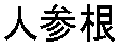 | 0 | |
| #4, Grape Seed Extract | GSE100 | 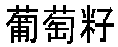 | 78 | Ref 7,8,9 |
| Grapefruit Seed Extract - 100g Pure Powder | GFS100 | 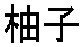 | 0 | |
| #5, Green Tea Pure Extract 50% EGCG | GTEGCG100 | 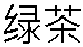 | 77 | Ref 10,11,12 |
| Guanabana Extract Powder | GNABNA100 | 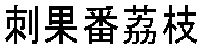 | 0 | |
| Gymnema leaf Extract Powder | GYMN100 | 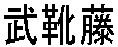 | 13 | |
| Gynostemma Extract Powder | GYST100 | 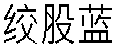 | 27 | Ref 13,14 |
| Hawthorn Berry Extract Powder | HWTHNB100 | 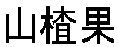 | 0 | |
| Hawthorn Leaf Extract Powder | HWTHNLF100 | 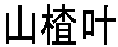 | 25 | Ref 15 |
| Hibiscus Flower Extract Powder | HIBSC100 | 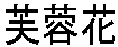 | 18 | |
| Hoodia Extract | HOOD 100 | 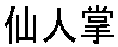 | 0 | |
| Hops Extract Powder | HOP100 | 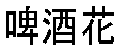 | 13 | |
| Horse Chestnut fruit Extract Powder | HORCHE100 | 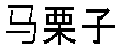 | 23.5 | NA |
| Horsetail Extract Powder | HORS100 | 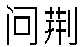 | 0 | |
| Kava Kava Extract Powder | KVA100 | 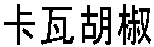 | 12 | |
| Kelp Extract Powder | KLP100 | 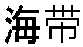 | 0 | |
| Kola Nut Extract Powder | KOLA100 | 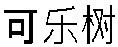 | 20 | NA |
| Kudzu Root Extract Powder | KDZ100 | 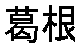 | 30.3 | Ref 15,16 |
| Lemon Balm Extract Powder | LMBLM100 | 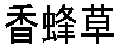 | 33 | Ref 17 |
| Licorice Root Extract Powder | LCRT100 | 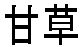 | 26 | Ref 18 |
| LongJack Extract | LJ25 | 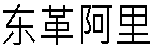 | 0 | |
| Maca Root Extract | MACA100 | 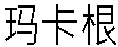 | 9 | |
| Magnolia Bark Extract Powder | MAGNO100 | 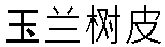 | 22 | NA |
| Marshmallow Root Extract Powder | MARSH-100 | 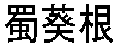 | 6 | |
| #6, Milk Thistle Extract Powder | MTE100 | 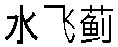 | 87% (33.3 ug/ml) | Ref 19 |
| Moringa Extract Powder | MORLE100 | 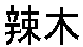 | 20 | Ref 20 |
| Motherwort Extract Powder | MOTH100 | 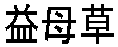 | 0 | |
| Mucuna Pruriens Extract Powder | MUPR100 | 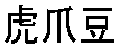 | 10 | |
| Muira Puama Bark Extract Powder | MUPU100 | 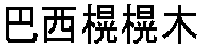 | 31.6 | NA |
| Nettle Extract Powder- | NTLLF100 | 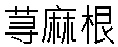 | 0 | |
| Oat Straw Extract Powder | OATSTW100 | 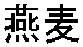 | 0 | |
| #7, Olive Leaf Extract Powder | OLE100 | 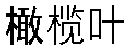 | 65 | Ref 21,22 |
| #8, Oolong Tea (leaf) Extract Powder | OOLT100 | 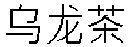 | 60 | NA |
| Spirulina (Organic) Powder | SPIORG100 | 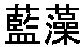 | 0 | |
| Papaya Fruit Extract Powder | PAPFRUIT100 | 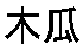 | 0-25 | Ref 23,24 |
| Papaya Seed Powder | PAPSEED100 | 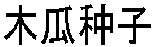 | 37.5 | Ref 23 |
| Parsley Extract Powder | PARS 100 | 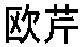 | 0 | |
| Passion Flower Extract Powder | PASSFLO100 | 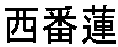 | 0 | |
| #9, Pine Bark Extract | PBE100 | 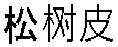 | 87 | Ref 25 |
| #10, Pomegranate Extract (40% Ellagic Acid) | POM40100 | 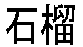 | 90 | Ref 26,27,28 |
| Portulaca Oleracea (Purslane) Extract | POR100 | 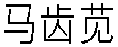 | 0 | |
| Pumpkin Seed Extract Powder | PUMP100 | 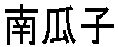 | 11 | |
| Red Clover (steam-leaf) Extract Powder | RCE100 | 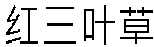 | 24.5 | Ref 29 |
| Red Yeast Rice Extract (RYR) | RYR100 | 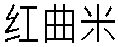 | 0 | |
| Reishi Mushroom Powder | REI100 | 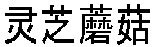 | 12 | |
| Rosemary Extract Powder | RSMRY100 | 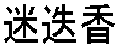 | 39 | Ref 30,31 |
| Sage Extract Powder | SAGE100 | 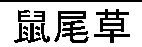 | 11 | |
| Sarsaparilla Root Powder | SARS100 | 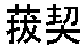 | 13 | |
| #11, Senna Leaf Extract | SENN250 | 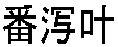 | 45 | NA |
| Slippery Elm Bark Extract Powder | SLPEM100 | 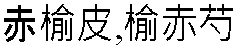 | 0 | |
| #12, Spearmint Leaf Extract Powder | SPMNT100 | 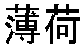 | 63.6 | Ref 15, 32 |
| Spinach Extract Powder | SPIN100 | 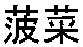 | 0 | |
| #13, St John's Wort Extract | SJW10 | 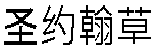 | 87 | Ref 33 |
| Taxillus Chinensis Danser Extract Powder | TAXCHI100 | 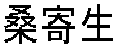 | 15 | |
| Turkey Rhubarb Extract Powder | RHU100 | 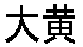 | 0 | |
| Valerian Root Extract | VRE-100 | 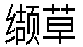 | 0 | |
| White Mulberry Fruit Extract Powder | WHML100 | 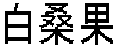 | 0 | |
| White Willow Bark Extract | WWB100 | 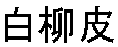 | 0 | |
| Wild Yam Extract Powder | YAM100 | 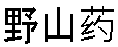 | 0 | |
| #14, Yohimbe bark Extract Powder | YMB500 | 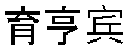 | 62 | NA |
| #15, 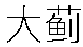 Extract 5:1 100g \| Da Ji Extract 5:1 I Japanese Thistle Extract 5:1 ( From 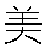 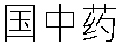 ) | EXD003 | | 80% at 33.3 ug/ml | NA |

| | | | | |
|---|---|---|---|---|
| NA means: the extract was not previously reported for XO inhibition Blank means: the extract has no XO inhibition as tested, and no literature search was performed | | | | |

### 2. Data of dose-dependent of 21 standard herb medicine extracts

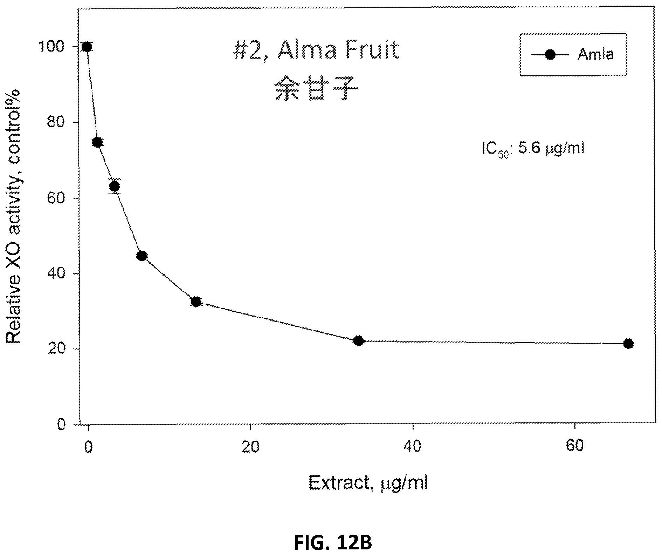

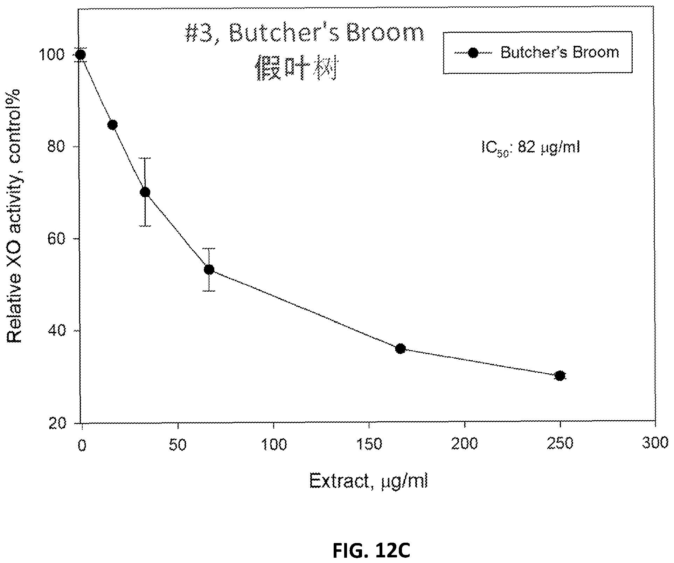

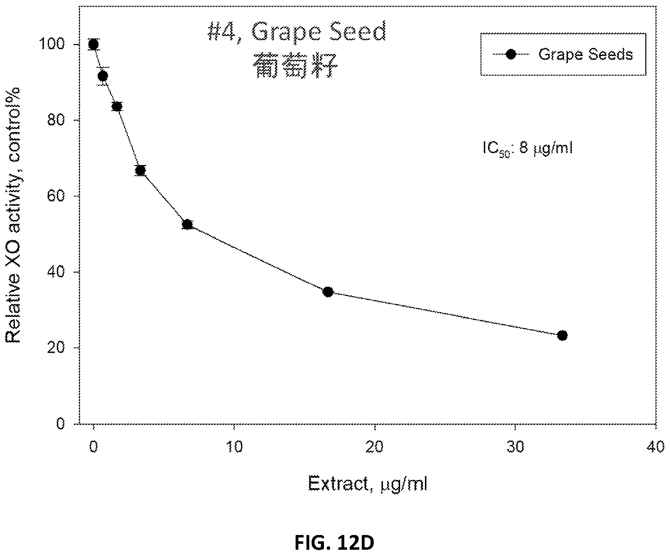

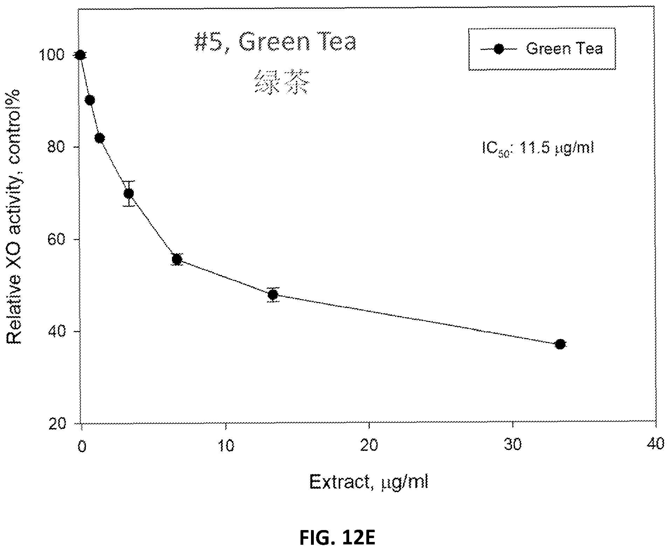

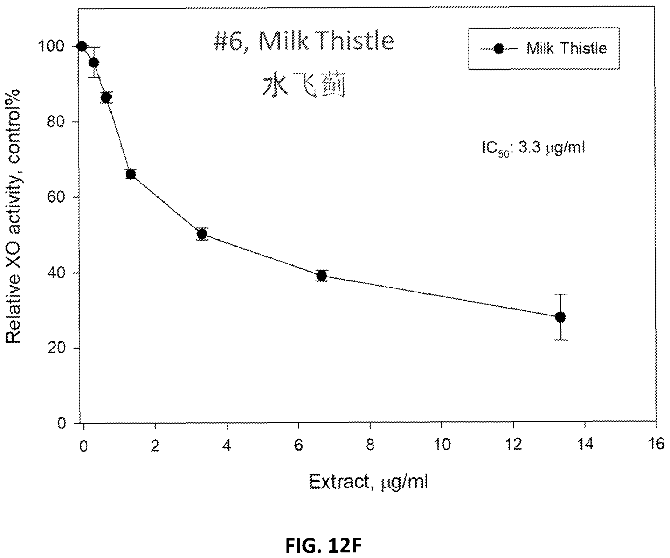

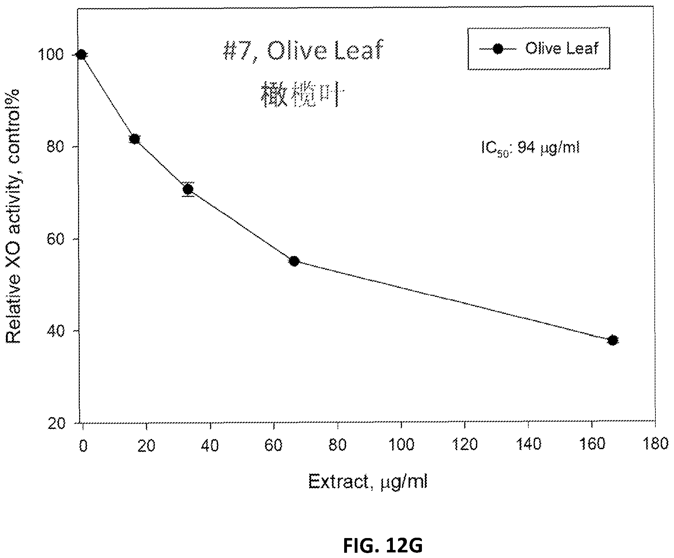

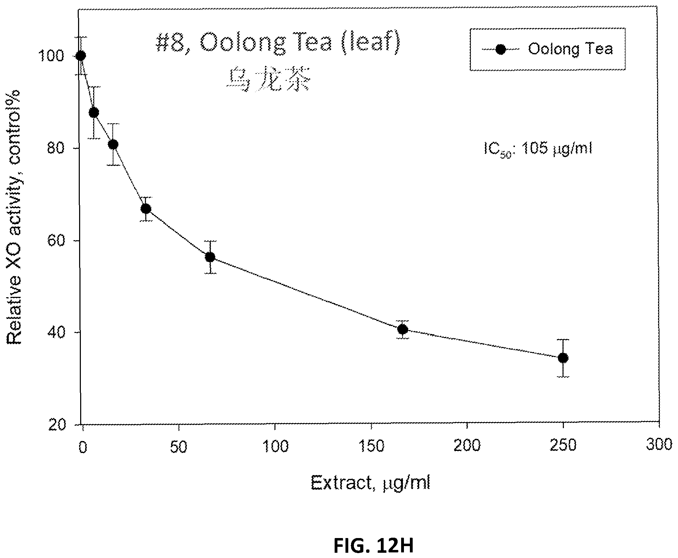

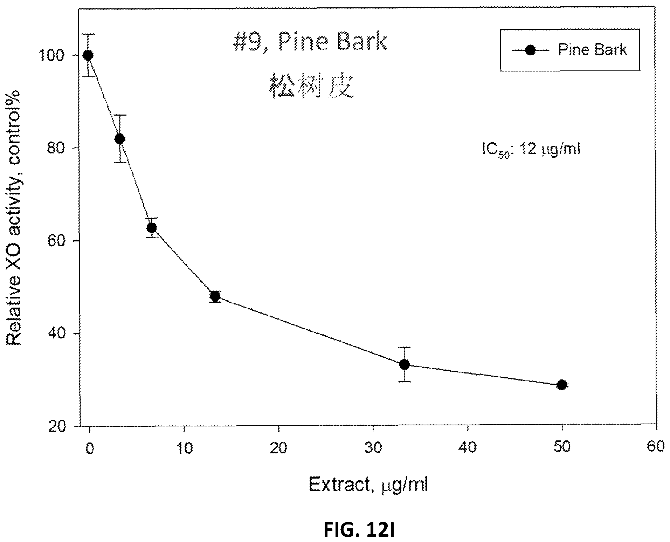

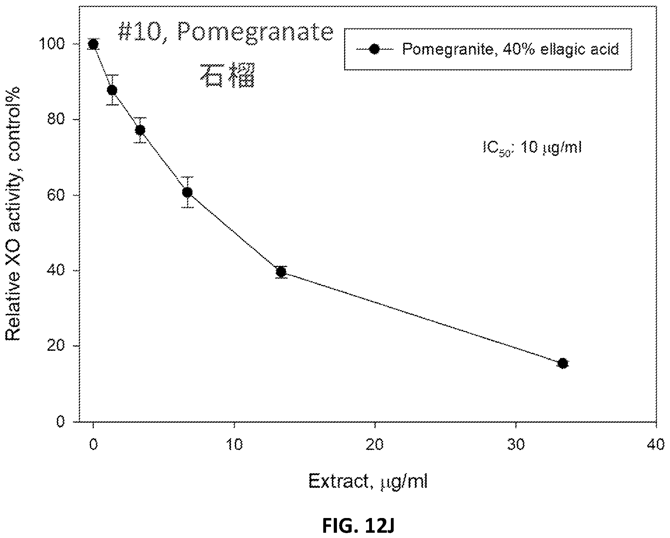

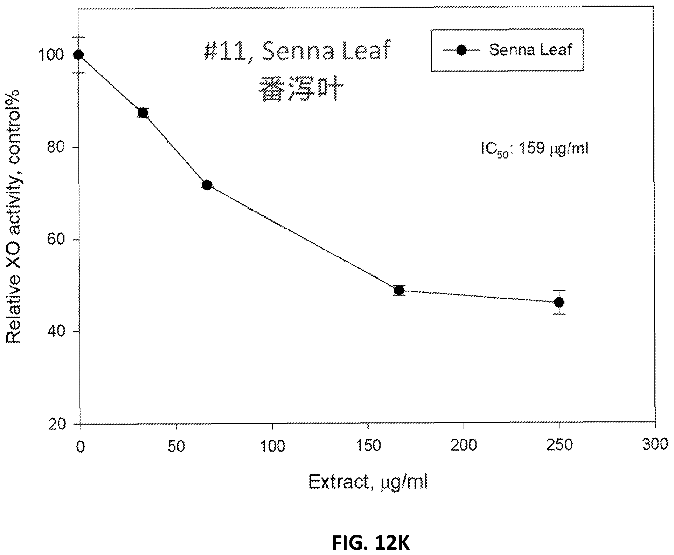

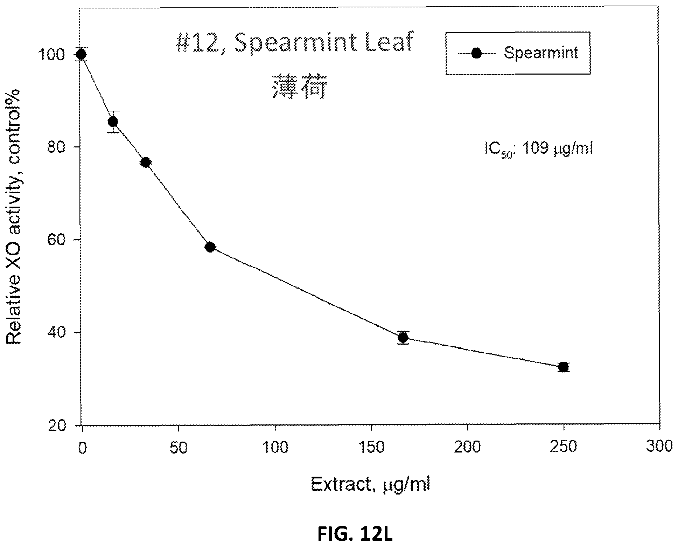

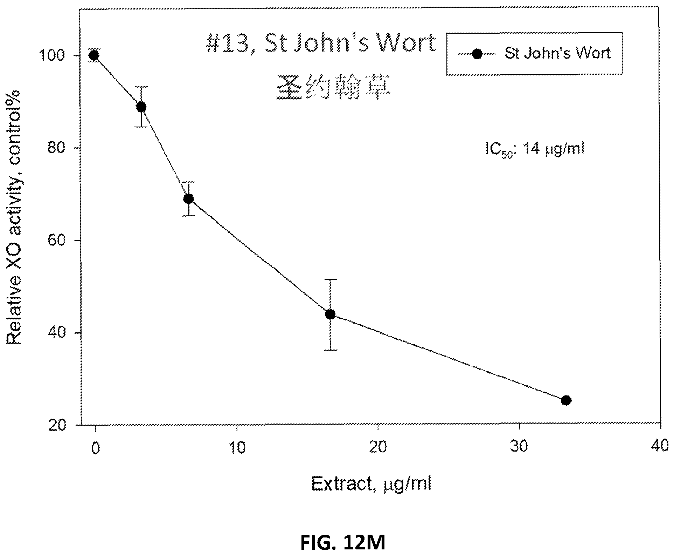

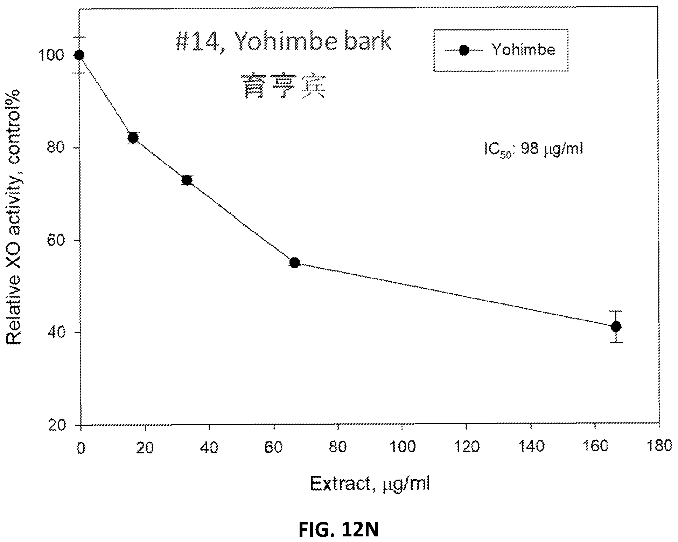

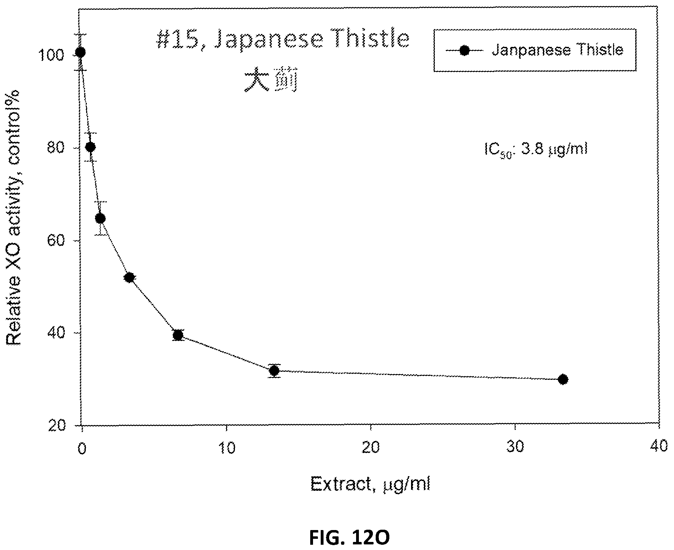

From initial screen of XO inhibitory effects of 112 herbal extracts with single dose (166.7 ug/ml), 15 extracts had more than 40% inhibition of XO activity. Furthermore, a dose dependent XO inhibition assay was performed for each of these 15 extracts. In addition, the inventors also tested a dose-dependent study for 6 extracts, which showed a 20-40% XO inhibitory effect at a single dose (166.7 ug/ml). Concentration range of each extract from 0 to 250, or 333 ug/ml (8 to 10 doses was studied). All 21 extracts showed a nice dose-dependent XO inhibition curve. IC₅₀ is an operational parameter defined as the concentration of inhibitor required for achieving 50% inhibition of the enzyme. The smaller IC₅₀, the more potent the inhibitor is. IC₅₀ for each extract is calculated (Table 10, FIG. 12). Top ten potent XO inhibitors are Milk Thistle ( 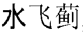 , IC₅₀ 3.3 ug/ml), Japanese Thistle extract ( 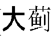 from 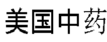 , IC50 3.8 ug/ml), Amla Fruit ( 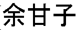 , IC₅₀ 5.6 ug/ml), Grape Seed ( 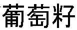 , IC₅₀ 8 ug/ml), Pomegranate (40% Ellagic Acid, 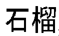 , IC₅₀ 10 ug/ml), Green Tea (50% EGCG, 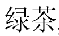 , IC₅₀ 11.5 ug/ml), Pine Bark ( 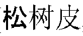 , IC₅₀ 12 ug/ml), St John's Wort ( 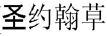 , IC₅₀ 14 ug/ml), African Mango seed ( 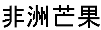 , IC₅₀ 35 ug/ml), and Butcher's Broom ( 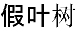 , IC₅₀ 82 ug/ml). These data is useful for understanding the potency of these extracts and provides rational design for combination assay of each extract with folic acid and 5-MTHF or multiple extracts with folic acid and 5-MTHF.

**Table 10. IC₅₀ (XO inhibitory effect) of 21 herbal extracts**

| **Herb extract and Description** | **Code** | **Chinese** | **XOI% (166.7 ug/ml)** | **IC₅₀ (ug/ml)** | **Previous report** |
|---|---|---|---|---|---|
| #1, African Mango seed Extract Powder | AFMAN100 | 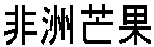 | 78 | 35 | Ref 1,2 |
| #2, Amla Fruit Powder | AMLA100 | 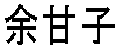 | 100 | 5.6 | Ref 5 |
| #3, Butcher's Broom Extract Powder | BROOM100 | 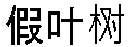 | 64 | 82 | NA |
| #4, Grape Seed Extract | GSE100 | 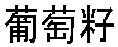 | 78 | 8 | Ref 7,8,9 |
| #5, Green Tea Pure Extract 50% EGCG | GTEGCG100 | 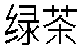 | 77 | 11.5 | Ref 10,11,12 |
| #6, Milk Thistle Extract Powder | MTE100 | 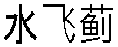 | 87% (33.3 ug/ml) | 3.3 | Ref 19 |
| #7, Olive Leaf Extract Powder | OLE100 | 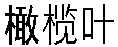 | 65 | 94 | Ref 21,22 |
| #8, Oolong Tea (leaf) Extract Powder | OOLT100 | 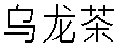 | 60 | 105 | NA |
| #9, Pine Bark Extract | PBE100 | 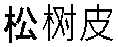 | 87 | 12 | Ref 25 |
| #10, Pomegranate Extract (40% Ellagic Acid) | POM40100 | 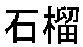 | 90 | 10 | Ref 26,27,28 |
| #11, Senna Leaf Extract | SENN250 | 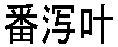 | 45 | 159 | NA |
| #12, Spearmint Leaf Extract Powder | SPMNT100 | 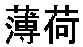 | 63.6 | 109 | Ref 15, 32 |
| #13, St John's Wort Extract | SJW10 | 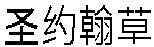 | 87 | 14 | Ref 33 |
| #14, Yohimbe bark Extract Powder | YMB500 | 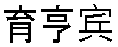 | 62 | 98 | NA |
| #15, 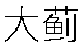 Extract 5:1100g \| Da Ji Extract 5:1 \| Japanese Thistle Extract 5:1 ( From 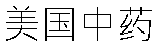 ) | EXD003 | 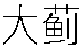 | 80% at 33.3 ug/ml | 3.8 | NA |
| Caralluma bark (wild) Extract Powder | CARFIM100 | 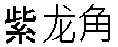 | 30 | >170 | NA |
| Kudzu Root Extract Powder | KDZ100 | 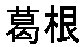 | 30.3 | >170 | Ref 15,16 |
| Lemon Balm Extract Powder | LMBLM100 | 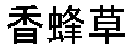 | 33 | >250 | Ref 17 |
| Muira Puama Bark Extract Powder | MUPU100 | 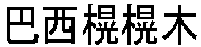 | 31.6 | >250 | NA |
| Papaya Seed Powder | PAPSEED100 | 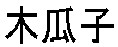 | 37.5 | 200 | Ref 23 |
| Rosemary Extract Powder | RSMRY100 | 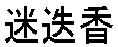 | 39 | 280 | Ref 30,31 |

| | | | | | |
|---|---|---|---|---|---|
| NA means: the extract was not previously reported for XO inhibition | | | | | |

### 3. Data of combination of single herb medicine extract with folic acid

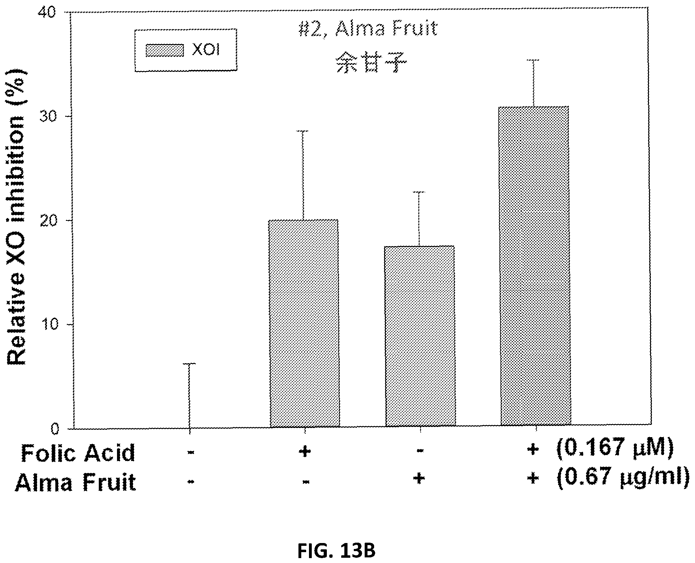

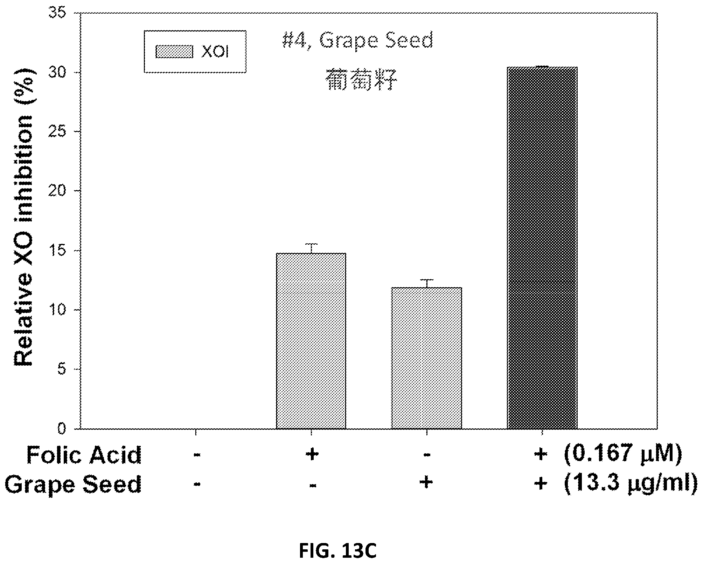

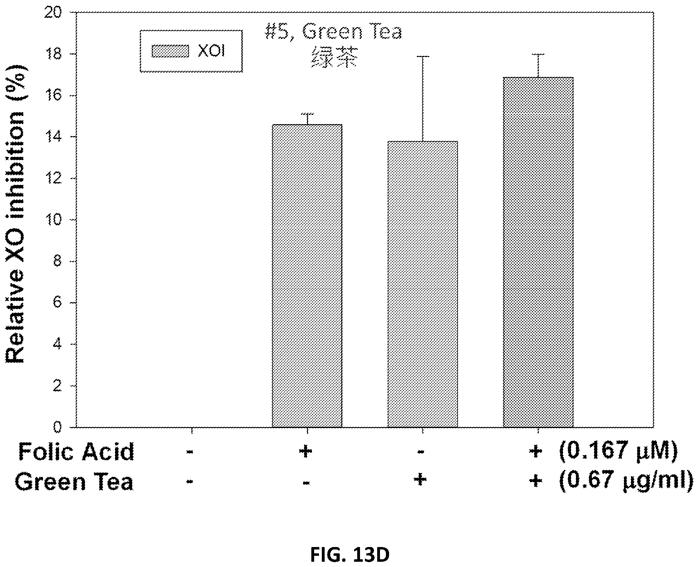

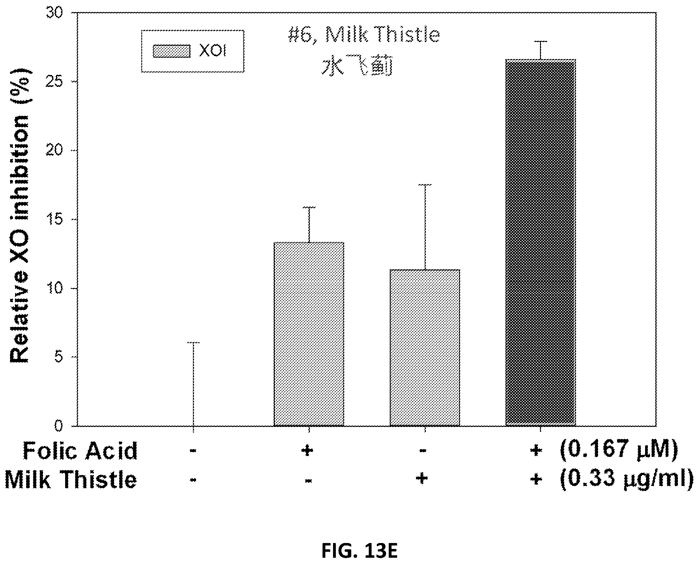

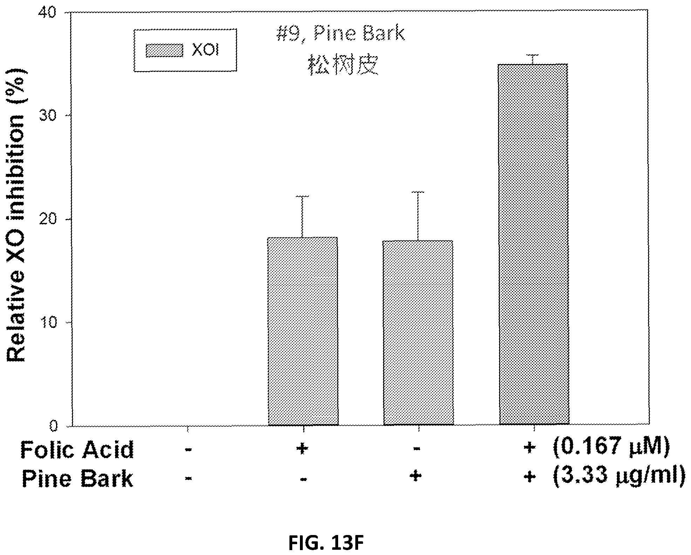

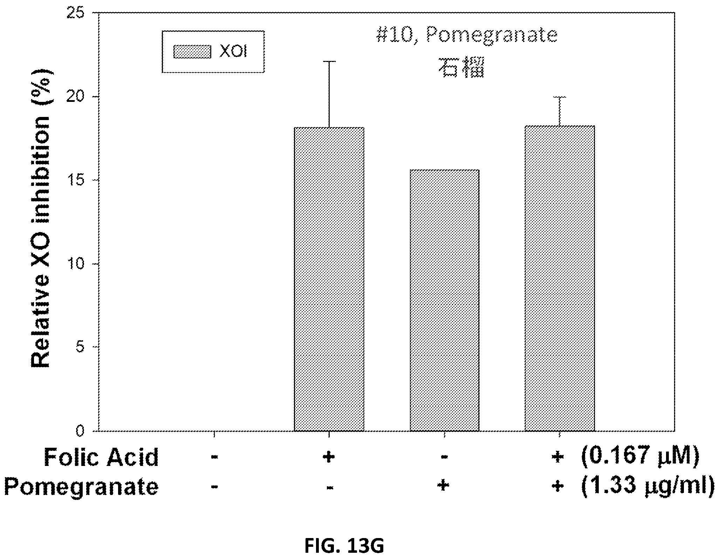

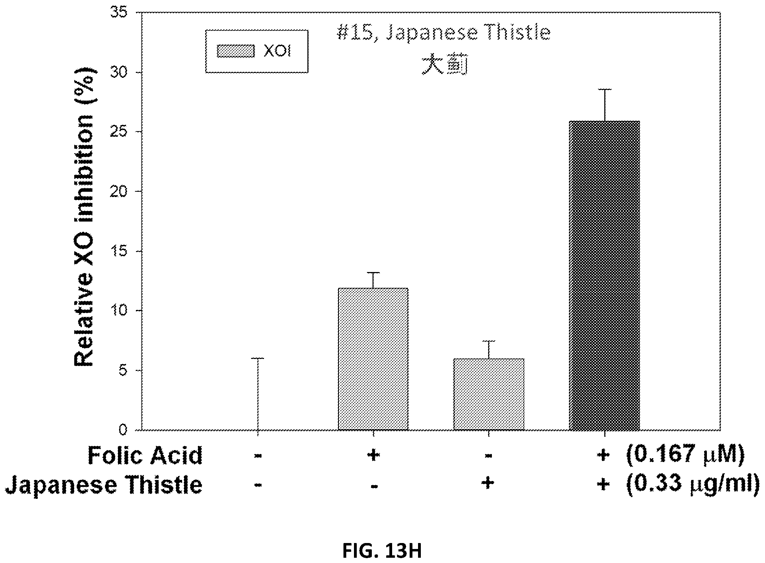

Based on the result of dose-dependent XO inhibition experiments for herb extracts and folic acid, a relatively small concentration of each of 8 herbal extracts and folic acid were selected for a combination assay. One of the important advantages of such combination supplements is enhancement of XO inhibitory function of the supplement; while reducing the dose of each component, thereby reducing potential side effects of the supplement for long-term use. Folic acid (0.167 uM) and extract at a defined concentration (ug/ml) were separately added into the XO reaction system without pre-mixing. XO activity was measured (FIG. 13). Three out of 8 extracts (Grape Seed, Milk Thistle and Japanese Thistle extract) showed a synergistic XO inhibitory effect with folic acid; 3 out of 8 extracts (Amla Fruit, Green Tea and Pine Bark) showed an additive XO inhibitory effect with folic acid; and 2 out of 8 extracts (African Mango and Pomegranate) did not shown any additive or synergistic XO inhibitory effect with folic acid.

### 4. Data of the hypouricemic effect of one dietary health supplement recipe in allantoxanamide-treated mice

**Design dietary health supplement recipe (combination).** One of the important advantages of combination of several herbal extracts and folic acid is enhancement of XO inhibitory function of the supplement; while reducing the dose of each component, thereby reducing potential side effects of the supplement for long-term use. Selected herbal extracts should have a strong XO inhibitory effect *in vitro,* and show an additive or synergistic effect with folic acid. Herbal extracts, which are not reported for their XO inhibitory function, are utilized, in specific embodiments. Based on this rationale, the inventors have designed one exemplary recipe, which contains five herbal extracts and folic acid (Table 11).

**Table 11. Design of an example of a dietary health supplement recipe (combination)**

| **Component** | **IC₅₀** | **XOI Daily Dose** | **+ Folic Acid** | **Report XOI** |
|---|---|---|---|---|
| Japanese Thistle Extract ( 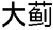 ) | 3.8 ug/ml | 250 mg (4.2 mg/kg) | Synergistic | No |
| Grape Seed Extract ( 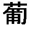 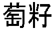 ) | 8 ug/ml | 200 mg (3.3 mg/kg) | Synergistic | Yes |
| Amla Fruit Extract ( 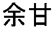 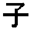 ) | 5.6 ug/ml | 800 mg (13.3 mg/kg) | additive | Yes |
| Pine Bark Extract ( 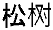 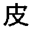 ) | 12 ug/ml | 250 mg (4.2 mg/kg) | additive | Yes |
| Chinese Mint Extract ( 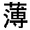 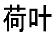 ) | 34 ug/ml | 1000 mg (16.7 mg/kg) | additive | No |
| Folic acid | 0.75 uM | 800 ug (13.3 ug/kg) | | Yes |

| | | **Mouse dose** | | |
|---|---|---|---|---|
| | | Low dose: 41.7 mg/kg | | |
| | | High dose: 83.4 mg/kg | | |

Mouse experiment design, method and results. The extract combination samples were prepared by weighing 4.2 mg Japanese thistle extract, 3.3 mg Grape seeds extract, 13.3 mg Amla extract, 4.2 mg Pine bark extract, and 16.7 mg Chinese mint extract; mixing them with 10 ml 1% PEG400, and making a stock solution of 41.7 mg/10 ml. Folic acid solution was prepared separately by dissolving 2.6 mg folic acid in 1 ml 0.1m potassium phosphate buffer, then 6 ul of this FA solution was added to the 10 ml extract solution. As a positive control, Allopurinol, a clinical XO-inhibitor drug, was used at 13.3 mg/kg for the mouse experiment. Human dose of Allopurinol is about 800 mg daily.

Adult C57BL/6 mice (about 20 g body weight) were used. Four groups of mice (n=4 per group) were assigned for different treatments or controls (Table 12). Intraperitoneal (i.p.) injection of uricase inhibitor allantoxanamide can effectively block the conversion of uric acid to 5-hydroxyisourate and thus cause a marked increase in serum uric acid levels in mice, providing a hyperuricemic animal model (34). Each mouse was ip injected with allatoxanamide at a dose of 200 mg/kg, the mice were then oral gavaged with 200 ul to 250 ul (for 20 to 25 g mice) allopurinol or extract solution, respectively. Blood was taken from facial vein at 1.5 hours and 3 hours after the treatment. Serum uric acid level was measured with a phosphotunstate method. Uricase inhibitor Allantoxanmide treatment (I.P. injection) significantly increased serum uric acid levels at 1.5 hours and 3 hours; these data are consistent with those in a previous publication (34). As a positive control, Allopurinol at a clinically relevant dose significantly reduced serum uric acid levels in Allantoxanmide-treated mice at both 1.5 hours and 3 hours. Although clinically relevant dose of dietary health supplement recipe was less potent than Allopurinol, it also significantly reduced serum uric acid levels in Allantoxanmide-treated mice at both 1.5 hours and 3 hours. This effect showed a dose-dependent manner (FIG. 14).

**Table 12. Mouse assignments for studying the hypouricemic effect of dietary health supplement**

| **Group (n=4)** | **Allantoxanamide** (200 mg/kg, IP) | **Treatment** | **End Point** |
|---|---|---|---|
| 1 | - | - | Serum uric acid (1.5 and 3 hours) |
| 2 | + | Allopurinol (13.3 mg/kg) | Serum uric acid (1.5 and 3 hours) |
| 3 | + | Recipe low dose (41.7 mg/kg) | Serum uric acid (1.5 and 3 hours) |
| 4 | + | Recipe high dose (83.4 mg/kg) | Serum uric acid (1.5 and 3 hours) |

### References for Example 4

1. 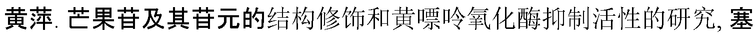 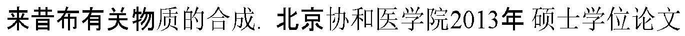
2. 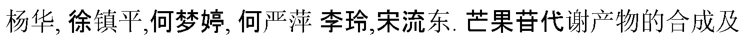 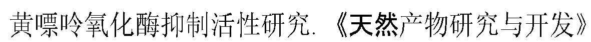 2015 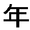 8 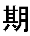
3. 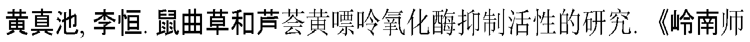 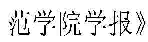 2017 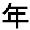 06 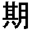
4. Taukoorah U, Mahomoodally MF. Crude Aloe vera Gel Shows Antioxidant Propensities and Inhibits Pancreatic Lipase and Glucose Movement In Vitro. Adv Pharmacol Sci. 2016;2016:3720850. doi: 10.1155/2016/3720850. Epub 2016 Jan 3.
5. Sarvaiya VN, Sadariya KA, Pancha PG, Thaker AM, Patel AC, Prajapati AS. Evaluation of antigout activity of Phyllanthus emblica fruit extracts on potassium oxonate-induced gout rat model. Vet World. 2015 Oct;8(10): 1230-6. doi: 10.14202/vetworld.2015.1230-1236. Epub 2015 Oct 23.
6. Bernardo J, Ferreres F, Gil-Izquierdo Á, Videira RA, Valentão P, Veiga F, Andrade PB. In vitro multimodal-effect of Trichilia catigua A. Juss. (Meliaceae) bark aqueous extract in CNS targets. J Ethnopharmacol. 2018 Jan 30;211:247-255. doi: 10.1016/j.jep.2017.09.039. Epub 2017 Sep 29.
7. Belviranli M, Gökbel H, Okudan N, Büyükba 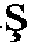 S. Effects of grape seed extract on oxidative stress and antioxidant defense markers in streptozotocin-induced diabetic rats. Turk J Med Sci. 2015;45(3):489-95.
8. Wang Y, Zhu JX, Kong LD, Yang C, Cheng CH, Zhang X. Administration of procyanidins from grape seeds reduces serum uric acid levels and decreases hepatic xanthine dehydrogenase/oxidase activities in oxonate-treated mice. Basic Clin Pharmacol Toxicol. 2004 May;94(5):232-7.
9. 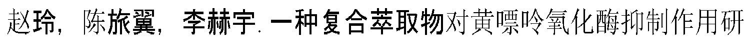 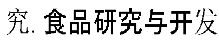 , 2 0 15 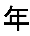 9 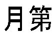 36 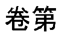 17 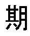 ; 198-200, DOI : 10.3969/j.issn.1005-6521.2015.17.049
10. Zhu C, Xu Y, Liu ZH, Wan XC, Li DX, Tai LL. The anti-hyperuricemic effect of epigallocatechin-3-gallate (EGCG) on hyperuricemic mice. Biomed Pharmacother. 2018 Jan;97:168-173. doi: 10.1016/j.biopha.2017.10.013. Epub 2017 Nov 6.
11. Zhu C, Tai LL, Wan XC, Li DX, Zhao YQ, Xu Y. Comparative effects of green and black tea extracts on lowering serum uric acid in hyperuricemic mice. Pharm Biol. 2017 Dec;55(1):2123-2128. doi: 10.1080/13880209.2017.1377736.
12. Chen G, Tan ML, Li KK, Leung PC, Ko CH. Green tea polyphenols decreases uric acid level through xanthine oxidase and renal urate transporters in hyperuricemic mice. J Ethnopharmacol. 2015 Dec 4;175:14-20. doi: 10.1016/j.jep.2015.08.043. Epub 2015 Sep 3.
13. Pang M, Fang Y, Chen S, Zhu X, Shan C, Su J, Yu J, Li B, Yang Y, Chen B, Liang K, Hu H, Lv G. Gypenosides Inhibits Xanthine Oxidoreductase and Ameliorates Urate Excretion in Hyperuricemic Rats Induced by High Cholesterol and High Fat Food (Lipid Emulsion). Med Sci Monit. 2017 Mar 4;23:1129-1140.
14. 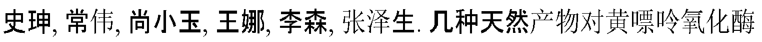 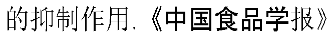 2014 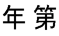 7 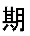
15. 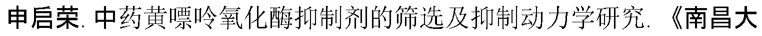 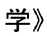 2015 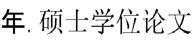
16. 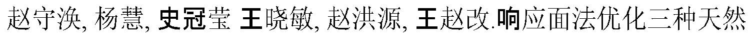 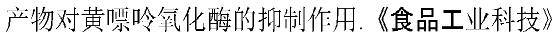 2018 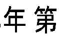 5 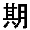 230-234 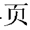
17. 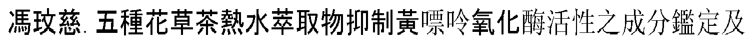 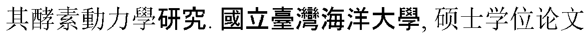 , 2017-06-29
18. Hatano T, Yasuhara T, Fukuda T, Noro T, Okuda T. Phenolic constituents of licorice. II. Structures of licopyranocoumarin, licoarylcoumarin and glisoflavone, and inhibitory effects of licorice phenolics on xanthine oxidase. Chem Pharm Bull (Tokyo). 1989 Nov;37(11):3005-9.
19. Zarrelli A, Romanucci V, Tuccillo C, Federico A, Loguercio C, Gravante R, Di Fabio G. New silibinin glyco-conjugates: synthesis and evaluation of antioxidant properties. Bioorg Med Chem Lett. 2014 Nov 15;24(22):5147-9. doi: 10.1016/j.bmcl.2014.10.023. Epub 2014 Oct 14.
20, 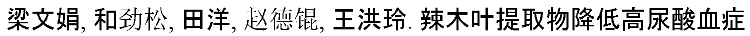 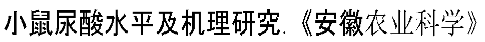 2017 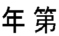 17 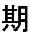 108-109 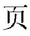
21. De Marino S, Festa C, Zollo F, Nini A, Antenucci L, Raimo G, Iorizzi M. Antioxidant activity and chemical components as potential anticancer agents in the olive leaf (Olea europaea L. cv Leccino.) decoction. Anticancer Agents Med Chem. 2014;14(10):1376-85.
22. Flemmig J, Kuchta K, Arnhold J, Rauwald HW. Olea europaea leaf (Ph.Eur.) extract as well as several of its isolated phenolics inhibit the gout-related enzyme xanthine oxidase. Phytomedicine. 2011 May 15;18(7):561-6. doi: 10.1016/j.phymed.2010.10.021. Epub 2010 Dec 8.
23. Azmi SMN, Jamal P, Amid A. Xanthine oxidase inhibitory activity from potential Malaysian medicinal plant as remedies for gout. International Food Research Journal, 2012;19(1), 159-65.
24. 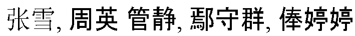 . 5 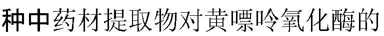 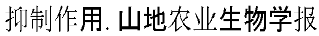 , 2015 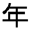 4 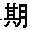 , 51-54
25. Moini H, Guo Q, Packer L. Enzyme inhibition and protein-binding action of the procyanidin-rich french maritime pine bark extract, pycnogenol: effect on xanthine oxidase. J Agric Food Chem. 2000 Nov;48(11):5630-9.
26. Les F, Prieto JM, Arbonés-Mainar JM, Valero MS, López V. Bioactive properties of commercialised pomegranate (Punica granatum) juice: antioxidant, antiproliferative and enzyme inhibiting activities. Food Funct. 2015 Jun;6(6):2049-57. doi: 10.1039/c5fo00426h.
27. Sestili P, Martinelli C, Ricci D, Fraternale D, Bucchini A, Giamperi L, Curcio R, Piccoli G, Stocchi V. Cytoprotective effect of preparations from various parts of Punica granatum L. fruits in oxidatively injured mammalian cells in comparison with their antioxidant capacity in cell free systems. Pharmacol Res. 2007 Ju1l56(1):18-26. Epub 2007 Feb 20.
28. Rummun N, Somanah J, Ramsaha S, Bahorun T, Neergheen-Bhujun VS. Bioactivity of Nonedible Parts of Punica granatum L.: A Potential Source of Functional Ingredients. Int J Food Sci. 2013;2013:602312. doi: 10.1155/2013/602312. Epub 2013 Jul 8.
29. Namuslu M, Kocaoglu H, Celik HT, Avci A, Devrim E, Gene Y, Gocmen E, Erguder IB, Durak I. Effects of aqueous soybean, mistletoe and red clover extracts on activities of adenosine deaminase and xanthine oxidase enzyme. Bratisl Lek Listy. 2014;115(6):367-71.
30. Satyal P, Jones TH, Lopez EM, McFeeters RL, Ali NA, Mansi I, Al-Kaf AG, Setzer WN. Chemotypic Characterization and Biological Activity of Rosmarinus officinalis. Foods. 2017 Mar 5;6(3). pii: E20. doi: 10.3390/foods6030020.
31. 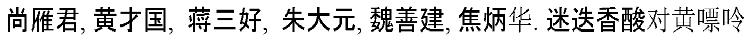 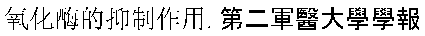 ; 27 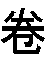 2 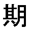 (2006 / 02 / 20), P189-191.
32. 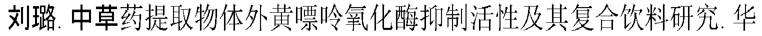 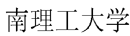 , 2014 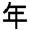 05 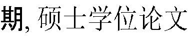
33. Havlik J, Gonzalez de la Huebra R, Hejtmankova K, Fernandez J, Simonova J, Melich M, Rada V. Xanthine oxidase inhibitory properties of Czech medicinal plants. J Ethnopharmacol. 2010 Nov 11;132(2):461-5. doi: 10.1016/j.jep.2010.08.044. Epub 2010 Aug 26.
34. Lü JM, Yao Q, Chen C. 3,4-Dihydroxy-5-nitrobenzaldehyde (DHNB) is a potent inhibitor of xanthine oxidase: a potential therapeutic agent for treatment of hyperuricemia and gout. Biochem Pharmacol. 2013 Nov 1;86(9): 1328-37.

## Claims

1. A composition comprising Japanese Thistle extract and one or more of: Grape Seed Extract; Amla Fruit Extract; Pine Bark Extract; and Chinese Mint Extract.

2. The composition of claim 1, further comprising one or more of folic acid and a functionally active derivative thereof, wherein the functionally active derivative is dihydrofolic acid, tetrahydrofolic acid, 5-methyl tetrahydrofolate (5-MTHF), or a combination thereof.

3. A composition of claim 1 or 2 for use in a method of treating or preventing a hyperuricemic condition in an individual, the method comprising the step of providing to the individual an effective amount of the composition.

4. The composition for use of claim 3, wherein the method comprises providing the composition to an individual who has received, is receiving, and/or will receive another therapy for a hyperuricemic condition, wherein the another therapy is Febuxostat, Allopurinol, or both.

## Patentansprüche

1. Zusammensetzung, umfassend japanischen Distel-Extrakt und einen oder mehrere von: Traubenkern-Extrakt; Amla-Frucht-Extrakt; Kiefernrinden-Extrakt; und chinesischem Minze-Extrakt.

2. Zusammensetzung nach Anspruch 1, die ferner eine oder mehrere von Folsäure und einem funktionell aktiven Derivat davon umfasst, wobei das funktionell aktive Derivat Dihydrofolsäure, Tetrahydrofolsäure, 5-Methyltetrahydrofolat (5-MTHF) oder eine Kombination davon ist.

3. Zusammensetzung nach Anspruch 1 oder 2 für die Verwendung in einem Verfahren zum Behandeln oder Vorbeugen eines hyperurikämischen Zustands bei einem Individuum, wobei das Verfahren den Schritt des Bereitstellens einer wirksamen Menge der Zusammensetzung an das Individuum umfasst.

4. Zusammensetzung für die Verwendung nach Anspruch 3, wobei das Verfahren das Bereitstellen der Zusammensetzung an ein Individuum umfasst, das eine andere Therapie für einen hyperurikämischen Zustand erhalten hat, erhält und/oder erhalten wird, wobei die andere Therapie Febuxostat, Allopurinol oder beides ist.

## Revendications

1. Composition comprenant un extrait de chardon du Japon et un ou plusieurs parmi : extrait de pépin de raisin ; extrait de fruit amla ; extrait d'écorce de pin ; et extrait de menthe de Chine.

2. Composition selon la revendication 1, comprenant en outre un ou plusieurs parmi de l'acide folique et un dérivé fonctionnellement actif de celui-ci, dans laquelle le dérivé fonctionnellement actif est de l'acide dihydrofolique, de l'acide tétrahydrofolique, du 5-méthyltétrahydrofolate (5-MTHF), ou une association de ceux-ci.

3. Composition selon la revendication 1 ou 2 pour l'utilisation dans un procédé de traitement ou de prévention d'une condition hyperuricémique chez un individu, le procédé comprenant l'étape de la fourniture, à l'individu, d'une quantité efficace de la composition.

4. Composition pour l'utilisation selon la revendication 3, dans laquelle le procédé comprend la fourniture de la composition à un individu qui a reçu, est en train de recevoir, et/ou recevra, une autre thérapie pour une condition hyperuricémique, dans laquelle l'autre thérapie est Febuxostat, Allopurinol, ou les deux.
